# EUROPEAN PATENT SPECIFICATION

(11) **EP 3 021 840 B1**
(45) Date of publication and mention of the grant of the patent: **11.03.2020**
(21) Application number: 14826325.4
(22) Date of filing: 18.07.2014
(51) Int. Cl.: A61K 31/18, A61K 31/05, A61K 31/4196, A61K 45/06, A61P 11/06, A61P 37/08

(54) **COMPOSITIONS FOR PREVENTION OF ALLERGIC REACTION**
ZUSAMMENSETZUNGEN ZUR VORBEUGUNG VON ALLERGISCHEN REAKTIONEN
COMPOSITIONS POUR LA PRÉVENTION D'UNE RÉACTION ALLERGIQUE

(30) Priority: 18.07.2013 US 201361847766 P
(43) Date of publication of application: 25.05.2016
(73) Proprietor: Baylor College of Medicine, Houston, TX 77030-3411 (US); The U.S.A. as represented by the Secretary, Department of Health and Human Services, Bethesda, MD 20892-7660 (US)
(72) Inventor: TWEARDY, David, J., Houston, TX 77005 (US); KASEMBELI, Moses, M., Houston, TX 77025 (US); XU, Marvin, X., Henan 475100 (CN); MILNER, Josh, Bethesda, MD 20892-7660 (US); BOCCHINI, Claire E., Houston, Texas 77030 (US)
(74) Representative: J A Kemp LLP
(86) International application number: PCT/US2014/047324
(87) International publication number: WO 2015/010106

(56) References cited:
- US-A- 4 279 909
- US-A1- 2010 041 685
- US-A1- 2010 209 950
- US-A1- 2013 123 266
- Aries C Gavino ET AL: "Small-Molecule Inhibition Of Stat3 Prevents House-Dust-Mite (HDM)-Induced Airway Inflammation By Blocking Lung Production Of Th17 and Th2 Cytokines", , 1 March 2014 (2014-03-01), page 191, XP055337244, Retrieved from the Internet: URL:https://aaaai.confex.com/aaaai/2014/we bprogram/Paper12410.html [retrieved on 2017-01-20]
- M. C. SIMEONE-PENNEY ET AL: "Airway Epithelial STAT3 Is Required for Allergic Inflammation in a Murine Model of Asthma", THE JOURNAL OF IMMUNOLOGY, vol. 178, no. 10, 2 May 2007 (2007-05-02), pages 6191-6199, XP055337485, US ISSN: 0022-1767, DOI: 10.4049/jimmunol.178.10.6191
- HOX VALERIE ET AL: "Diminution of signal transducer and activator of transcription 3 signaling inhibits vascular permeability and anaphylaxis", JOURNAL OF ALLERGY AND CLINICAL IMMUNOLOGY, ELSEVIER, AMSTERDAM, NL, vol. 138, no. 1, 2 March 2016 (2016-03-02) , pages 187-199, XP029627830, ISSN: 0091-6749, DOI: 10.1016/J.JACI.2015.11.024
- AVERY ET AL.: 'STAT3 is required for IL -21-induced secretion of IgE from human naive B cells' BLOOD vol. 112, no. 5, 01 September 2008, pages 1784 - 1793, XP055313278

## Description

This application claims priority to U.S. Provisional Patent Application Serial No. 61/847,766, filed July 18, 2013.

### STATEMENT REGARDING FEDERALLY SPONSORED RESEARCH OR DEVELOPMENT

This invention was made with government support from National Institute of Allergy and Infectious Diseases, and this invention was made with government support under P50 CA058183, K08 HL085018-01A2, P50 CA097007, R21 CA149783, and R41 CA153658, awarded by National Institutes of Health. The United States Government has certain rights in the invention.

### TECHNICAL FIELD

The present invention generally concerns at least the fields of cell biology, molecular biology, and medicine.

### BACKGROUND

Anaphylaxis is a systemic hyperacute allergic reaction that causes more than 1,500 deaths per year in the United States. It is associated with intense vasodilatation and bronchoconstriction, severe laryngeal edema, drop of cardiac pressure, and hypothermia.

Anaphylaxis can occur in response to almost any foreign substance, although usual triggers include insect venom, foods, medication, and in some cases semen, latex, hormonal changes, or food additives. Physical factors, including exercise or temperature (either hot or cold) may also act as triggers because of their direct effects on mast cells. Exercise induced events are frequently associated with the ingestion of certain foods. In some cases, the cause is idiopathic.

US 2010/041685 A1 describes small molecule inhibitors of Stat3 and their derivatives, methods to inhibit cell growth by the use of Stat3 inhibitors, and the use of stat3 inhibitors for the prevention and/or treatment of cancer.

M. C. Simeone-Penney et al., J. Immunol., 178(10), 2007, 6191-6199 describes the role of the Stat3 transcription factor in mediating allergic asthma and inflammation, and the action of this transcription factor as an epithelial regulator of the allergic response.

D. T. Avery et al., Blood, 112(5), 2008, 1784-1793 describes the role of IL-21 as an inducer of IgE production by CD40L-stimulated human naive B cells and the Stat3-dependence of this induction.

The present disclosure satisfies a need in the art to provide novel compounds and methods for treating and/or preventing anaphylaxis in individuals.

### SUMMARY

The invention is defined by the claims. Any subject-matter falling outside the scope of the claims is provided for information purposes only. Any references in the description to methods of treatment refer to the compounds, pharmaceutical compositions and medicaments of the present invention for use in a method for treatment of the human or animal body by therapy.

Embodiments of the disclosure include compositions for use in the prevention and/or reduction in the risk or severity of anaphylaxis. In alternative embodiments, one or more compositions herein are useful for the treatment of anaphylaxis In embodiments of the invention, there are compositions for use in the prevention and/or reduction in the risk or severity of anaphylaxis associated with mast cell degranulation.

Embodiments of the disclosure include or compositions for use in the prevention of anaphylaxis in an individual known to have anaphylaxis, suspected of having anaphylaxis, or at risk for having anaphylaxis. The compositions include small molecules as described herein. In some embodiments, the individual is receiving an additional therapy for the prevention and/or treatment of anaphylaxis.

In at least certain embodiments, an individual receives an effective amount of the composition for the inhibition of mast cell activity, such as the inhibition of mast cell degranulation.

In at least certain embodiments, an individual receives an effective amount of the composition as a preventative indication. The composition may be administered continually through the life of the patient following a realization of a need thereof. The composition may be administered only in anticipation of being in an environment that puts the individual at risk for being in need thereof. For example, the individual may be susceptible for allergic reaction (including anaphylaxis) from a particular food allergen but may be administered the composition prior to consumption of the food (days, hours, or minutes before consumption, for example). An individual with a susceptibility to allergic reaction to insect stings may be administered the composition prior to exposure to an environment or situation where the individual is at risk of being stung by the insect. An individual may be susceptible to allergic reaction because the allergen is only present in an environment of the individual in a seasonal pattern, and in such cases the individual may be administered the composition prior to and/or during the season.

In embodiments of the disclosure, an individual is given more than one dose of one or more compositions described herein. The dosing regimen may be separated in time by minutes, hours, days, months or years.

An individual in need thereof may be an individual that has at least one symptom of allergic reaction, is susceptible to having allergic reaction, has a biological marker for having allergic reaction but never been exposed to the allergen in a natural environment, or has had allergic reaction in the past. In certain cases, the individual has a family history of allergic reaction, including a family history of anaphylaxis; in such cases, the individual may or may not be known to have allergic reaction, including anaphylaxis.

Delivery of the composition of the invention may occur by any suitable route, including systemic or local, although in specific embodiments, the delivery route is oral, intravenous, topical, subcutaneous, intraarterial, intraperitoneal, buccal, and so forth, for example.

In some embodiments of the invention, the compositions of the invention are useful for preventing and/or reducing the risk of or severity of anaphylaxis, and in specific cases such action occurs by inhibiting Stat3 and/or Stat1 activity. In some embodiments of the invention, the compositions of the invention are useful for preventing and/or reducing the risk of or severity of anaphylaxis, and in specific cases such action occurs by inhibiting or reducing mast cell degranulation. In certain embodiments, the compositions inhibit Stat3 but fail to inhibit Stat1. In some embodiments, compounds of the invention interact with the Stat3 SH2 domain, competitively inhibit recombinant Stat3 binding to its immobilized pY-peptide ligand, and/or inhibit IL-6-mediated tyrosine phosphorylation of Stat3, for example. In particular embodiments, the compositions of the invention fulfill the criteria of interaction analysis (CIA): 1) global minimum energy score ≤-30; 2) formation of a salt-bridge and/or H-bond network within the pY-residue binding site of Stat3; and/or 3) formation of a H-bond with or blocking access to the amide hydrogen of E638 of Stat3, for example. In some embodiments, the composition(s) interacts with a hydrophobic binding pocket with the Stat3 SH2 domain. In some embodiments, the composition(s) inhibit the binding of Stat3 to its cognate phosphopeptide ligand. In some embodiments, the composition(s) inhibit cytokine-mediated Stat3 phosphorylation within cells. In some embodiments, the composition(s) inhibit nuclear translocation of Stat3 within cells.

The invention provides a composition for use in a method of preventing and/or reducing the risk or severity of anaphylaxis in an individual comprising delivering to the individual a therapeutically effective amount of a compound selected from the group consisting of N-(1',2-dihydroxy-1,2'-binaphthalen-4'-yl)-4-methoxybenzenesulfonamide (which may be referred to as Cpd 188-9), -N-(3,1'-Dihydroxy-[1,2']binaphthalenyl-4'-yl)-4-methoxy-benzenesulfonamide, N-(4,1'-Dihydroxy-[1,2']binaphthalenyl-4'-yl)-4-methoxy-benzenesulfonamide, N-(5,1'-Dihydroxy-[1,2']binaphthalenyl-4'-yl)-4-methoxy-benzenesulfonamide, N-(6,1'-Dihydroxy-[1,2']binaphthalenyl-4'-yl)-4-methoxy-benzenesulfonamide, N-(7,1'-Dihydroxy-[1,2']binaphthalenyl-4'-yl)-4-methoxy-benzenesulfonamide, N-(8,1'-Dihydroxy-[1,2']binaphthalenyl-4'-yl)-4-methoxy-benzenesulfonamide, 4-Bromo-N-(1,6'-dihydroxy-[2,2']binaphthalenyl-4-yl)-benzenesulfonamide, and a mixture thereof.

The invention additionally provides the composition above wherein said method comprises providing the composition to said individual in multiple doses, wherein preferably the multiple doses are separated by minutes, hours, days or weeks.

In another embodiment, the invention provides the composition above wherein in said method the composition is provided to said individual prior to exposure or following exposure to at least one allergen that induces the anaphylaxis.

In this embodiment, the composition is preferably provided to said individual at least 0.25, 0.5, 0.75, 1, 2, 3, 4, 5, 6, 7, 8, 9, 10, 11, 12, 13, 14, 15, 16, 17, 18, 19, 20, 21, 22, 23, or 24 hours; or at least 1, 2, 3, 4, 5, 6, or 7 days; or at least 1, 2, 3, or 4 weeks prior to or following exposure to at least one allergen that induces the anaphylaxis.

In a preferable embodiment, said allergen is a food allergen, wherein preferably the food allergen is one or more food allergens selected from the group consisting of milk, legumes, shellfish, tree nuts, eggs, fish, soy, and wheat.

In another preferable embodiment, said allergen is an environmental allergen or seasonal allergen, wherein preferably the environmental allergen or seasonal allergen is pollen or mold.

In an additional preferable embodiment, the allergen is a venom allergen, wherein preferably the venom allergen is from an organism selected from the group consisting of wasp, bee, ant, hornet, yellow jacket, and asp.

In a further preferable embodiment, said allergen is a medication allergen, wherein preferably the medication allergen is selected from the group consisting of anesthetics, β-lactam antibiotics, aspirin, non-steroidal anti-inflammatory drug, chemotherapy, vaccine, protamine and herbal preparations.

In another preferable embodiment, the allergen is latex.

The invention further provides the composition above wherein said method further comprises the step of diagnosing the anaphylaxis.

Also described herein are methods of treating anaphylaxis in an individual wherein the composition(s) is an inhibitor of any members of the STAT protein family, including STAT1, STAT2, STAT3, STAT4, STAT5 (STAT5A and STAT5B), or STAT6, for example.

### DESCRIPTION OF THE DRAWINGS

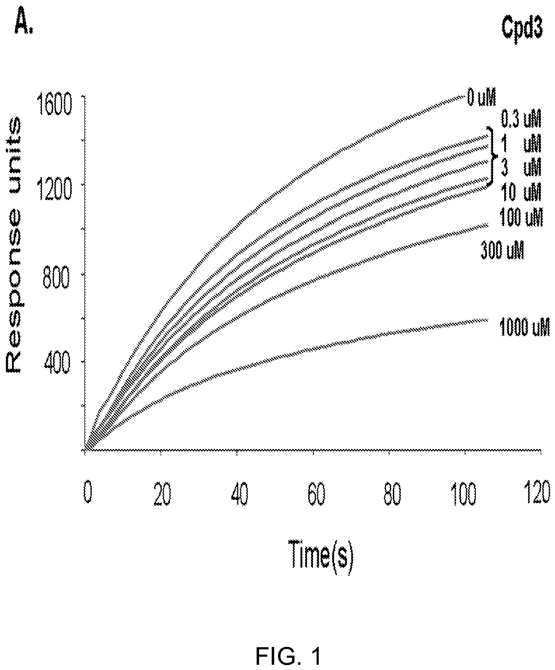

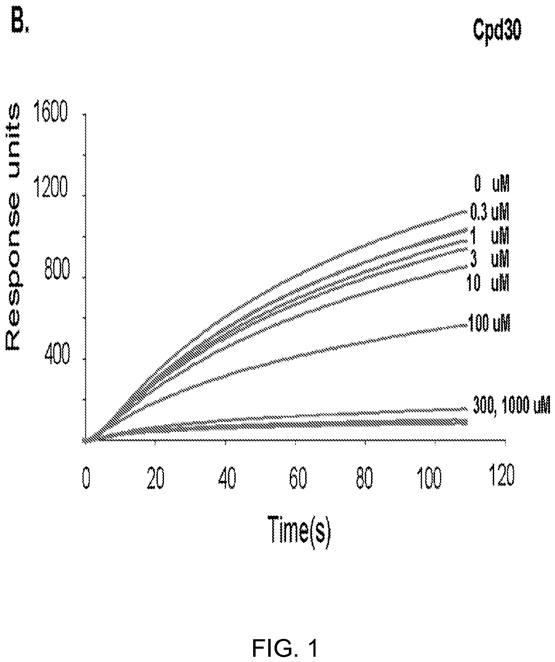

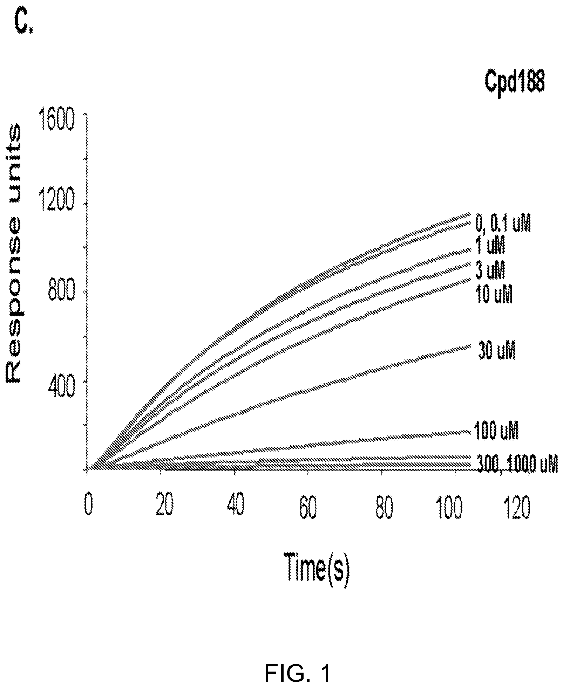

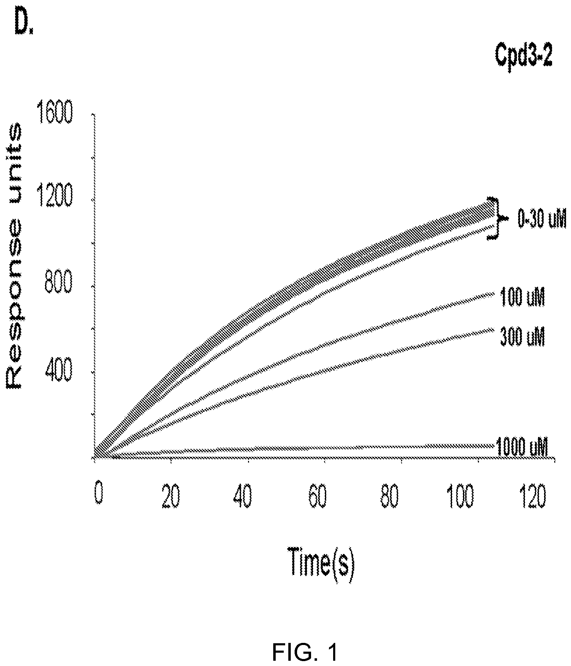

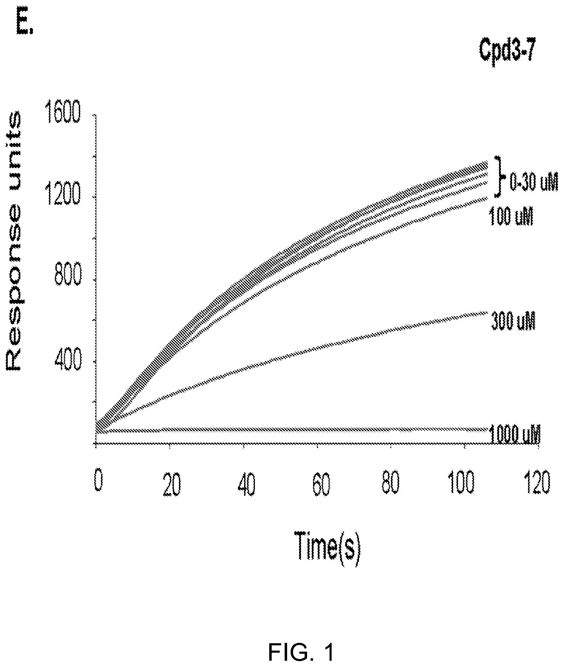

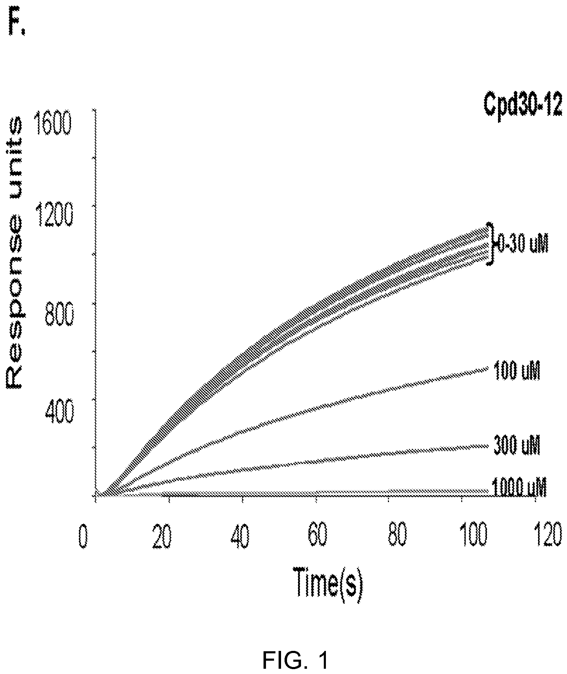

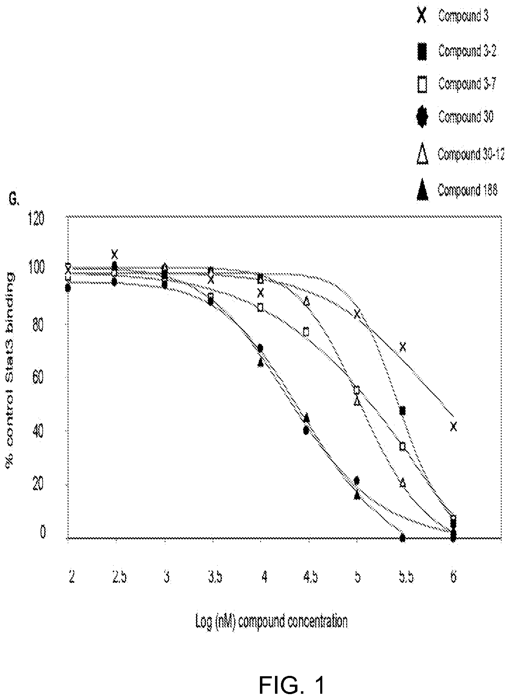

FIG. 1 demonstrates inhibition of Stat3 binding to immobilized phosphopeptide ligand by compounds. Binding of recombinant Stat3 (500nM) to a BiaCore sensor chip coated with a phosphododecapeptide based on the amino acid sequence surrounding Y1068 within the EGFR was measured in real time by SPR (Response Units) in the absence (0 µM) or presence of increasing concentrations (0.1 to 1,000 µM) of Cpd3 (panel A), Cpd30 (panel B), Cpd188 (panel C), Cpd3-2 (panel D), Cpd3-7 (panel E) and Cpd30-12 (panel F). Data shown are representative of 2 or more experiments. The equilibrium binding levels obtained in the absence or presence of compounds were normalized (response obtained in the presence of compound ÷ the response obtained in the absence of compound x 100), plotted against the log concentration (nM) of the compounds (panel G). The experimental points fit to a competitive binding curve that uses a four-parameter logistic equation (see exemplary methods for details). These curves were used to calculate IC₅₀ (Table 4).

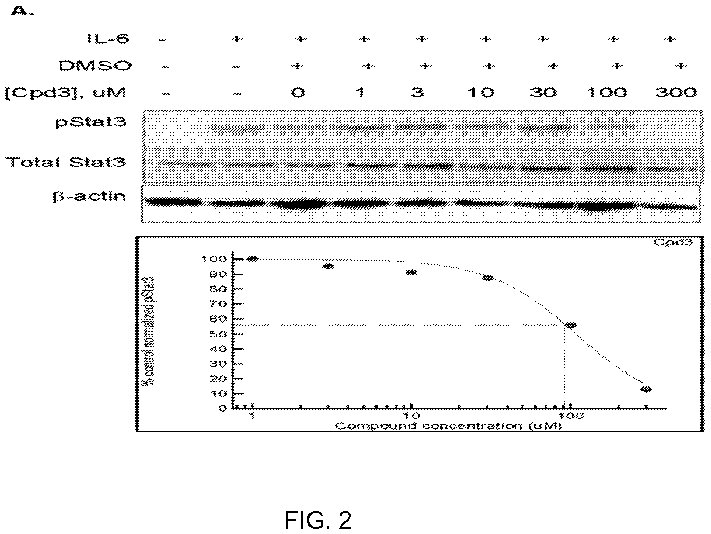

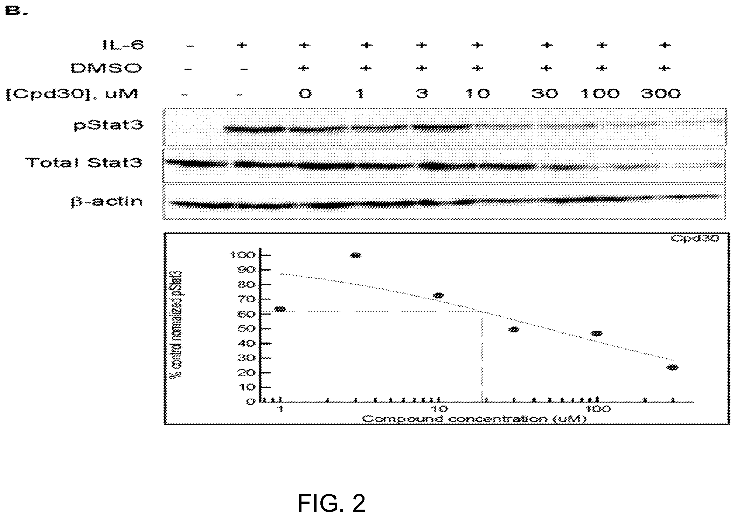

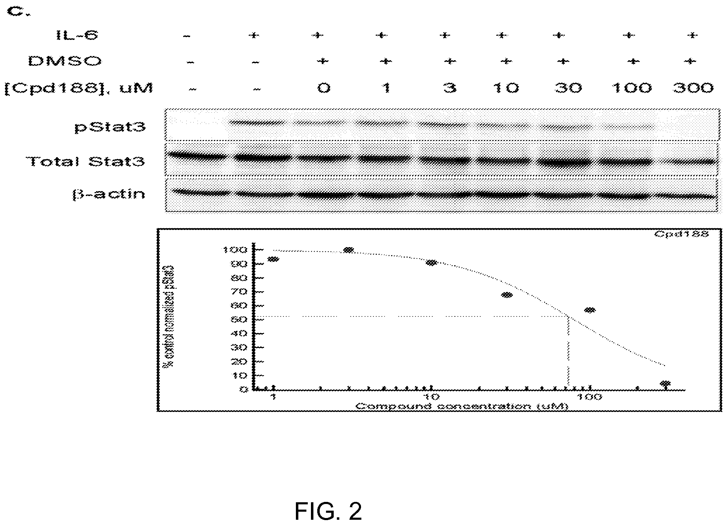

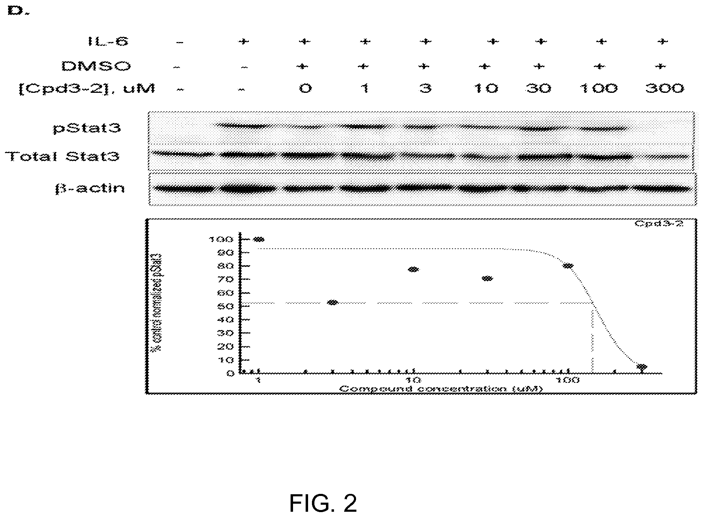

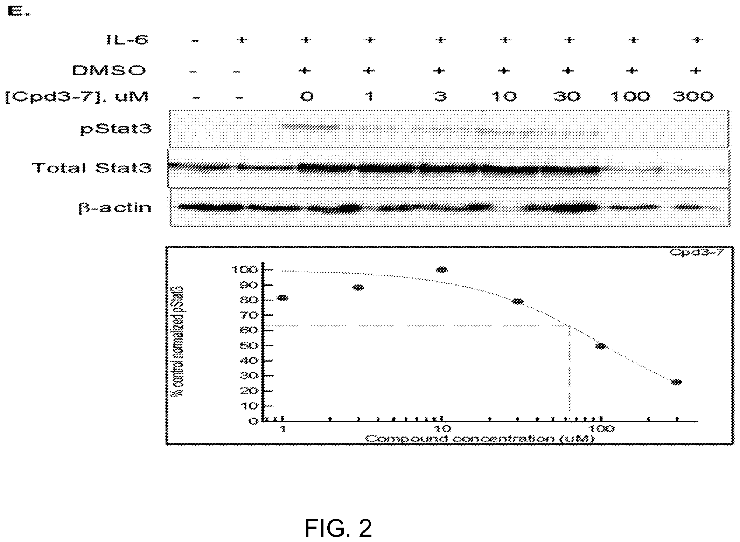

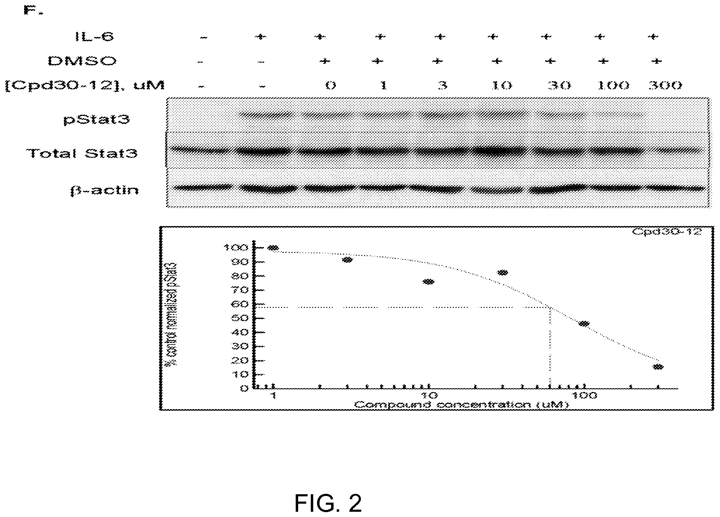

FIG. 2 demonstrates inhibition of IL-6-mediated activation of Stat3 by compounds. HepG2 cells were pretreated with DMSO alone or DMSO containing Cpd3 (panel A), Cpd188 (panel B), Cpd30 (panel C), Cpd3-2 (panel D), Cpd3-7 (panel E) or Cpd30-12 (panel F) at the indicated concentration for 60 min. Cells were then stimulated with IL-6 (30 ng/ml) for 30 min. Protein extracts of cells were separated by SDS-PAGE, blotted and developed serially with antibodies to pStat3, total Stat3 and β-actin. Blots were stripped between each antibody probing. The bands intensities of immunoblot were quantified by densitometry. The value of each pStat3 band's intensity was divided by each corresponding value of total Stat3 band intensity and the results normalized to the DMSO-treated control value and plotted as a function of the log compound concentration. The best-fit curves were generated based on 4 Parameter Logistic Model/Dose Response One Site/XLfit 4.2, IDBS. Each panel is representative of 3 or more experiments.
FIG. 3 provides chemical formulas and names of compounds. The chemical formulas and names are indicated for Cpd3 (panel A), Cpd30 (panel B), Cpd188 (panel C), Cpd3-2 (panel D), Cpd3-7 (panel E) and Cpd30-12 (panel F).
FIG. 4 shows effect of compounds on Stat1 activation. HepG2 cells were pretreated with DMSO alone or DMSO containing each of the compounds at a concentration of 300 µM for 60 min. Cells were then stimulated with IFN-γ (30 ng/ml) for 30 min. Protein extracts of cells were separated by SDS-PAGE and immunoblotted serially with antibodies to pStat1, total Stat1 and β-actin. Blots were stripped between each immunoblotting. The results shown are representative of 2 or more experiments.

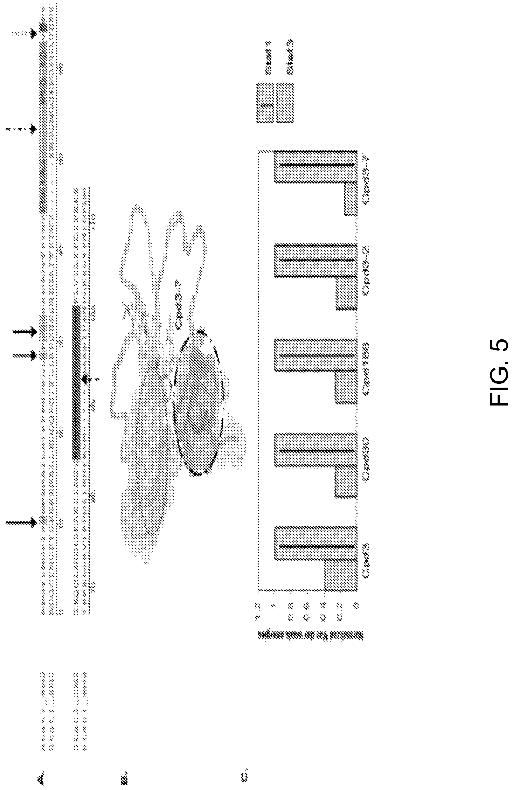

FIG. 5 provides comparisons of the Stat3 and Stat1 SH2 domain sequences, 3-D structures and van der Waals energies of compound binding. Sequence alignment of Stat3 and Stat1 SH2 domains is shown in panel A. The residues that bind the pY residue are highlighted in and pointed to by a solid arrow, the residue (E638) that binds to the +3 residue highlighted and pointed to by a dotted arrow and Loop_{βC_βD} and Loop_{αB-αC}, which comprise the hydrophobic binding site consisting, are highlighted and pointed to by dot-dashed and dashed arrows, respectively. Panel B shows an overlay of a tube-and-fog van der Waals surface model of the Stat3 SH2 domain and a tube-and-fog van der Waals surface model of the Stat1 SH2. The residues of the Stat3 SH2 domain represents Loop_{βC_βD} are highlighted and shown by dotted circles and the residues represent Loop_{αB-αC} are highlighted and shown by a dotted-dashed circle; the corresponding loop residues within the Stat1 SH2 domain are shown in a light fog surrounding the circles. This overlay is shown bound by Cpd3-7 as it would bind to the Stat3 SH2 domain. The van der Waals energy of each compound bound to the Stat1 SH2 domain or the Stat3 SH2 domain was calculated, normalized to the value for Stat1 and depicted in panel C.

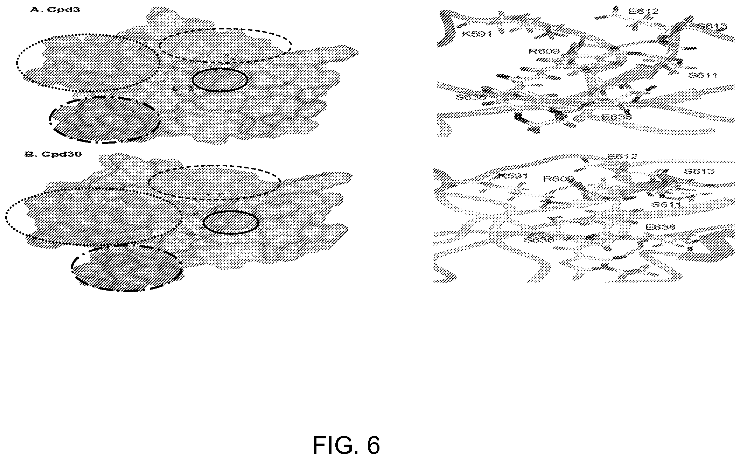

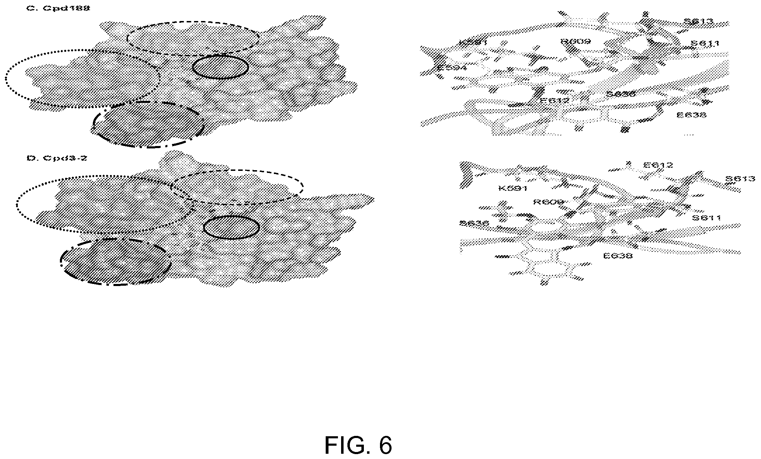

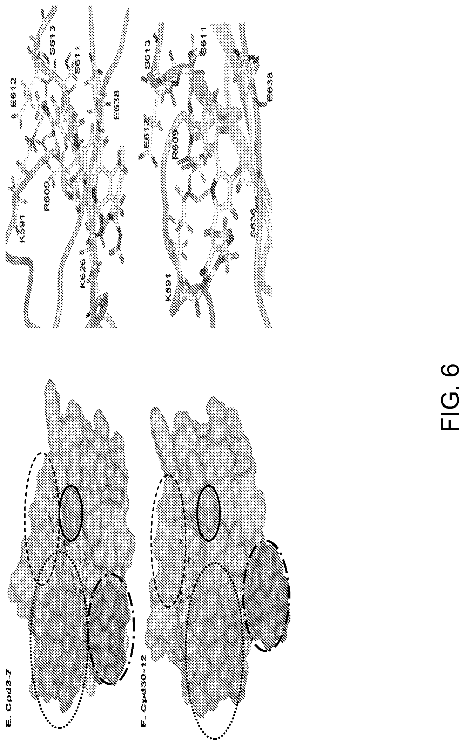

FIG. 6 shows a computer model of each compound bound by the Stat3 SH2 domain. The results of computer docking to the Stat3 SH2 domain is shown for Cpd3 (panel A), Cpd30 (panel B), Cpd188 (panel C), Cpd3-2 (panel D), Cpd3-7 (panel E) and Cpd30-12 (panel F). The image on the left of each panel shows the compound binding to a spacefilling model of the Stat3 SH2 domain. The pY-residue binding site is represented by dashed circle, the +3 residue binding site is represented by a solid circle, loop Loop_{βC_βD} is represented by dotted circle and loop Loop_{αB-αC} is represented by dot-dashed circle. Residues R609 and K591 critical for binding pY are shown within a dashed circle, residue E638 that binds the +3 residue shown within a solid circle and the hydrophobic binding site consisting of Loop_{βC_βD} and LoopαB-αC is shown within a dash-dot and dotted circle, respectively. The image on the right side of each panel is a closer view of this interaction with hydrogen bonds indicated by dotted lines. In FIG. 6A the negatively charged benzoic acid moiety of Cpd3 has electrostatic interactions with the positively-charge pYresidue binding site consisting mainly of the guanidinium cation group of R609 and the basic ammonium group of K591. The benzoic acid group also forms a hydrogen-bond network consisting of double H-bonds between the carboxylic oxygen and the ammonium hydrogen of R609 and the amide hydrogen of E612. H-bond formation also occurs between the benzoic acid carbonyl oxygen and the side chain hydroxyl hydrogen of Serine 611. Within the +3 residue-binding site, the oxygen atom of 1,4-benzodioxin forms a hydrogen bond with the amide hydrogen of E638. In addition, the 2,3-dihydro-1,4- benzodioxin of Cpd3 interacts with the loops forming the hydrophobic binding site. In FIG. 6B the carboxylic terminus of the benzoic acid moiety of Cpd30, which is negatively charged under physiological conditions, forms a salt bridge with the guanidinium group of R609 within the pYresidue binding site. Within the +3 residue-binding site, the oxygen of the thiazolidin group forms a H-bond with the peptide backbone amide hydrogen of E638. In addition, the thiazolidin moiety plunges into the hydrophobic binding site. In FIG. 6C there is an electrostatic interaction between the (carboxymethyl) thio moiety of Cpd188 carrying a negative charge and the pY-residue binding site consisting of R609 and K591 carrying positive charge under physiological conditions. There are H-bonds between the hydroxyloxygen of the (carboxymethyl) thio group of Cpd188 and the guanidinium hydrogen of R609, between the hydroxyl-oxygen of the (carboxymethyl) thio group and the backbone amide hydrogen of E612, and between the carboxyl-oxygen of the (carboxymethyl) thio group of Cpd188 and the hydroxyl-hydrogen of S611. Within the +3 residue-binding site, there is a H-bond between the hydroxyl-oxygen of benzoic acid group of Cpd188 and the amide-hydrogen of E638. In addition, the benzoic acid group extends and interacts with the hydrophobic binding site. In FIG. 6D the benzoic acid group of Cpd3-2 has significant electrostatic interactions with the pY-residue binding site pocket, mainly contributed by R609 and K591, and forms two H bonds; the carboxylic oxygen of the benzoic acid group binds the guanidinium hydrogen of R609, and the carbonyl oxygen of the benzoic acid group binds to the carbonyl hydrogen of S611. Within the +3 residue-binding site, oxygen within the 1,3-dihydro-2H-inden-2-ylidene group forms an H bond to the backbone amide-hydrogen of E638. In addition, the 1,3-dihydro-2H-inden-2-ylidene group plunges into the hydrophobic binding site. In FIG. 6E H-bonds are formed between the carbonyl-oxygen of the methyl 4-benzoate moiety of Cpd 3-7 and the side chain guanidinium of R609 and between the methoxy-oxygen and the hydrogen of the ammonium terminus of K591. The (2-methoxy-2-oxoethyl)-4,8-dimethyl-2-oxo-2H-chromen group of Cpd3-7 blocks access to the amide hydrogen of E638 within the +3 residue-binding site. In addition, this group plunges into the hydrophobic binding site. In FIG. 6F there are electrostatic interactions between the benzoic acid derivative group of Cpd30-12 and R609 and 591 within the pY-residue binding site. Also, H-bonds are formed between the hydroxyl-oxygen of Cpd30-12 and the guanidinium-hydrogen of R609, between the carboxyl-oxygen of Cpd30-12 and the hydroxyl-hydrogen of S611 and between the furyl group of Cpd30-12 and the hydrogen of ammonium of K591. The 1,3-diethyl-4, 6-dioxo-2-thioxotetrahydro-5(2H)- pyrimidinylidene groups blocks access to the +3 residue binding site; however, it extends into the groove between the pY-residue binding site and LoopβC-βD, while sparing the hydrophobic binding site.

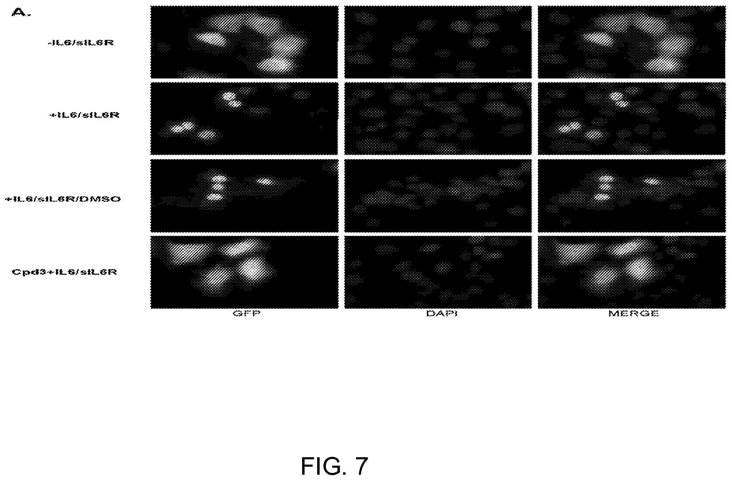

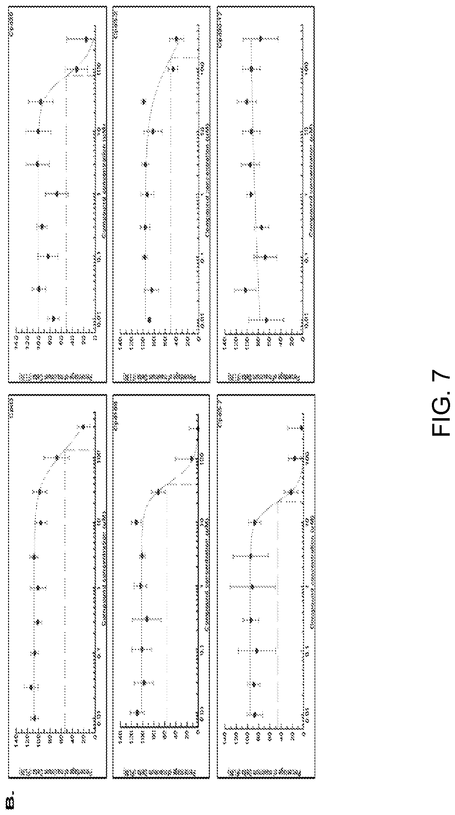

FIG. 7 shows inhibition of cytoplasmic-to-nuclear translocation of Stat3 assessed by confocal and high-throughput fluorescence microscopy. In panel A, MEF/GFP-Stat3 cells grown on coverslips were pretreated with DMSO that either contained (row four) or did not contain (row three) Cpd3 (300 µM) for 60 min before being stimulated without (row one) or with IL-6 (200ng/ml) and IL-6sR (250ng/ml) for 30 minutes (rows two, three and four). Coverslips were examined by confocal fluorescent microscopy using filters to detect GFP (column one), DAPI (column two) or both (merge; column three). In panel B, MEF-GFP-Stat3 cells were grown in 96-well plates with optical glass bottoms and pretreated with the indicated compound at the indicated concentrations in quadruplicate for 1 hour then stimulated with IL-6 (200ng/ml) and IL-6sR (250ng/ml) for 30 minutes. Cells were fixed and the plates were examined by high-throughput microscopy to determine the fluorescence intensity in the nucleus (FLIN) and the %ΔFLIN_{Max} was calculated as described in Example 1. Data shown are mean ± SD and are representative of 2 or more studies. Best-fit curves were generated based on 4 Parameter Logistic Model/Dose Response One Site /XLfit 4.2, IDBS and were used to calculate IC₅₀ (Table 1).
FIG. 8 demonstrates inhibition of Stat3 DNA binding by compounds. Electrophoretic mobility shift assays were performed using whole-cell extracts prepared from HepG2 cells without and with stimulation with IL-6 (30ng/ml) for 30 min. Protein (20 µg) was incubated with radiolabeled duplex oligonucleotide (hSIE) and DMSO without or with the indicated compounds (300uM) for 60 minutes at 37° C then separated by PAGE. The gel was dried and autoradiographed; the portion of the gel corresponding to the Stat3-bound hSIE band is shown. Data shown are representative of 2 studies.

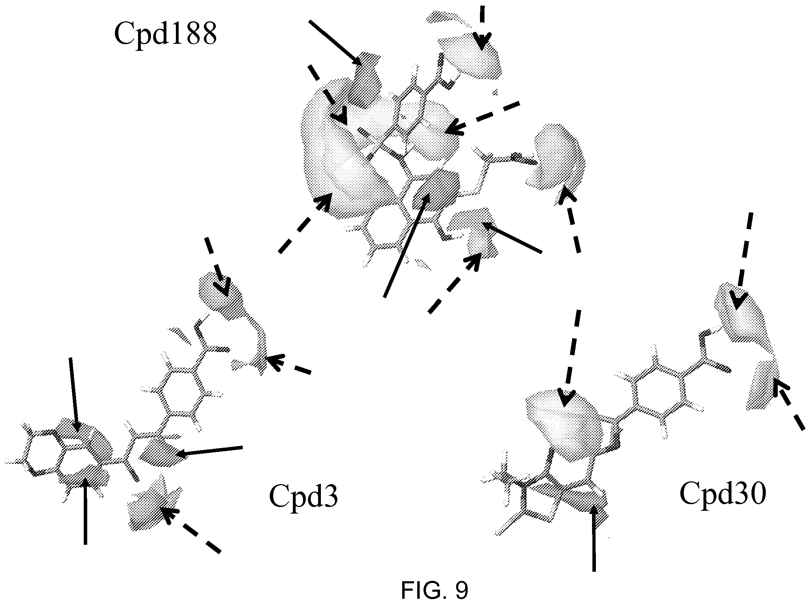

FIG. 9 shows Cpd3, Cpd30 and Cpd188 and the hydrophobicity or hydrophilicity of the surface of the molecule. The dashed arrows point to hydrophilic surfaces, and the solid arrows point to hydrophobic surfaces.

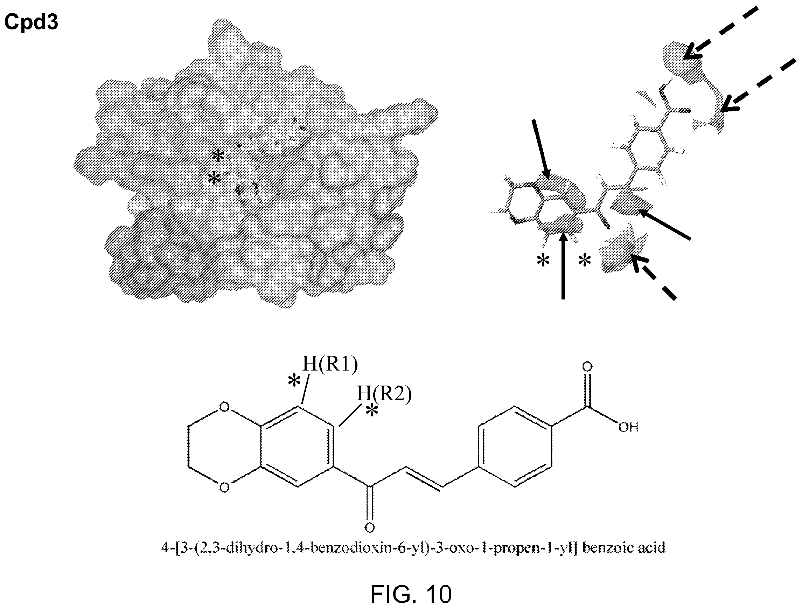

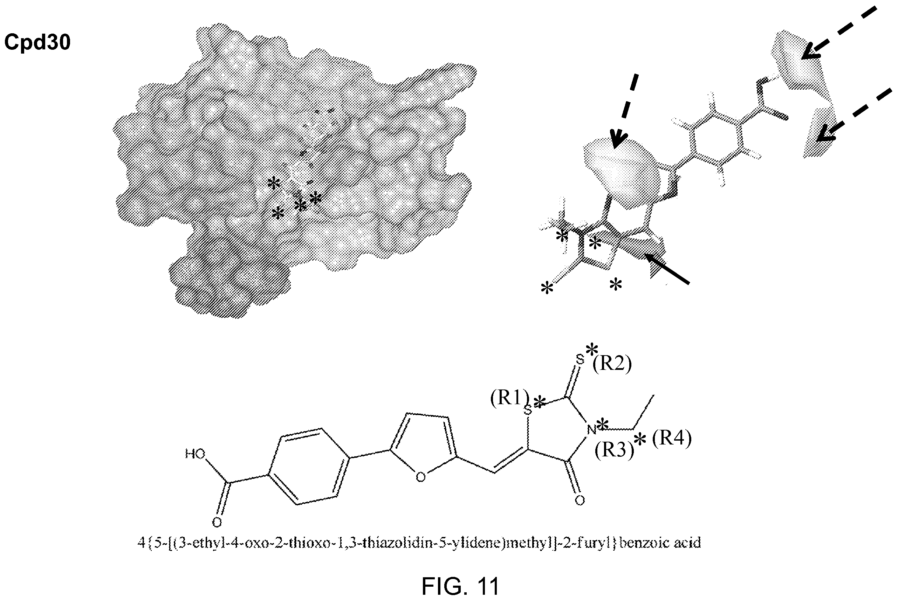

FIG. 10 illustrates reference compound 3 (Cpd3). The top-left picture of FIG. 11 shows Cpd3 docked into Stat3 and the interaction between Cpd3 and the surface of the protein and derivatives of Cpd3 that can fit into the surface of the protein. Stars represent atoms and chemical groups that can be replaced with other atoms or chemical groups to create one or more functional derivatives. The hydrophobic/hydrophilic surfaces of Cpd3 are also demonstrated on the top-right picture. The dashed arrows point to hydrophilic surfaces, and the solid arrows point to hydrophobic surfaces. R₁ and R₂ could be identical or different and may comprise hydrogen, carbon, sulfur, nitrogen, oxygen, alkanes. cyclic alkanes, alkane-based derivatives, alkenes, cyclic alkenes, alkene-based derivatives, alkynes, alkyne-based derivative, ketones, ketone-based derivatives, aldehydes, aldehyde-based derivatives, carboxylic acids, carboxylic acid-based derivatives, ethers, ether-based derivatives, esters and ester-based derivatives, amines, amino-based derivatives, amides, amide-based derivatives, monocyclic or polycyclic arene, heteroarenes. arene-based derivatives, heteroarene-based derivatives, phenols, phenol-based derivatives, benzoic acid, or benzoic acid-based derivatives.

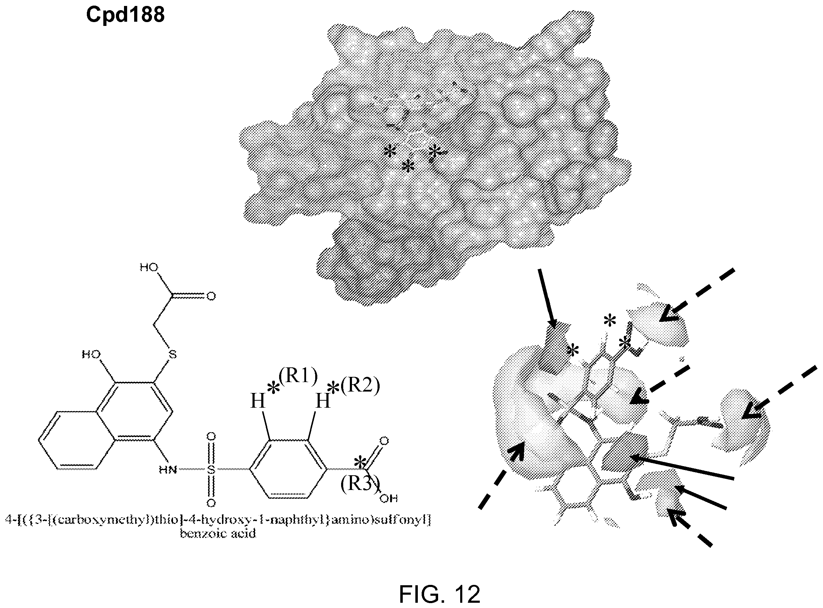

FIG. 11 illustrates reference compound 30 (Cpd30). The top-left picture of FIG. 12 shows Cpd30 docked into Stat3 and the interaction between Cpd30 and the surface of the protein, and derivatives of Cpd30 that fit into the surface of the protein. Stars represent atoms and chemical groups that can be replaced with other atoms or chemical groups to create one or more functional derivatives. The hydrophobic/hydrophilic surfaces of Cpd30 are also demonstrated on the top-right picture. The dashed arrows point to hydrophilic surfaces, and the solid arrows point to hydrophobic surfaces. 2-D structure of Cpd30 shown on the bottom picture, R₁, R₂ R₃ and R₄ could identical or different and may comprise be hydrogen, carbon, sulfur, nitrogen, oxygen, alkanes. cyclic alkanes, alkane-based derivatives, alkenes, cyclic alkenes, alkene-based derivatives, alkynes, alkyne-based derivative, ketones, ketone-based derivatives, aldehydes, aldehyde-based derivatives, carboxylic acids, carboxylic acid-based derivatives, ethers, ether-based derivatives, esters and ester-based derivatives, amines, amino-based derivatives, amides, amide-based derivatives, monocyclic or polycyclic arene, heteroarenes. arene-based derivatives, heteroarene-based derivatives, phenols, phenol-based derivatives, benzoic acid, aor benzoic acid-based derivatives.
FIG. 12 illustrates reference compound 188 (Cpd188). The top picture of FIG. 12 shows Cpd188 docked into Stat3 SH2 domain and the interaction between Cpd188 and the surface of the protein, and derivatives of Cpd188 that fit into the surface of the protein. Stars represent atoms and chemical groups that can be replaced with other atoms or chemical groups to create one ore more functional derivative. The hydrophobic/hydrophilic surfaces of Cpd188 are also demonstrated on the left picture on the bottom. The dashed arrows point to hydrophilic surfaces, and the solid arrows point to hydrophobic surfaces. Shown on the right bottom picture, R₁ and R₂ could be identical or different and may comprise hydrogen, carbon, sulfur, nitrogen, oxygen, alkanes. cyclic alkanes, alkane-based derivatives, alkenes, cyclic alkenes, alkene-based derivatives, alkynes, alkyne-based derivative, ketones, ketone-based derivatives, aldehydes, aldehyde-based derivatives, carboxylic acids, carboxylic acid-based derivatives, ethers, ether-based derivatives, esters and ester-based derivatives, amines, amino-based derivatives, amides, amide-based derivatives, monocyclic or polycyclic arene, heteroarenes. arene-based derivatives, heteroarene-based derivatives, phenols, phenol-based derivatives, benzoic acid, or benzoic acid-based derivatives.
FIG. 13 illustrates schematic diagrams of Stat1 and Stat3.

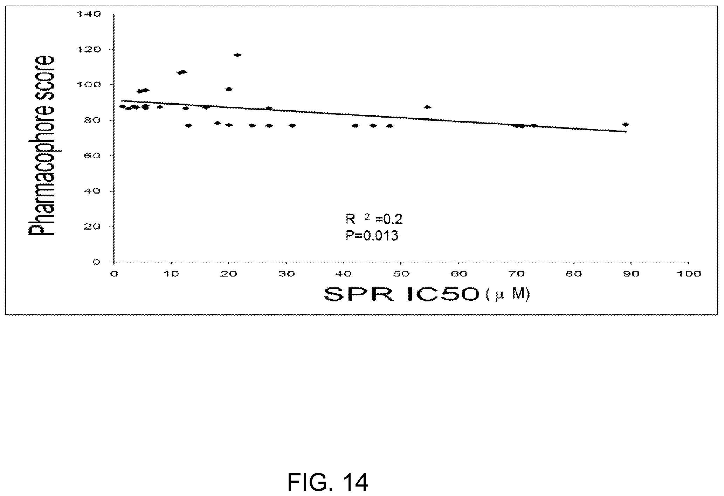

FIG. 14 demonstrates that SPR IC₅₀ of 2nd generation Stat3 chemical probes is inversely correlated with 3-D pharmacophore score.
FIG. 15. shows SPR IC₅₀ and AML apoptosis EC₅₀ of parent Cpd188 and two 2nd generation 188-like Stat3 chemical probes.
FIG. 16 provides an illustration of structure-activity relationships of 38 Cpd188-like, 2nd generation Stat3 probes.
FIG. 17 shows an exemplary modification scheme for 3rd generation Stat3 probe development using Cpd188-15 as a scaffold.
FIG. 18 provides illustration of the electrostatic surface of Stat3 SH2 domain (positive area in blue, neutral in white and negative in red in a color figure) and 20 docking poses of 5 (R = CH₂PO₃²⁻), showing strong interactions between phosphonate groups (in purple and red) and K591/R609.
FIG. 19A shows physician diagnosed food allergies in healthy volunteers, AD-HIES, and atopic control patients were determined by interview. FIG. 19B demonstrates incidence of physician diagnosed anaphylaxis in AD-HIES and atopic control patients. Significance determined by a two-tailed Chi-squared test.
FIG. 20A shows that mast cell degranulation was measured by FcεRI crosslinking and subsequent β-hexosaminidase release in LAD2 cells transduced with five different shRNAs against STAT3. Data representative of two independent experiments. LAD2 = unstimulated control, LAD+ = FceRI crosslinking. FIG. 20B shows that mast cell degranulation was measured by FcεRI crosslinking and subsequent β-hexosaminidase release in primary human mast cells transduced with two different shRNAs against STAT3. HuMC = unstimulated control, HuMC+ = FcεRI crosslinking.
FIG. 21 demonstates correlation between STAT23 knockdown and inhibition of mast cell degranulation (r²=0.9463).
FIG. 22 demonstrates effective treatment in an anaphylaxis model using Cpd188-9.
FIG. 23 provides a dose response curve using different dosages of Cpd188-9 in an anaphylaxis model utilizing beta-hexosaminidase (% release) as a measure of mas cell degranulation.
FIG. 24 shows that systemic anaphylaxis was prevented *in vivo* with an exemplary STAT3 inhibitor.
FIG 25 demonstrates that peripheral and central vascular leakage is decreased by Cpd 188-9.
FIG. 26 illustrates the effect of Cpd188-9 is not because of a decreased mast cell degranulation *in vivo.*
FIG. 27 demonstrates the effct of Cpd 188-9 on Ag-induced degranulation in murine mast cells.
FIG. 28 illustrates an exemplary transwell permeability assay.
FIG. 29 shows that Cpd188-9 pretreated (as an example, for 7 days) HUVECS resistant to histamine-induced permeability.
FIG. 30 shows HIES mouse is resistant to anaphylaxis (Siegel *et al.,* JACI, 2013).
FIG. 31 demonstrates STAT3 mutant (HIES6) HUVECS resistant to histamine-induced permeability.

### DETAILED DESCRIPTION OF THE INVENTION

In some embodiments, there is a composition for use in a method of preventing, and/or reducing the risk or severity of anaphylaxis in an individual, comprising delivering to the individual one or more particular compounds. In some embodiments, the compound(s) is a STAT3 inhibitor. In particular cases, the compound(s) is a STAT1 inhibitor, but in particular cases it is not a STAT1 inhibitor. In certain aspects, there are some compounds that are both STAT3 and STAT1 inhibitors or is neither a STAT3 or STAT1 inhibitor. In some cases, the composition is a mast cell inhibitor, including a mast cell degranulation inhibitor.

The invention provides a compound for use in the prevention and/or reduction in risk or severity of anaphylaxis, wherein the compound is selected from the group consisting of N-(1',2-dihydroxy-1,2'-binaphthalen-4'-yl)-4-methoxybenzenesulfonamide, N-(3,1'-Dihydroxy-[1,2']binaphthalenyl-4'-yl)-4-methoxy-benzenesulfonamide, N-(4,1 '-Dihydroxy-[1,2']binaphthalenyl-4'-yl)-4-methoxy-benzenesulfonamide, N-(5,1'-Dihydroxy-[1,2']binaphthalenyl-4'-yl)-4-methoxy-benzenesulfonamide, N-(6,1'-Dihydroxy-[1,2']binaphthalenyl-4'-yl)-4-methoxy-benzenesulfonamide, N-(7,1'-Dihydroxy-[1,2']binaphthalenyl-4'-yl)-4-methoxy-benzenesulfonamide, N-(8,1'-Dihydroxy-[1,2']binaphthalenyl-4'-yl)-4-methoxy-benzenesulfonamide, 4-Bromo-N-(1,6'-dihydroxy-[2,2']binaphthalenyl-4-yl)-benzenesulfonamide, or a combination thereof, and a mixture thereof.

In a specific embodiment of the invention, the composition is a Stat3 inhibitor but does not inhibit Stat1. The composition may be a mast cell degranulation inhibitor.

In a specific embodiment of the invention, the composition is delivered *in vivo* in a mammal. In another embodiment the mammal is a human. In another specific embodiment the human is known to have anaphylaxis, is suspected of having anaphylaxis, or is at risk for developing anaphylaxis. In another embodiment, the human is known to have anaphylaxis and is receiving an additional therapy for the anaphylaxis. Composition(s) of the disclosure prevent and/or reduce the risk or severity of anaphylaxis.

### I. Definitions

As used herein the specification, "a" or "an" may mean one or more. As used herein in the claim(s), when used in conjunction with the word "comprising", the words "a" or "an" may mean one or more than one. As used herein "another" may mean at least a second or more. Still further, the terms "having", "including", "containing" and "comprising" are interchangeable and one of skill in the art is cognizant that these terms are open ended terms. Some embodiments of the invention may consist of or consist essentially of one or more elements, method steps, and/or methods of the invention. It is contemplated that any method or composition described herein can be implemented with respect to any other method or composition described herein.

The term "inhibitor" as used herein refers to one or more molecules that interfere at least in part with the activity of Stat3 to perform one or more activities, including the ability of Stat3 to bind to a molecule and/or the ability to be phosphorylated. In alternative embodiments, an inhibitor reduces the level of degranulation of mast cells, which may be measured *in vitro* by % release of beta-hexosaminidase or other mast cell mediators such as cytokines, histamine, leukotrienes, *etc.* The level of degranulation of mast cells may be measured *in vivo* in a multitude of allergy and anaphylaxis models that primarily measure core temperature reductions with acute challenge, vascular permeability, inflammation, or systemic mast cell mediators, such as histamine or tryptase.

The phrase "therapeutically effective amount" as used herein means that amount of a compound, material, or composition comprising a compound of the present invention that is effective for producing some desired therapeutic effect, *e.g*., treating (*i.e*., preventing and/or ameliorating) anaphylaxis in a subject, or inhibiting protein-protein interactions mediated by an SH2 domain in a subject, at a reasonable benefit/risk ratio applicable to any medical treatment. In one embodiment, the therapeutically effective amount is enough to reduce or eliminate at least one symptom. One of skill in the art recognizes that an amount may be considered therapeutically effective even if the anaphylaxis is not totally eradicated but improved partially. For example, a symptom from the anaphylaxis may be partially reduced or completed eliminated, and so forth.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "at risk for having allergic reaction" as used herein refers to an individual that has had an allergic reaction before, has one or more family members with allergic reaction history, or is a child.

As used herein, "binding affinity" refers to the strength of an interaction between two entities, such as a protein-protein interaction. Binding affinity is sometimes referred to as the Kₐ, or association constant, which describes the likelihood of the two separate entities to be in the bound state. Generally, the association constant is determined by a variety of methods in which two separate entities are mixed together, the unbound portion is separated from the bound portion, and concentrations of unbound and bound are measured. One of skill in the art realizes that there are a variety of methods for measuring association constants. For example, the unbound and bound portions may be separated from one another through adsorption, precipitation, gel filtration, dialysis, or centrifugation, for example. The measurement of the concentrations of bound and unbound portions may be accomplished, for example, by measuring radioactivity or fluorescence, for example. Kₐ also can be inferred indirectly through determination of the Kᵢ or inhibitory constant. Determination of the Kᵢ can be made several ways for example by measuring the Kₐ of STAT3 binding to its phosphopeptide ligand within the EGFR at position Y1068 and by measuring the concentration of a molecule that reduces binding of STAT3 by 50%. In certain embodiments of the invention, the binding affinity of a Stat3 inhibitor for the SH2 domain of Stat3 is similar to or greater than the affinity of the compounds listed herein.

The term "domain" as used herein refers to a subsection of a polypeptide that possesses a unique structural and/or functional characteristic; typically, this characteristic is similar across diverse polypeptides. The subsection typically comprises contiguous amino acids, although it may also comprise amino acids that act in concert or that are in close proximity due to folding or other configurations. An example of a protein domain is the Src homology 2 (SH2) domain of Stat3. The term "SH2 domain" is art-recognized, and, as used herein, refers to a protein domain involved in protein-protein interactions, such as a domain within the Src tyrosine kinase that regulates kinase activity. The invention contemplates modulation of activity, such as activity dependent upon protein-protein interactions, mediated by SH2 domains of proteins (*e.g*., tyrosine kinases such as Src) or proteins involved with transmission of a tyrosine kinase signal in organisms including mammals, such as humans.

As used herein, a "mammal" is an appropriate subject for the method of the present invention. A mammal may be any member of the higher vertebrate class Mammalia, including humans; characterized by live birth, body hair, and mammary glands in the female that secrete milk for feeding the young. Additionally, mammals are characterized by their ability to maintain a constant body temperature despite changing climatic conditions. Examples of mammals are humans, cats, dogs, cows, mice, rats, and chimpanzees. Mammals may be referred to as "patients" or "subjects" or "individuals".

### II. General Embodiments

General embodiments include one or more compositions for the prevention of anaphylaxis. An individual in need of anaphylaxis prevention, including reduction in the severity of at least one symptom of anaphylaxis, is provided with an effective amount of one or more compositions as disclosed herein. Although any composition disclosed herein may be suitable, in specific embodiments the composition is N-(1',2-dihydroxy-1,2'-binaphthalen-4'-yl)-4-methoxybenzenesulfonamide (Cpd 188-9).

In some cases an individual prevents the anaphylaxis or reduces the severity of the anaphylaxis with one or more compositions as disclosed herein by intaking the composition routinely, such as routinely after having a first anaphylaxis or after identifying the risk of having anaphylaxis (such as by a standard allergy test, for example). The term "routinely" may be described as a regular course of procedure, such as once or more than once daily, biweekly, weekly, monthly, and so forth, for example.

In some cases, an individual prevents the anaphylaxis or reduces the severity of the anaphylaxis with one or more compositions as disclosed herein by intaking the composition periodically, such as periodically after having a first anaphylaxis or after identifying the risk of having anaphylaxis (such as by a standard allergy test, for example). The period may be one or more seasons of the year. The period may be one or more periods of time for one or more increased allergens in an environment, such as during pollination of one or more types of plants, for example.

In some cases, an individual prevents the anaphylaxis or reduces the severity of the anaphylaxis with one or more compositions as disclosed herein by intaking the composition prior to an event or environment or condition where the individual is likely to be or known to be exposed to the allergen. For example, the individual may be administered the composition prior to consumption of a particular food allergen, prior to close proximity to or exposure to a particular plant allergen, prior to exposure to an environment having stinging insects, prior to an exposure to latex, prior to sexual intercourse, and so forth.

In some cases, an individual may intake the composition routinely but may take an increased dosage of the composition prior to an event or environment or condition where the individual is likely to be or known to be exposed to the allergen.

In some cases, an individual may intake the composition periodically but may take an increased dosage of the composition prior to an event or environment or condition where the individual is likely to be or known to be exposed to the allergen.

In certain cases, an individual is receiving, has received, and/or will receive an effective dosage of one or more compositions of the disclosure, but the individual will also receive another medical composition for the anaphylaxis. In some cases, the other medical composition may be one or more doses of an antihistamine, steroids epinephrine, or a combination thereof, for example.

In cases wherein an individual has at least one symptom of anaphylaxis, the individual may be provided with an effective amount of one or more compositions of the invention prior to and/or after the appearance of anaphylaxis. When the individual is provided one of more compositions prior to the appearance of anaphylaxis, the onset of anaphylaxis may be delayed or completely inhibited and/or the severity of the anaphylaxis may be reduced, compared to the condition of the individual without having received the composition(s), for example.

In particular embodiments, an individual has been diagnosed with allergic reaction. An individual may be tested for allergic reaction by standard means in the art. For example, one can perform skin tests (where a small amount of a suspected allergen is placed on or below the skin to see if a reaction develops) and/or blood tests for antibodies to the allergen, such as using ELISA to measure IgE. Such test may be performed before or after it is known that the individual has one or more allergies or that the individual has had an allergic reaction.

### III. Allergic Reaction

Embodiments of the invention concern compositions for treatment and/or prevention or reduction in the risk of an allergic reaction of anaphylaxis. An allergic reaction is a hypersensitivity disorder of the immune system in which a person's immune system reacts to a normally harmless substance (an allergen), such as from the environment. Allergic reactions are characterized by excessive activation of mast cells and basophils by Immunoglobulin E (IgE), and the reaction results in an inflammatory response with a range from discomfort to being fatal.

In the invention, the allergic reaction is anaphylaxis, which is characterized by rapid onset and can be fatal. It typically causes a number of symptoms including an itchy rash, throat swelling, and low blood pressure, for example. Common causes include insect bites/stings, foods, and medications.

On a pathophysiologic level, anaphylaxis is caused by the release of mediators from mast cells, such as by the release of inflammatory mediators and cytokines from mast cells and basophils, typically due to an immunologic reaction but sometimes non-immunologic mechanism. In the immunologic mechanism, immunoglobulin E (IgE) binds to the antigen (the foreign material that provokes the allergic reaction). Antigen-bound IgE then activates FcεRI receptors on mast cells and basophils. This leads to the release of inflammatory mediators such as histamine. These mediators subsequently increase the contraction of bronchial smooth muscles, trigger vasodilation, increase the leakage of fluid from blood vessels, and cause heart muscle depression. Non-immunologic mechanisms involve substances that directly cause the degranulation of mast cells and basophils.

Anaphylaxis typically presents with many different symptoms over minutes or hours. The most common affected areas include the skin, respiratory system, gastrointestinal system, heart and vasculature, and central nervous system and often include more than one system or organ.

Skin symptoms usually include generalized hives, itchiness, flushing, and/or swelling of the afflicted tissues. The tongue may swell, and some experience a runny nose and swelling of the conjunctiva. The skin may also be blue tinted because of reduced oxygen. Respiratory symptoms include shortness of breath, wheezing, or stridor, hoarseness, pain with swallowing, and/or a cough. Cardiac symptoms include coronary artery spasm, myocardial infarction, dysrhythmia, cardiac arrest, changes in heart rate, and/or a drop in blood pressure or shock. Gastrointestinal symptoms may include crampy abdominal pain, diarrhea, vomiting, confusion, a loss of bladder control and/or pelvic pain similar to that of uterine cramps.

Anaphylaxis may be diagnosed based on clinical criteria, such as when within minutes or hours of exposure to an allergen there is involvement of the skin or mucosal tissue in addition to either respiratory difficulty or a low blood pressure. In certain cases anaphylaxis is diagnosed with two or more of the following symptoms: a. involvement of the skin or mucosa; b. respiratory difficulties; c. low blood pressure; and d. gastrointestinal symptoms. Low blood pressure after exposure to a known allergen may be involved. Diagnosis may include blood tests for tryptase or histamine (released from mast cells) for anaphylaxis because of insect stings or medications.

There are three main classifications of anaphylaxis, all of which may be preventable or reduced in severity with one or more compositions as disclosed herein: anaphylactic shock; biphasic anaphylaxis; and pseudoanaphylaxis or anaphylactoid reactions (which are a type of anaphylaxis that does not involve an allergic reaction but is due to direct mast cell degranulation and may be referred to as non-immune anaphylaxis).

In certain embodiments, an individual in need thereof is provided one or more compositions as disclosed herein but also is exposed to desensitization.

Individual with food allergies are suitable for exposure to one or more compositions as disclosed herein. Typical food allergens include milk, legumes (such as peanuts), shellfish, tree nuts, eggs, fish, soy, and wheat, for example. Severe cases are usually caused by ingesting the allergen, but some people experience a severe reaction upon contact and/or close proximity.

Individual with medication allergies are suitable for exposure to one or more compositions as disclosed herein. Any medication may potentially trigger allergic reaction, including anesthetics, β-lactam antibiotics, aspirin, NSAIDs, chemotherapy, vaccines, protamine and herbal preparations. Some medications (such as vancomycin, morphine, or x-ray contrast) cause anaphylaxis by directly triggering mast cell degranulation.

Individuals with venom allergies are suitable for exposure to one or more compositions as disclosed herein. Venom from stinging or biting animals (such as Hymenoptera (bees and wasps), jellyfish, sting ray) may induce anaphylaxis in susceptible people. Individuals with previous systemic reactions (anything more than a local reaction around the site of the sting) are at risk for future anaphylaxis, although some individuals have had no previous systemic reaction.

In some cases, people that have had one type of allergic reaction are also susceptible to having another type of allergic reaction, and these individual may receive effective amounts of one or more compositions as disclosed herein.

Allergic reaction symptoms can develop quickly, often within seconds or minutes. They may include the following: abdominal pain; abnormal (high-pitched) breathing sounds; anxiety; chest discomfort or tightness; cough; diarrhea; difficulty breathing; difficulty swallowing; dizziness or light-headedness; hives; itchiness; nasal congestion; nausea or vomiting; palpitations; skin redness; slurred speech; swelling of the face, eyes, or tongue; unconsciousness; wheezing; rapid pulse, arrhythmia; pulmonary edema; low blood pressure; blue skin; weakness; and/or wheezing.

U.S. Patent No. 8,099,167 describes methods and devices for treating anaphylaxis, anaphylactic shock, bronchial constriction, and/or asthma.

### IV. Compositions

Embodiments of the invention encompass compositions that are useful for preventing and/or reducing the risk or severity of anaphylaxis. Specific compositions are disclosed herein.

In particular embodiments, compositions as described herein are utilized in treatment and/or prevention of anaphylaxis.

The invention provides a method of preventing or reducing the risk or severity of anaphylaxis in an individual comprising delivering to the individual a compound selected from the group consisting of N-(1',2-dihydroxy-1,2'-binaphthalen-4'-yl)-4-methoxybenzenesulfonamide, N-(3,1'-Dihydroxy-[1,2']binaphthalenyl-4'-yl)-4-methoxy-benzenesulfonamide, N-(4,1'-Dihydroxy-[1,2']binaphthalenyl-4'-yl)-4-methoxy-benzenesulfonamide, N-(5,1'-Dihydroxy-[1,2']binaphthalenyl-4'-yl)-4-methoxy-benzenesulfonamide, N-(6,1'-Dihydroxy-[1,2']binaphthalenyl-4'-yl)-4-methoxy-benzenesulfonamide, N-(7,1'-Dihydroxy-[1,2']binaphthalenyl-4'-yl)-4-methoxy-benzenesulfonamide, N-(8,1'-Dihydroxy-[1,2']binaphthalenyl-4'-yl)-4-methoxy-benzenesulfonamide, 4-Bromo-N-(1,6'-dihydroxy-[2,2']binaphthalenyl-4-yl)-benzenesulfonamide, and a mixture thereof.

The compositions of the present invention may be obtained by any suitable means. In specific embodiments, the derivatives of the invention are provided commercially, although in alternate embodiments the derivatives are synthesized. The chemical synthesis of the derivatives may employ well known techniques from readily available starting materials. Such synthetic transformations may include, but are not limited to protection, de-protection, oxidation, reduction, metal catalyzed C-C cross coupling, Heck coupling or Suzuki coupling steps (see for example, March's Advanced Organic Chemistry: Reactions, Mechanisms, and Structures, 5th Edition John Wiley and Sons by Michael B. Smith and Jerry March).

### V. Embodiments for Targeting Stat3

STAT proteins, of which there are seven (1, 2, 3, 4, 5A, 5B and 6), transmit peptide hormone signals from the cell surface to the nucleus. Detailed structural information of STAT proteins currently is limited to Stat1 and Stat3. Stat1 was the first STAT to be discovered (Fu *et al.,* 1992) and is required for signaling by the Type I and II IFNs (Meraz et al,. 1996; Wiederkehr-Adam et al,. 2003; Durbin et al,. 1996; Haan *et al.,* 1999). Studies in Stat1-deficient mice (Meraz et al,. 1996; Durbin et al,. 1996; Ryan *et al.,* 1998) support an essential role for Stat1 in innate immunity, notably against viral pathogens. In addition, Stat1 is a potent inhibitor of growth and promoter of apoptosis (Bromberg and Darnell, 2000). Also, because tumors from carcinogen-treated wild-type animals grow more rapidly when transplanted into the Stat1-deficient animals than they do in a wild-type host, Stat1 contributes to tumor surveillance (Kaplan *et al.,* 1998).

Stat3 was originally termed acute-phase response factor (APRF) because it was first identified as a transcription factor that bound to IL-6-response elements within the enhancer-promoter region of various acute-phase protein genes (Akira, 1997). In addition to receptors for the IL-6 cytokine family, other signaling pathways are linked to Stat3 activation include receptors for other type I and type II cytokine receptors, receptor tyrosine kinases, G-protein-coupled receptors and Src kinases (Schindler and Darnell, 1995; Turkson *et al.,* 1998). Targeted disruption of the mouse Stat3 gene leads to embryonic lethality at 6.5 to 7.5 days (Takeda *et al.,* 1997) indicating that Stat3 is essential for early embryonic development possibly gastrulation or visceral endoderm function (Akira, 2000). Tissue-specific deletion of Stat3 using Cre-lox technology has revealed decreased mammary epithelial cell apoptosis resulting in delayed breast involution during weaning (Chapman *et al.,* 1999). Recent findings indicate that switching of the predominant STAT protein activated by a given receptor can occur when a STAT downstream of that receptor is genetically deleted (Costa-Pereira *et al.,* 2002; Qing and Stark, 2004). These findings suggest the possibility that the effect of Stat3 deletion in breast tissue may be mediated indirectly by increased activation of other STAT proteins, especially Stat5.

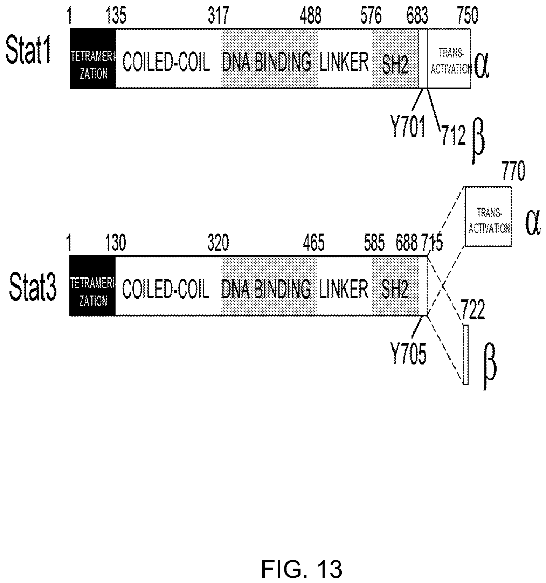

Stat1 and Stat3 isoforms. Two isoforms of Stat1 and Stat3 have been identified--α (p91 and p92, respectively) and β (p84 and p83, respectively) (Schindler *et al.,* 1992; Schaefer *et al.,* 1995; Caldenhoven *et al.,* 1996; Chakraborty *et al.,* 1996)--that arise due to alternative mRNA splicing (FIG. 13). In contrast to Stat1β (712 aa), in which the C-terminal transactivation is simply deleted, the 55 amino acid residues of Stat3α are replaced in Stat3 β by 7 unique amino acid residues at its C-terminus. Unlike Stat1 β, Stat3 β is not simply a dominant-negative of Stat3α (Maritano *et al.,* 2004) and regulates gene targets in a manner distinct from Stat3 β (Maritano *et al.,* 2004; Yoo *et al.,* 2002). Stat3α has been demonstrated to contribute to transformation in cell models and many human cancers including breast cancer. Stat3α was shown to be constitutively activated in fibroblasts transformed by oncoproteins such as v-Src (Yu *et al.,* 1995; Garcia and Jove, 1998) and to be essential for v-Src-mediated transformation (Turkson *et al.,* 1998; Costa-Pereira *et al.,* 2002). In contrast to Stat3α, Stat3β antagonized v-Src transformation mediated through Stat3α (Turkson *et al.,* 1998). Overexpression of a constitutively active form of Stat3α in immortalized rat or mouse fibroblasts induced their transformation and conferred the ability to form tumors in nude mice (Bromberg *et al.,* 1999). Stat3 has been shown to be constitutively activated in a variety of hematological and solid tumors including breast cancer (Dong *et al.,* 2003; Redell and Tweardy, 2003) as a result of either autocrine growth factor production or dysregulation of protein tyrosine kinases. In virtually all cases, the isoform demonstrating increased activity is Stat3α.

Targeting Stat3α while sparing Stat1. Given its multiple contributory roles to oncogenesis, Stat3 has recently gained attention as a potential target for cancer therapy (Bromberg, 2002; Turkson, 2004). While several methods of Stat3 inhibition have been employed successfully and have established proof-of-principle that targeting Stat3 is potentially beneficial in a variety of tumor systems including breast cancer in which Stat3 is constitutively activated (Epling-Burnette *et al.,* 2001; Yoshikawa *et al.,* 2001; Li and Shaw, 2002; Catlett-Falcone *et al.,* 1999; Mora *et al.,* 2002; Grandis *et al.,* 2000; Leong *et al.,* 2003; Jing *et al.,* 2003; Jing *et al.,* 2004; Turkson *et al.,* 2001; Ren *et al.,* 2003; Shao *et al.,* 2003; Turkson *et al.,* 2004; Uddin *et al.,* 2005); all have potential limitations for translation to clinical use for cancer therapy related to issues regarding delivery, specificity or toxicity.

Specific strategies that target Stat3 by identifying inhibitors of Stat3 recruitment and/or dimerization have been pursued by several groups (Turkson *et al.,* 2001; Ren *et al.,* 2003; Shao *et al.,* 2003; Uddin *et al.,* 2005; Song *et al.,* 2005; Schust *et al.,* 2006). As outlined below, this strategy has the potential to achieve specificity based on the observation that the preferred pY peptide motif of each STAT protein is distinct. When coupled to a small molecule approach, this strategy has the potential to overcome issues of delivery and toxicity.

Targeting Stat3α while sparing Stat3β. Some of the distinct biochemical features of Stat3β *vs.* Stat3α, notably constitutive activation and a 10-to-20 fold increased DNA binding affinity, have been attributed to the absence of the C-terminal transactivation domain (TAD) resulting in increased Stat3β dimer stability (Park *et al.,* 1996; Park *et al.,* 2000). Increased dimer stability likely results from higher binding affinity of the SH2 domain to pY peptide motifs when in the context of Stat3β compared to Stat3α because of reduced steric hindrance conferred by removal of the TAD. These differential biochemical features between Stat3 α and Stat3 β are exploited to develop a chemical compound that selectively targets Stat3 α, in some embodiments. This selectivity enhances the anti-tumor effect of such compounds, in certain cases, because they would spare Stat3β, which functions to antagonize the oncogenic functions of Stat3α.

In certain embodiments of the invention, specific therapies targeting Stat3 signaling are useful for treatment of anaphylaxis.

### VI. Combination Therapy

It is an aspect of this invention that a composition as disclosed herein is used in combination with another agent or therapy method, such as allergic reaction treatment. The composition(s) (which may or may not be a Stat3 inhibitor) may precede or follow the other agent treatment by intervals ranging from minutes to weeks, for example. In embodiments where the other agent and the composition of the invention are applied separately to an individual with anaphylaxis, such as upon delivery to an individual suspected of having anaphylaxis, known to have anaphylaxis, or at risk for having anaphylaxis, one would generally ensure that a significant period of time did not expire between the time of each delivery, such that the agent and composition of the invention would still be able to exert an advantageously combined effect on the individual.

For example, in such instances, it is contemplated that one may contact the individualwith one, two, three, four or more modalities substantially simultaneously (*i.e*., within less than about a minute) with the composition of the invention. In other aspects, one or more agents may be administered within about 1 minute, about 5 minutes, about 10 minutes, about 20 minutes about 30 minutes, about 45 minutes, about 60 minutes, about 2 hours, about 3 hours, about 4 hours, about 5 hours, about 6 hours, about 7 hours about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about 24 hours, about 25 hours, about 26 hours, about 27 hours, about 28 hours, about 29 hours, about 30 hours, about 31 hours, about 32 hours, about 33 hours, about 34 hours, about 35 hours, about 36 hours, about 37 hours, about 38 hours, about 39 hours, about 40 hours, about 41 hours, about 42 hours, about 43 hours, about 44 hours, about 45 hours, about 46 hours, about 47 hours, to about 48 hours or more prior to and/or after administering the composition of the invention. In certain other embodiments, an agent may be administered within of from about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20, to about 21 days prior to and/or after administering the composition of the invention, for example. In some situations, it may be desirable to extend the time period for treatment significantly, such as where several weeks (*e.g*., about 1, about 2, about 3, about 4, about 5, about 6, about 7 or about 8 weeks or more) lapse between the respective administrations. In some situations, it may be desirable to extend the time period for treatment significantly, such as where several months (*e.g*., about 1, about 2, about 3, about 4, about 5, about 6, about 7 or about 8 weeks or more) lapse between the respective administrations.

Various combinations may be employed, the composition of the invention is "A" and the secondary agent, which can be any other cancer therapeutic agent, is "B":
A/B/A B/A/B B/B/A A/A/B A/B/B B/A/A A/B/B/B B/A/B/B
B/B/B/A B/B/A/B A/A/B/B A/B/A/B A/B/B/A B/B/A/A
B/A/B/A B/A/A/B A/A/A/B B/A/A/A A/B/A/A A/A/B/A

Administration of the therapeutic compositions of the present invention to a patient will follow general protocols for the administration of drugs, taking into account the toxicity. It is expected that the treatment cycles would be repeated as necessary.

Exemplary combination therapies include antihistamines, steroids, epinephrine, and so on.

### VII. Pharmaceutical Compositions

Pharmaceutical compositions of the present invention comprise an effective amount of a composition as disclosed herein dissolved or dispersed in a pharmaceutically acceptable carrier. The phrases "pharmaceutical" or "pharmacologically acceptable" refer to molecular entities and compositions that do not produce an adverse, allergic or other untoward reaction when administered to an animal, such as, for example, a human, as appropriate. The preparation of a pharmaceutical composition that in some cases contains at least one Stat3 inhibitor of the invention, and in some cases an additional active ingredient, will be known to those of skill in the art in light of the present disclosure, as exemplified by Remington's Pharmaceutical Sciences, 18th Ed. Mack Printing Company, 1990. Moreover, for animal (*e.g*., human) administration, it will be understood that preparations should meet sterility, pyrogenicity, general safety and purity standards as required by FDA Office of Biological Standards.

As used herein, "pharmaceutically acceptable carrier" includes any and all solvents, dispersion media, coatings, surfactants, antioxidants, preservatives (*e.g*., antibacterial agents, antifungal agents), isotonic agents, absorption delaying agents, salts, preservatives, drugs, drug stabilizers, gels, binders, excipients, disintegration agents, lubricants, sweetening agents, flavoring agents, dyes, such like materials and combinations thereof, as would be known to one of ordinary skill in the art (see, for example, Remington's Pharmaceutical Sciences, 18th Ed. Mack Printing Company, 1990, pp. 1289-1329). Except insofar as any conventional carrier is incompatible with the active ingredient, its use in the therapeutic or pharmaceutical compositions is contemplated.

The composition(s) may comprise different types of carriers depending on whether it is to be administered in solid, liquid or aerosol form, and whether it needs to be sterile for such routes of administration such as injection. The present invention can be administered intravenously, intradermally, intraarterially, intraperitoneally, intralesionally, intracranially, intraarticularly, intraprostaticaly, intrapleurally, intratracheally, intranasally, intravitreally, intravaginally, intrarectally, topically, intratumorally, intramuscularly, intraperitoneally, subcutaneously, subconjunctival, intravesicularlly, mucosally, intrapericardially, intraumbilically, intraocularally, orally, topically, locally, injection, infusion, continuous infusion, localized perfusion bathing target cells directly, *via* a catheter, *via* a lavage, in lipid compositions (*e.g*., liposomes), as an aerosol, or by other method or any combination of the forgoing as would be known to one of ordinary skill in the art (see, for example, Remington's Pharmaceutical Sciences, 18th Ed. Mack Printing Company, 1990).

The actual dosage amount of a composition of the present invention administered to an individual can be determined by physical and physiological factors such as body weight, severity of condition, the type of disease being treated, previous or concurrent therapeutic interventions, and the route of administration. The practitioner responsible for administration will, in any event, determine the concentration of active ingredient(s) in a composition and appropriate dose(s) for the individual subject.

In certain embodiments, pharmaceutical compositions may comprise, for example, at least about 0.1% of a composition. In other embodiments, the active compound may comprise between about 2% to about 75% of the weight of the unit, or between about 25% to about 60%, for example, and any range derivable therein. In other non-limiting examples, a dose may also comprise from about 0.1 mg/kg/body weight, 0.5 mg/kg/ body weight, 1 mg/kg/body weight, about 5 mg/kg/body weight, about 10 mg/kg/body weight, about 20 mg/kg/body weight, about 30 mg/kg/body weight, about 40 mg/kg/body weight, about 50 mg/kg/body weight, about 75 mg/kg/body weight, about 100 mg/kg/body weight, about 200 mg/kg/body weight, about 350 mg/kg/body weight, about 500 mg/kg/body weight, about 750 mg/kg/body weight, to about 1000 mg/kg/body weight or more per administration, and any range derivable therein. In non-limiting examples of a derivable range from the numbers listed herein, a range of about 10 mg/kg/body weight to about 100 mg/kg/body weight, *etc*., can be administered, based on the numbers described above. In certain embodiments of the invention, various dosing mechanisms are contemplated. For example, the composition may be given one or more times a day, one or more times a week, or one or more times a month, and so forth.

In any case, the composition may comprise various antioxidants to retard oxidation of one or more component. Additionally, the prevention of the action of microorganisms can be brought about by preservatives such as various antibacterial and antifungal agents, including, but not limited to parabens (*e.g*., methylparabens, propylparabens), chlorobutanol, phenol, sorbic acid, thimerosal or combinations thereof.

The composition may be formulated in a free base, neutral or salt form. Pharmaceutically acceptable salts include the salts formed with the free carboxyl groups derived from inorganic bases such as for example, sodium, potassium, ammonium, calcium or ferric hydroxides; or such organic bases as isopropylamine, trimethylamine, histidine or procaine.

In embodiments where the composition is in a liquid form, a carrier can be a solvent or dispersion medium comprising, but not limited to, water, ethanol, polyol (*e.g*., glycerol, propylene glycol, liquid polyethylene glycol, *etc*.), lipids (*e.g*., triglycerides, vegetable oils, liposomes) and combinations thereof. The proper fluidity can be maintained, for example, by the use of a coating, such as lecithin; by the maintenance of the required particle size by dispersion in carriers such as, for example, liquid polyol or lipids; by the use of surfactants such as, for example, hydroxypropylcellulose; or combinations thereof such methods. In many cases, it will be preferable to include isotonic agents, such as, for example, sugars, sodium chloride or combinations thereof.

Sterile injectable solutions are prepared by incorporating the instant invention in the required amount of the appropriate solvent with various amounts of the other ingredients enumerated above, as required, followed by filtered sterilization. Generally, dispersions are prepared by incorporating the various sterilized active ingredients into a sterile vehicle which contains the basic dispersion medium and/or the other ingredients. In the case of sterile powders for the preparation of sterile injectable solutions, suspensions or emulsion, the preferred methods of preparation are vacuum-drying or freeze-drying techniques which yield a powder of the active ingredient plus any additional desired ingredient from a previously sterile-filtered liquid medium thereof. The liquid medium should be suitably buffered if necessary and the liquid diluent first rendered isotonic prior to injection with sufficient saline or glucose. The preparation of highly concentrated compositions for direct injection is also contemplated, where the use of DMSO as solvent is envisioned to result in extremely rapid penetration, delivering high concentrations of the active agents to a small area.

The composition must be stable under the conditions of manufacture and storage, and preserved against the contaminating action of microorganisms, such as bacteria and fungi. It will be appreciated that endotoxin contamination should be kept minimally at a safe level, for example, less that 0.5 ng/mg protein.

In particular embodiments, prolonged absorption of an injectable composition can be brought about by the use in the compositions of agents delaying absorption, such as, for example, aluminum monostearate, gelatin or combinations thereof.

### VIII. Kits

Any of the compositions described herein may be comprised in a kit, and they are housed in a suitable container. The kits will thus comprise, in suitable container means, one or more compositions and, in some cases, an additional agent of the present invention. In some cases, there are one or more agents other than the composition of the disclosure that are included in the kit, such as one or more other agents for the treatment of anaphylaxis. In particular embodiments, there is an apparatus or any kind of means for the diagnosing of anaphylaxis.

The components of the kits may be packaged either in aqueous media or in lyophilized form. The container means of the kits will generally include at least one vial, test tube, flask, bottle, syringe or other container means, into which a component may be placed, and preferably, suitably aliquoted. Where there are more than one component in the kit, the kit also will generally contain a second, third or other additional container into which the additional components may be separately placed. However, various combinations of components may be comprised in a vial. The kits of the present invention also will typically include a means for containing the composition, additional agent, and any other reagent containers in close confinement for commercial sale. Such containers may include injection or blow molded plastic containers into which the desired vials are retained.

Compositions may also be formulated into a syringeable composition. In which case, the container means may itself be a syringe, pipette, and/or other such like apparatus, from which the formulation may be applied to an infected area of the body, injected into an animal, and/or even applied to and/or mixed with the other components of the kit. However, the components of the kit may be provided as dried powder(s). When reagents and/or components are provided as a dry powder, the powder can be reconstituted by the addition of a suitable solvent. It is envisioned that the solvent may also be provided in another container means.

### EXAMPLES

The following examples are included to demonstrate preferred embodiments of the invention. It should be appreciated by those of skill in the art that the techniques disclosed in the examples that follow represent techniques discovered by the inventors to function well in the practice of the invention, and thus can be considered to constitute preferred modes for its practice. Examples 1-11 are reference examples.

### EXAMPLE 1

### EXEMPLARY MATERIALS AND METHODS

**Virtual ligand screening.** The inventors isolated the three-dimensional structure of the Stat3 SH2 domain from the core fragment structure of phosphorylated Stat3 homodimers bound to DNA (Becker *et al.,* 1998) deposited in the RCSB Protein Data Bank (PDB) databank (PDB code 1BG1) and converted it to be an Internal Coordinate Mechanics (ICM)- compatible system by adding hydrogen atoms, modifying unusual amino acids, making charge adjustments and performing additional cleanup steps. In addition, the inventors retrieved the coordinates of the Stat1 SH2 domain from the PDB databank (PDB code 1BF5) for use in computational selectivity analysis (Chen *et al.,* 1998). Commercial chemical databases (Chembridge, Asinex, ChemDiv, Enamine, Keyorganics and Life Chemicals) were chosen as sources of compounds for screening *in silico.* Selection was of the amide hydrogen of E638 within the site that binds the +3 residue (Q, C or T) within the pY-peptide ligand (Shao *et al.,* 2006) as the central point of the binding pocket, which consisted of a cube with dimensions 16.0 x 16.9 x 13.7 angstrom. In addition to the +3 binding site, this cube contained the pY residue binding site consisting mainly of R609 and K591 (Shao *et al.,* 2006) and a hydrophobic binding site consisting of Loop_{βC_βD} and Loop_{αB-αC}. Sequence alignment and overlay of the Stat3 and Stat1 structures revealed substantial differences in sequence of these loops; lack of their superimposition indicated that this region might serve as a selectivity filter (Cohen *et al.,* 2005). A flexible docking calculation (Totrov and Abagyan 1997) was performed in order to determine the global minimum energy score and thereby predict the optimum conformation of the compound within the pocket. A compound was selected for purchase and biochemical testing based on fulfilling the criteria of interaction analysis (CIA): 1) global minimum energy score ≤-30, 2) formation of a salt-bridge and/or H-bond network within the pY-residue binding site and 3) formation of a H-bond with or blocking access to the amide hydrogen of E638. Most, but not all, compounds also interacted with the hydrophobic binding site.

Stat3 SH2/pY-peptide binding assay. Stat3 binding assays were performed at 25°C with a BIAcore 3000 biosensor using 20mM Tris buffer pH 8 containing 2mM mercaptoethanol and 5% DMSO as the running buffer (Kim *et al.,* 2005). Phosphorylated and control non-phosphorylated biotinylated EGFR derived dodecapeptides based on the sequence surrounding Y1068 (Shao *et al.,* 2004) were immobilized on a streptavidin coated sensor chip (BIAcore inc., Picataway NJ). The binding of Stat3 was conducted in 20mM Tris buffer pH 8 containing 2mM β-mercaptoethanol at a flow rate of 10uL/min for 1-2 minute. Aliquots of Stat3 at 500nM were premixed with compound to achieve a final concentration of 1-1,000uM and incubated at 4°C prior to being injected onto the sensor chip. The chip was regenerated by injecting 10uL of 100mM glycine at pH 1.5 after each sample injection. A control (Stat3 with DMSO but without compound) was run at the beginning and the end of each cycle (40 sample injections) to ensure that the integrity of the sensor chip was maintained throughout the cycle run. The average of the two controls was normalized to 100% and used to evaluate the effect of each compound on Stat3 binding. Responses were normalized by dividing the value at 2 min by the response obtained in the absence of compounds at 2 min and multiplying by 100. IC₅₀ values were determined by plotting % maximum response as a function of log concentration of compound and fitting the experimental points to a competitive binding model using a four parameter logistic equation: R = R_{high} - (R_{high} - R_{low})/ (1 + conc/A1)^A2, where R = percent response at inhibitor concentration, R_{high} = percent response with no compound, R_{low}= percent response at highest compound concentration, A2 = fitting parameter (slope) and A1 = IC₅₀ (BIAevaluation Software version 4.1).

Immunoblot assay. The human hepatocellular carcinoma cell line (HepG2) was grown in 6-well plates under standard conditions. Cells were pretreated with compounds (0, 1, 3, 10, 30, 100 and 300uM) for 1 hour then stimulated under optimal conditions with either interferon gamma (IFN-γ; 30 ng/ml for 30 min) to activate Stat1 or interleukin-6 (IL-6; 30 ng/ml for 30 min) to activate Stat3 (30-31). Cultures were then harvested and proteins extracted using high-salt buffer, as described (Shao *et al.,* 2006). Briefly, extracts were mixed with 2X sodium dodecyl sulfate (SDS) sample buffer (125mmol/L Tris-HCL pH 6.8; 4% SDS; 20% glycerol; 10%2-mercaptoethanol) at a 1:1 ratio and heated for 5 minutes at 100°C. Proteins (20 µg) were separated by 7.5% SDS-PAGE and transferred to polyvinylidene fluoride (PVDF) membrane (Millipore, Waltham, MA) and immunoblotted. Prestained molecular weight markers (Biorad, Hercules, CA) were included in each gel. Membranes were probed serially with antibody against Stat1 pY⁷⁰¹ or Stat3 pY⁷⁰⁵ followed by antibody against Stat1 or Stat3 (Transduction labs, Lexington, KY) then antibody against β-actin (Abcam, Cambridge, MA). Membranes were stripped between antibody probing using Restore™ Western Blot Stripping Buffer (Thermo Fisher Scientific Inc., Waltham, MA) per the manufacturer's instructions. Horseradish peroxidase-conjugated goat-anti-mouse IgG was used as the secondary antibody (Invitrogen Carlsbad, CA) and the membranes were developed with enhanced chemiluminescence (ECL) detection system (Amersham Life Sciences Inc.; Arlington Heights, IL.).

Similarity screen. Three compounds identified in the initial virtual ligand screening (VLS)-Cpd3, Cpd30 and Cpd188-inhibited Stat3 SH2/pY-peptide binding and IL-6-mediated Stat3 phosphorylation and were chosen as reference molecules for similarity screening. A fingerprint similarity query for each reference compound was submitted to Molcart/ICM (Max Distance, 0.4). Similarity between each reference molecule and each database molecule was computed and the similarity results were ranked in decreasing order of ICM similarity score (Eckert and Bajorath 2007). The databases searched included ChemBridge, LifeChemicals, Enamine, ChemDiv, Asinex, AcbBlocks, KeyOrganics and PubChem for a total of 2.47 million compounds. All compounds identified were docked into the binding pocket of Stat3 SH2 domain *in silico.* Compounds that fulfilled CIA criteria were purchased and tested as described for compounds identified in the primary screen.

Electrophoretic Mobility Shift Assay (EMSA): EMSA was performed using the hSIE radiolabeled duplex oligonucleotide as a probe as described (Tweardy *et al.,* 1995). Briefly, high salt extracts were prepared from HepG2 cells incubated without or with IL- 6 (30ng/ml) for 30 minutes. Protein concentration was determined by Bradford Assay and 20ug of extract was incubated with compound (300uM) for 60 minutes at 37o C. Bound and unbound hSIE probe was separated by polyacrylamide gel electrophoresis (4.5%). Gels were dried and autoradiographed.

Molecular modeling. All 3-D configurations of the Stat3 SH2 domain complexed with compounds were determined by global energy optimization that involves multiple steps: 1) location of organic molecules were adjusted as a whole in 2 Å amplitude by pseudo- Brownian random translations and rotations around the molecular center of gravity, 2) the internal variables of organic molecules were randomly changed. 3) coupled groups within the Stat3 SH2 domain side-chain torsion angles were sampled with biased probability shaking while the remaining variables of the protein were fixed, 4) local energy minimizations were performed using the Empirical Conformation Energy Program for Peptides type-3 (ECEPP3) in a vacuum (Nemethy *et al.,* 1992) with distance-dependent dielectric constant ε=4r, surface-based solvent energy and entropic contributions from the protein side chains evaluated added and 5) conformations of the complex, which were determined by Metropolis criteria, were selected for the next conformation-scanning circle. The initial 3-dimensional configuration of the Stat1 SH2 domain in a complex with each compound was predicted and generated by superimposing, within the computational model, the 3-dimensional features of the Stat1 SH2 onto the 3-dimensional configuration of the Stat3 SH2 domain in a complex with each compound. The final computational model of Stat1 SH2 in a complex with each compound was determined by local minimization using Internal Coordinate Force Field (ICFF)-based molecular mechanics (Totrov and Abagyan 1997). The inventors computed the van der Waals energy of the complex of Stat1 or 3-SH2 bound with each compound using Lennard-Jones potential with ECEPP/3 force field (Nemethy *et al.,* 1992).

Confocal and high-throughput fluorescence microscopy. Confocal and highthroughput fluorescence microscopy (HTFM) of MEF/GFP-Stat3α cells were performed as described (Huang *et al.,* 2007). Briefly, for confocal fluorescence microscopy, cells were grown in 6-well plates containing a cover slip. For HTFM, cells were seeded into 96-well CC3 plates at a density of 5,000 cells/well using an automated plating system. Cells were cultured under standard conditions until 85-90% confluent. Cells were pretreated with compound for 1 hour at 37 °C then stimulated with IL-6 (200ng/ml) and IL- 6sR (250ng/ml) for 30 minutes. Cells were fixed with 4% formaldehyde in PEM Buffer (80 mM Potassium PIPES, pH 6.8, 5 mM EGTA pH 7.0, 2 mM MgCl₂) for 30 minutes at 4°C, quenched in 1 mg/ml of NaBH4 (Sigma) in PEM buffer and counterstained for 1 min in 4,6-diamidino-2-phenylindole (DAPI; Sigma; 1mg/ml) in PEM buffer. Cover slips were examined by confocal fluorescent microscopy. Plates were analyzed by automated HTFM using the Cell Lab IC Image Cytometer (IC100) platform and CytoshopVersion 2.1 analysis software (Beckman Coulter). Nuclear translocation is quantified by using the fraction localized in the nucleus (FLIN) measurement (Sharp *et al.,* 2006).

### EXAMPLE 2

### IDENTIFICATION BY VLS OF COMPOUNDS THAT BLOCKED STAT3 BINDING TO ITS PHOSPHOPEPTIDE LIGAND AND INHIBITED IL-6-MEDIATED PHOSPHORYLATION OF STAT3

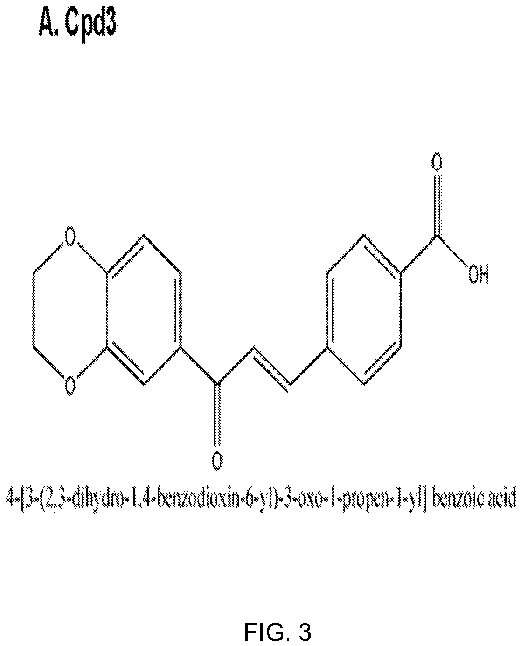

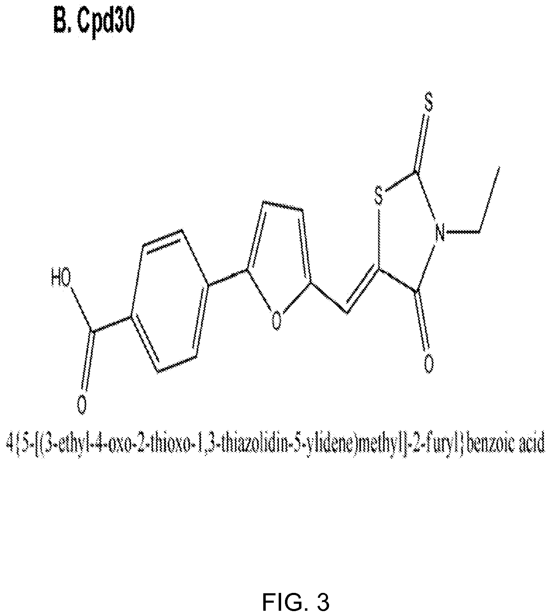

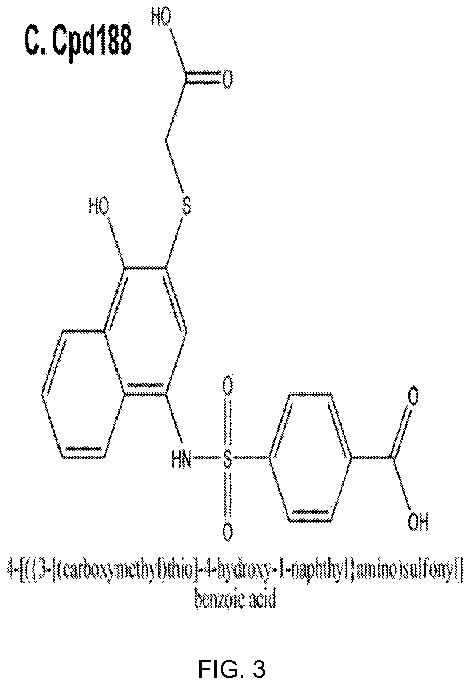

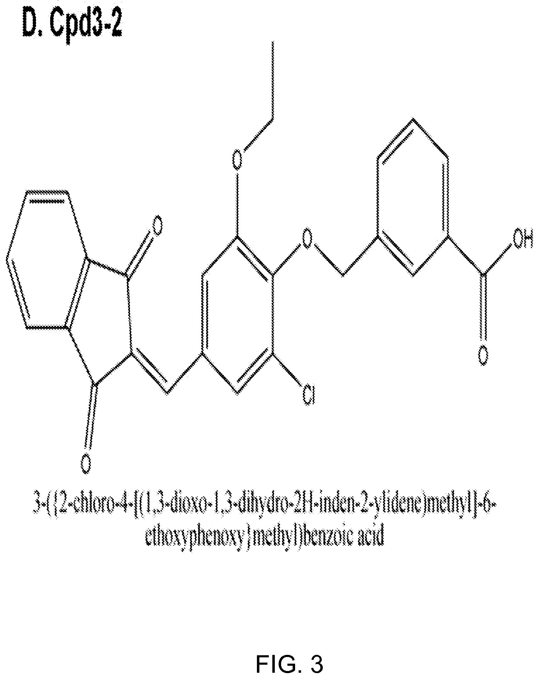

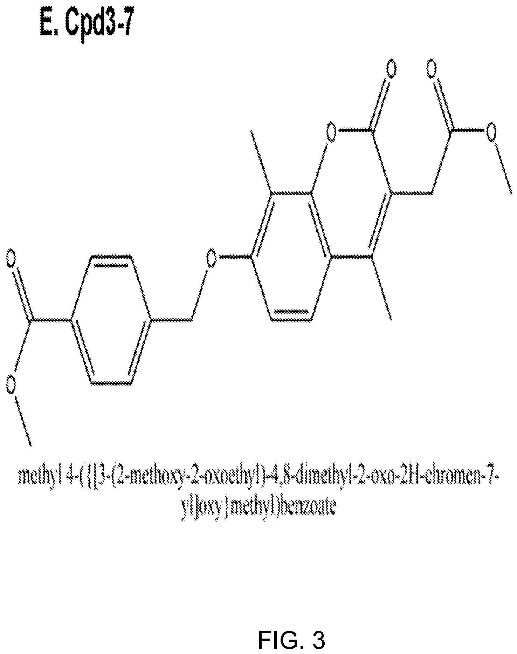

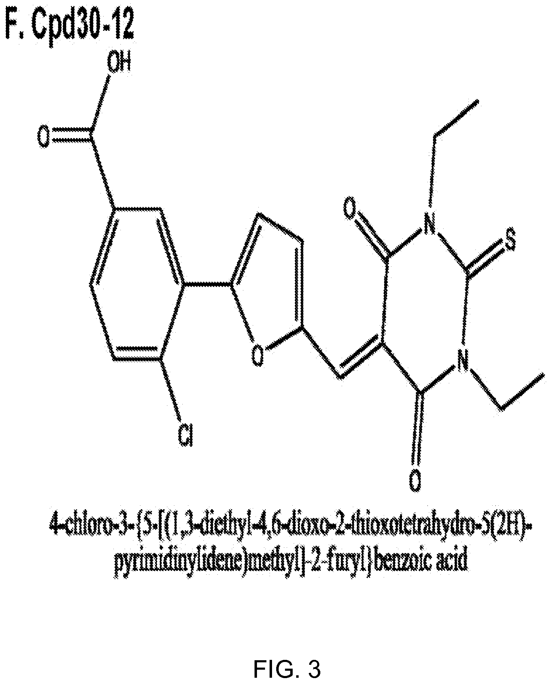

The VLS protocol was used to evaluate a total of 920,000 drug-like compounds. Of these, 142 compounds fulfilled CIA criteria. These compounds were purchased and tested for their ability to block Stat3 binding to its phosphopeptide ligand in a surface plasmon resonance (SPR)-based binding assay and to inhibit IL-6-mediated phosphorylation of Stat3. SPR competition experiments showed that of the 142 compounds tested, 3 compounds-Cpd3, Cpd30 and Cpd188-were able to directly compete with pY-peptide for binding to Stat3 with IC₅₀ values of 447, 30, and 20 µM, respectively (FIGS. 1 and 3; Table 4).

**Table 4. IC₅₀ values (µM) of 6 active compounds**

| **Assay** | **Cpd3** | **Cpd30** | **Cpd188** | **Cpd3-2** | **Cpd3-7** | **Cpd30-12** |
|---|---|---|---|---|---|---|
| **SPR** | 447¹ | 30 | 20 | 256 | 137 | 114 |
| **pStat3** | 91 | 18 | 73 | 144 | 63 | 60 |
| **HTM** | 131 | 77 | 39 | 150 | 20 | >300 |

| | | | | | | |
|---|---|---|---|---|---|---|
| ¹Data presented are the mean or mean ± SD; ND = not determined. | | | | | | |

In addition, each compound inhibited IL-6-mediated phosphorylation of Stat3 with IC50 values of 91, 18 and 73 µM respectively (FIG. 2; Table 4).

Similarity screening with Cpd3, Cpd30 and Cpd188 identified 4,302 additional compounds. VLS screening was performed with each of these compounds, which identified 41 compounds that fulfilled CIA criteria; these were purchased and tested. SPR competition experiments showed that of these 41 compounds, 3 compounds-Cpd3-2, Cpd3-7 and Cpd30-12-were able to directly compete with pY-peptide for binding to Stat3 with IC₅₀ values of 256, 137 and 114 µM, respectively (FIGS. 1 and 3; Table 4). In addition, each compound inhibited IL-6-mediated phosphorylation of Stat3 with IC50 values of 144, 63 and 60 µM, respectively (FIG. 2; Table 4).

### EXAMPLE 3

### COMPOUND-MEDIATED INHIBITION OF LIGAND-STIMULATED PHOSPHORYLATION OF STAT3 IS SPECIFIC FOR STAT3 VS. STAT1

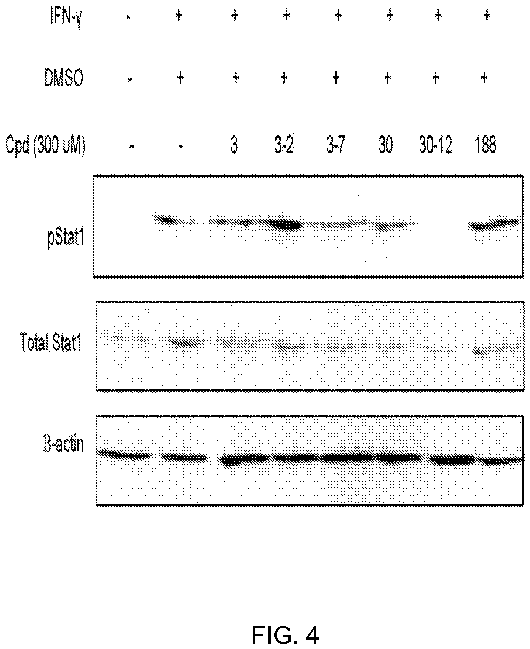

While Stat3 contributes to oncogenesis, in part, through inhibition of apoptosis, Stat1 is anti-oncogenic; it mediates the apoptotic effects of interferons and contributes to tumor surveillance (Kaplan *et al.,* 1998; Ramana *et al.,* 2000). Consequently, compounds that target Stat3 while sparing Stat1, leaving its anti-oncogenic functions unopposed, may result in a synergistic anti-tumor effect. To assess the selectivity of the compounds for Stat3 *vs.* Stat1, HepG2 cells were incubated with Cpd3, Cpd30, Cpd188, Cpd3-2, Cpd3- 7, and Cpd30-12 (300 µM) for 1 hour at 37°C before IFN-γ stimulation (FIG. 4). Only treatment with Cpd30-12 blocked Stat1 phosphorylation while each of the other five compounds-Cpd3, Cpd30, Cpd188, Cpd3-2 and Cpd3-7-did not. Thus, five of the six compounds identified were selective and inhibited ligand-stimulated phosphorylation of Stat3 but not Stat1.

### EXAMPLE 4

### SEQUENCE ANALYSIS AND MOLECULAR MODELING OF THE INTERACTION OF EACH COMPOUND WITH THE STAT3 VS. STAT1 SH2 DOMAIN

To understand at the molecular level the basis for the selectivity of Cpds 3, 30, 188, 3-2 and 3-7 and the absence of selectivity in the case of Cpd 30-12, the amino acid sequence and available structures of the Stat1 and Stat3 SH2 domain were compared and also it was examined how each compound interacted with both. Sequence alignment revealed identity in the residues within Stat3 and Stat1 corresponding to the binding site for the pYresidue and the +3 residue (FIG. 5A). In addition, overlay of the Stat3 and Stat1 SH2 structures revealed that the loops that contained these binding sites were superimposed (FIG. 5B). In contrast, sequence alignment revealed substantial differences in the sequence of the regions of the SH2 domain corresponding to the loops forming the hydrophobic binding site (FIG. 5A). In addition, review of the overlay of Stat3 and Stat1 SH2 domains revealed that, in contrast to the close apposition of the two loops of Stat3 that form the hydrophobic binding site, the corresponding two loops of Stat1 are not closely apposed to form a pocket (FIG. 5B).

Review of computational models of Cpd3, Cpd30, Cpd188, Cpd3-2 and Cpd3-7 in a complex with the Stat3 SH2 domain revealed that each has significant interactions with the Stat3 SH2 domain binding pocket at all three binding sites, the pY-residue binding site, the +3 residue binding site and the hydrophobic binding site (FIGS. 6A, B, C, D, and E). In contrast, Cpd30-12 interacts with the pY-residue binding site and blocks access to the +3 residue-binding site but does not interact with or block access to the hydrophobic binding site (FIG. 6F). In addition, van der Waals energies of the 5 selective compounds were much more favorable for their interaction with the loops of Stat3 forming the hydrophobic binding site than with corresponding loops of Stat1 (FIG. 5C). Thus, computer modeling indicated that activity of compounds against Stat3 derives from their ability to interact with the binding sites for the pY and the +3 residues within the binding pocket, while selectivity for Stat3 *vs.* Stat1 derives from the ability of compounds to interact with the hydrophobic binding site within the Stat3 SH2 binding pocket, which served as a selectivity filter.

### EXAMPLE 5

### INHIBITION OF NUCLEAR TRANSLOCATION OF PHOSPHORYLATED STAT3 BY CPD3, CPD30, CPD188, CPD3-2 AND CPD3-7 ASSESSED BY HTFM

Following its phosphorylation on Y705, Stat3 undergoes a change in conformation from head-to-head dimerization mediated through its N-terminal oligomerization domain to tail-to-tail dimerization mediated by reciprocal SH2/pY705-peptide ligand interactions. This conformational change is followed by nuclear accumulation. Compounds that targeted SH2/pY-peptide ligand interactions of Stat3 would be expected to inhibit nuclear accumulation of Stat3. To determine if this was the case with the compounds herein, a nuclear translocation assay (FIG. 7) was employed using murine embryonic fibroblast (MEF) cells that are deficient in endogenous Stat3 but constitutively express GFP-tagged Stat3α at endogenous levels, MEF/GFP-Stat3 α (Huang *et al.,* 2007). Preincubation of MEF/GFP-Stat3 α cells with Cpd3, Cpd30, Cpd188, Cpd3-2 and Cpd3-7, but not Cpd30- 12, blocked ligand-mediated nuclear translocation of GFP-Stat3 α with IC₅₀ values of 131, 77, 39, 150 and 20 µM (FIG. 7 and Table 4).

### EXAMPLE 6

### DESTABILIZATION OF STAT3-DNA COMPLEXES BY CPD3 AND CPD3-7

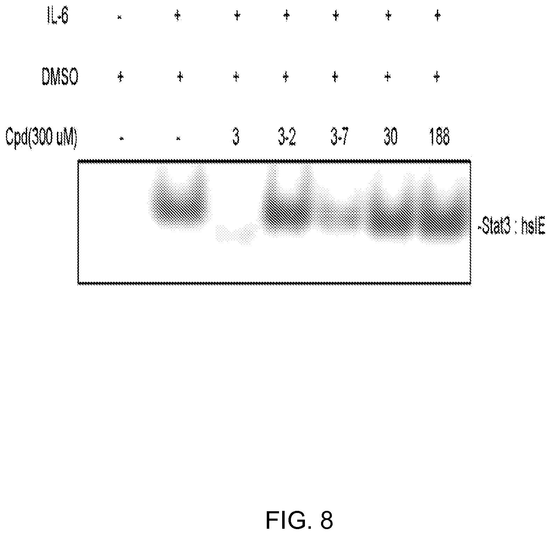

Once in the nucleus, Stat3 dimers bind to specific DNA elements to activate and, in some instances, repress gene transcription. Tyrosine-phosphorylated dodecapeptides based on motifs within receptors that recruit Stat3 have previously been shown to destabilize Stat3 (Chakraborty *et al.,* 1999; Shao *et al.,* 2003). Compounds that bind to the phosphopeptide-binding site of Stat3 might be expected to do the same. To determine if this was the case for any of the identified compounds, extracts of IL-6-stimulated HepG2 cells were incubated in binding reactions containing radiolabeled hSIE (FIG. 8) and each of the five selective compounds (300 µM). Incubation with Cpd3 or Cpd3-7 reduced the amount of hSIE shifted by half or greater. The other compounds did not have a detectable effect on the Stat3:hSIE band intensity. Thus, 2 of the 5 selective compounds destabilized Stat3:hSIE complexes.

### EXAMPLE 7

### EXEMPLARY APPROACH FOR STAT3 INHIBITORS FOR CANCER STEM CELLS

In the field of Stat3 probe development the inventors have focused on small molecule Stat3 probes (Xu et al., 2009), and several features of the small molecule program are useful, including: 1) a clearly defined mode of action of these probes: they target the Stat3 Src-homology (SH) 2 domain that is involved in 2 steps in the Stat3 activation pathway; 2) their specificity of action; and 3) the potential for using lead probes identified so far to identify probes with 2-to-3 logs greater activity based on recent and exemplary SAR analysis and medicinal chemistry considerations outlined below.

In specific embodiments, compound affinity is improved upon gaining a log greater affinity upon moving from 1^{st} generation to 2nd generation probes using 3-D pharmacophore analysis. In addition, selectivity is improved through modeling embodiments, in particular through identification of a distinct hydrophobic binding domain in the phosphopeptide binding pocket of Stat3 SH2 *vs.* the Stat1 SH2 (Xu et al., 2009).

Identification of 1st generation Stat3 chemical probes. To develop chemical probes that selectively target Stat3, the inventors virtually screened 920,000 small drug-like compounds by docking each into the peptide-binding pocket of the Stat3 SH2 domain, which consists of three sites-the pY-residue binding site, the +3 residue-binding site and a hydrophobic binding site, which served as a selectivity filter (Xu et al., 2009). Three compounds (Cpd3, Cpd30 and Cpd188) satisfied criteria of interaction analysis, competitively inhibited recombinant Stat3 binding to its immobilized pY-peptide ligand and inhibited IL-6-mediated tyrosine phosphorylation of Stat3. These compounds were used in a similarity screen of 2.47 million compounds, which identified 3 more compounds (Cpd3-2, Cpd3- 7 and Cpd30-12) with similar activities. Examinations of the 6 active compounds for the ability to inhibit IFN-γ-mediated Stat1 phosphorylation revealed that all but Cpd30-12 were selective for Stat3. Molecular modeling of the SH2 domains of Stat3 and Stat1 bound to compound revealed that compound interaction with the hydrophobic binding site was the basis for selectivity. All 5 selective compounds inhibited nuclear-tocytoplasmic translocation of Stat3, while 3 of 5 compounds (Cpd3, Cpd30 and Cpd188) induced apoptosis preferentially of exemplary breast cancer cell lines with constitutive Stat3 activation.

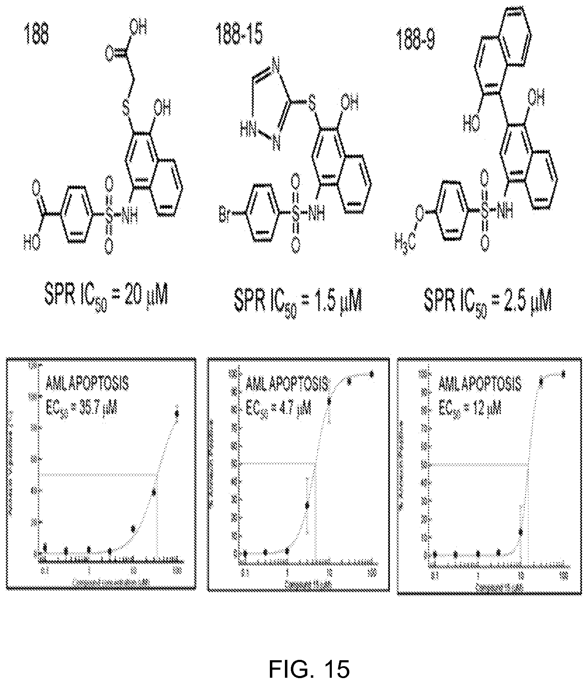

Identification of 2nd generation Stat3 chemical probes. The similarity screening described above did not yield any hits using Cpd188, the most active of the 3 lead compounds, as the query compound. Consequently, the inventors repeated 2-D similarity screening using the scaffold of Cpd188 as the query structure and the Life Chemicals library, which yielded 207 hits. 3-D pharmacophore analysis was performed on these 207 compounds using Ligand Scout and the top 39 scoring compounds were purchased and tested for inhibition of Stat3 binding to its phosphopeptide ligand by SPR. All but six of these 39 compounds have measurable SPR IC50s, with 19 having IC50 values equal to or less than the parent compound and 2 (Cpd188-9 and Cpd188-15) having IC50 values one log lower. Examination of these 19 compounds has revealed a statistically significant correlation between 3-D pharmacophore scores and SPR IC50s and as well as 3-D pharmacophore score and IC50s for inhibition of ligand-mediated cytoplasmic-to-nuclear translocation. In addition, both Cpd188-9 and Cpd188-15 exhibited a log greater activity in inducing human leukemic cell line apoptosis than the parent Cpd188 (FIG. 15). In addition, Cpd188-38 exhibited a 2 logs greater activity than parent Cpd188 in inhibiting cytoplasmic-to-nuclear translocation in HTFM assay, while Cpd188-15 exhibited a 1 log greater activity than parent Cpd188 in decreasing MSFE (Table 5). Furthermore, several of the second-generation 188-like compounds represent a substantial improvement over Cpd188 from a medicinal chemistry, metabolism and bioavailability standpoint. In particular, Cpd188-9 lacked both carboxyl groups, which in particular cases improves cell permeability and/or the thioether group, which is subject to oxidation. R2=0.2 P=0.013 (µM)

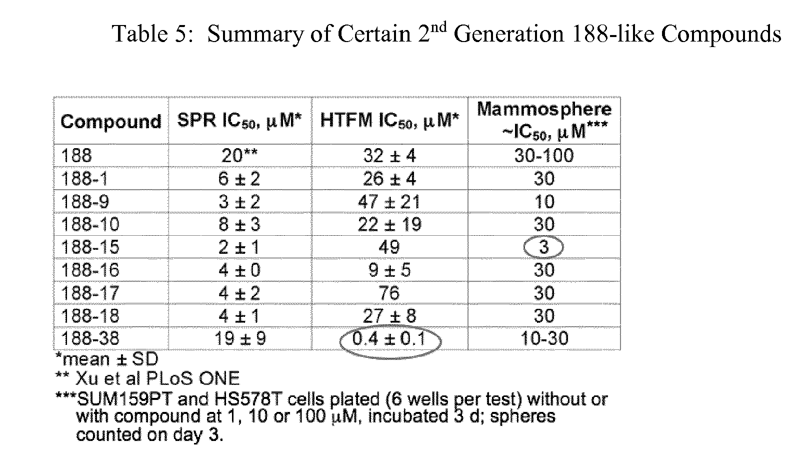

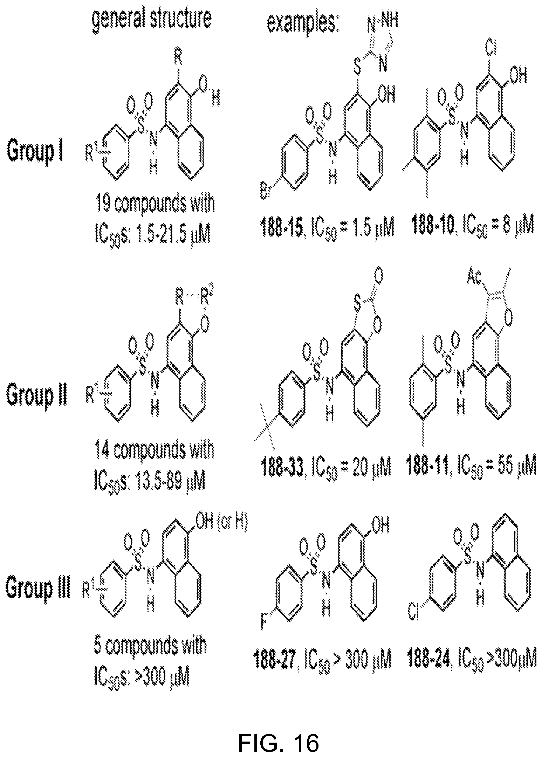

Structure-activity relationship (SAR) analysis of 2nd generation Stat3 probes. All of the 39 second generation compounds described above, plus Cpd188 itself, are derivatives of N-naphth-1-yl benzenesulfamide. Upon careful analysis of their structure-activity relationships (SAR), the inventors found that most of these Cpd188-like compounds (38 out of 40: the rest of 2 are weak and will be described below in EXP ID) can be divided into three structural groups in a general trend of decreased activity, as shown in FIG. 16. Five compounds in Group III are actually the parents of compounds in Groups I and II. Addition of a variety of groups (the -R group highlighted in red in the general structure of Group I in FIG. 16), such as a triazole-3-yl-mercapto (188-15) or a chloro (188-10) group, to the 3-position of the naphthylamine ring led to the Group I compounds, which are the most potent series of Stat3 probes. In a specific embodiment, this is the most important contributor to the inhibitory activity: a total of eight 3-substituents are found in Group I compounds, which invariably enhance the activity by several orders of magnitude.

Most Stat3 probes in Group II contain a 5- membered ring that combines the 3-R and 4-OR2 groups, such as a furan (188-11). However, the compounds in this group are, in average, ∼5x less active than the Group I compounds, which indicates that in certain aspects the H atom of the 4-hydroxy group (highlighted in blue in the general structure of Group I in FIG. 16) is important, *e.g.,* involved in a favorable H-bond with the protein. Lacking the ability to form the H-bond attributes to the weaker activities of Group II probes, in particular cases. These considerations underlie a medicinal chemistry approach outlined below.

### EXAMPLE 8

### MEDICINAL CHEMISTRY FOR SYNTHESIS OF 3^{rd} GENERATION 188-LIKE SULFAMIDE STAT3 PROBES

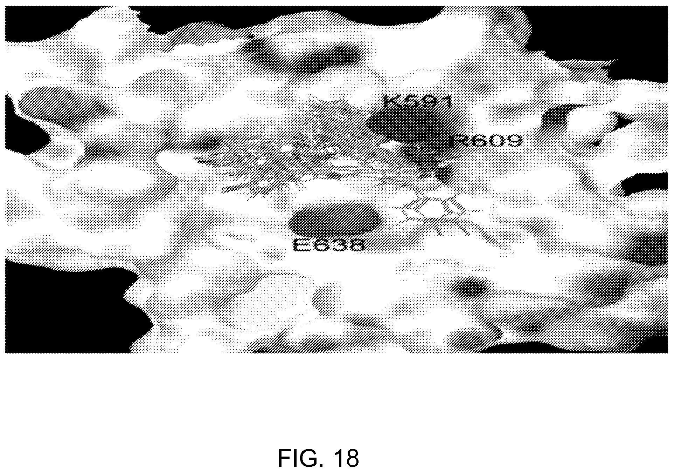

The crystal structure of Stat3 shows that the SH2 domain has a large, widely dispersed and generally shallow binding area with several valleys and hills that recognize the pY-peptide ligand (FIG. 18). Structure-based molecular modeling (docking) was useful in identifying the contribution of the hydrophobic binding surface of the Stat3 SH2 domain as a selectivity filter (Xu et al., 2009). However, different docking programs gave distinct binding poses for the same probe over the binding surface with similar predicted binding affinities. The inventors therefore in particular embodiments, based on initial SAR results outlined above, use traditional medicinal chemistry to further carry out an exemplary comprehensive structure activity relationship study, to optimize the activity as well as the selectivity of this novel class of sulfamide probes of Stat3. Compound 188-15 serves as a scaffold for making the new generation compounds, as shown schematically (FIG. 16).

In addition, chemistry for making these compounds is straightforward with a good yield, involving the reaction of a sulfonyl chloride with an aniline/amine, which can be either obtained commercially or synthesized readily.

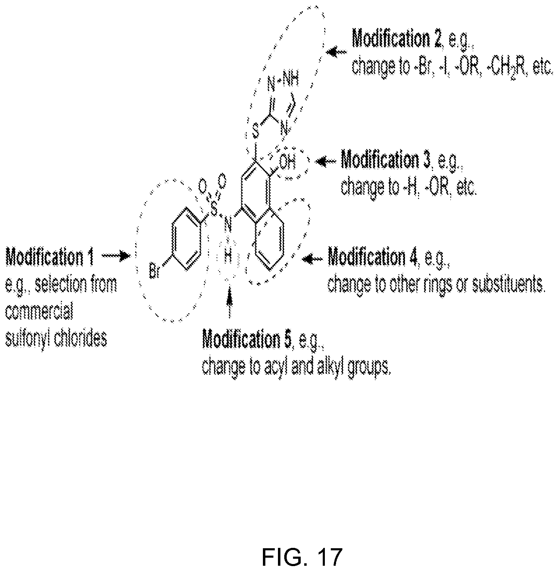

For the proposed modifications described below, one can consult FIG. 17. EXP IA. Modification 1. Since almost all of the 2^{nd} generation probes contain a phenylsulfonyl group, the first step towards activity optimization focuses on synthesizing a series of compounds that have a larger (*e.g*., bicyclic or tricyclic) or an alkyl sulfonyl group. The general synthetic route is shown as follows:

There are about 4,300 commercially available sulfonyl chlorides, among which 25, such as those shown above, are selected to make probes. Aniline 2, which is the amine component of compound 188-10 (FIG. 16), one the most active probes, is readily made in a simple two step reaction from nitro compound 1. One can first make 25 (for example) compounds and test their activities in an *in vitro* rapid throughput SPR and *in vivo* HTFM assays. Based on the outcomes of structure-activity relationship study, more compounds can be designed and synthesized and tested in an iterative manner until optimization of this modification.

EXP IB. Modification 2. Next, one can modify the 3-substituent of the naphthylamine ring, based on either the structure of compound 188-15, for example. Prior SAR studies demonstrated this substituent is useful to the activity of this class of probes, in certain embodiments. However, a total of 8 groups at this position with a huge difference in size, from a single atom Cl to a large, bicyclic benzothiazole-2-ylmercapto group, showed similar activities. This feature indicates that in certain embodiments modifications at this position should be more focused on other properties, such as electrostatic interactions with the protein, as exemplified below. In addition, many of these groups are thioethers, which may be subjected to oxidation/degradation *in vivo* and lead to an unfavorable pharmacokinetic profile, in particular aspects. The central -S- atom is changed to a more metabolically stable isosteres, such as -CH₂-, -NH-, and -O-, in certain cases. In certain aspects one can synthesize the following compounds to optimize the 3-substituent:

The synthesis is also started from 1, in certain cases. Regio-selective halogenation and formylation at the 3-position gives rise to two compounds, *i.e*., bromo- or iodo-compound 3 and aldehyde 4, which are versatile, common starting compounds for introducing a wide range of substituents at this position (*e.g*., those listed above).

Moreover, the crystal structure of Stat3 SH2 domain also provides strong evidence that more compounds with different electrostatic properties are useful for characterization. The electrostatic molecular surface of the protein shows two distinct features, as shown in FIG. 18. The first one is the negatively charged Glu638 surface stands out in the center. Next, of particular interest is a positively charged area, composed of Arg609 and Lys591 located in the edge of the domain, which is actually the pY (phosphorylated tyrosine) binding site of the receptor. The inventors also found that introducing a negatively charged group targeting the pY binding site leads to particularly active probes, in certain embodiments. For example, the docking study of the 3-phosphomethyl compound 5 (R = CH₂PO₃ ²⁻) showed all of the phosphonate groups of the 20 docking poses are tightly clustered together and located in the pY binding site, indicating strong electrostatic and H-bond interactions with the residues Arg609 and Lys591 (FIG. 18).

EXP IC. Modifications 3 and 4. Collectively, Modifications 3 and 4 test the effects of changing the substituents at the 4, 5, and 6- positions. The -OH at 4-position may be superior to -OR, in certain aspects. One can test whether the H atom in -OH is responsible for a better activity by synthesizing compounds 6 (acylated or alkylated 5), as schematically shown below. In addition, dehydroxy compounds 7 may also be made, starting from 3-bromonaphthyl-1-amine.

Regarding the general synthetic methods for modifying positions 5 and 6, one can first synthesize about a dozen of these compounds in this category and if very active compounds emerge, one can make more compounds to optimize the activity for these two positions.

EXP ID. Modification 5. The only two compounds not included in the SAR analysis (due to a different 4-substituent) are shown here, as well as their inhibitory activities against Stat3:

Despite the weak activity, masking the polar H of the sulfamide for the second compound is favorable, in certain aspects, which provides an easy route to making more potent probes. One can therefore use the following method to make a series of N-acyl or N-alkyl sulfamides 5:

### EXAMPLE 9

### IDENTIFICATION OF STAT3-SELECTIVE CHEMICAL PROBES FROM SULFAMIDE COMPOUNDS SYNTHESIZED IN EXAMPLE 11

Each novel sulfamide compound is tested for the ability to inhibit Stat3 binding to its phosphopeptide ligand by SPR and the ability to block IL-6-stimulated cytoplasmic-to-nuclear translocation in the HTFM assay. Probes with activity in these assays equivalent to or greater than the most active 2nd generation compounds are tested for inhibition of IL-6-stimulated Stat3 phosphorylation and lack of ability to inhibit IFN-γ-stimulated Stat1 phosphorylation as outlined below.

EXP IIA. Stat3/pY-peptide SPR binding inhibition assay. Stat3 pY-peptide binding assays is performed at 25°C using a BIAcore 3000 biosensor as described (Xu et al., 2009). Briefly, phosphorylated and control nonphosphorylated biotinylated EGFR derived dodecapeptides based on the sequence surrounding Y1068 are immobilized on a streptavidin coated sensor chip (BIAcore Inc., Piscataway NJ). The binding of Stat3 is performed in 20mM Tris buffer pH 8 containing 2mM β-mercaptoethanol at a flow rate of 10uL/min for 1-2 minute. Aliquots of Stat3 at 500nM are premixed with compound to achieve a final concentration of 1-1,000uM and incubated at 4°C prior to being injected onto the sensor chip. The chip is regenerated by injecting 10uL of 100mM glycine at pH 1.5 after each sample injection. A control (Stat3 with DMSO but without compound) is run at the beginning and the end of each cycle (40 sample injections) to ensure that the integrity of the sensor chip is maintained throughout the cycle run. The average of the two controls is normalized to 100% and used to evaluate the effect of each compound on Stat3 binding. Responses are normalized by dividing the value at 2 min by the response obtained in the absence of compounds at 2 min and multiplying by 100. IC₅₀ values are determined by plotting % maximum response as a function of log concentration of compound and fitting the experimental points to a competitive binding model using a four parameter logistic equation: R = R_{high} - (R_{high} -R_{low})/ (1 + conc/A1)^{A2}, where R = percent response at inhibitor concentration, R_{high} = percent response with no compound, R_{low}= percent response at highest compound concentration, A2 = fitting parameter (slope) and A1 = IC₅₀ (BIAevaluation Software version 4.1).

EXP IIB. High throughput fluorescence microscopy (HTFM), cytoplasm-to-nucleus translocation inhibition assays. HTFM of MEF/GFP-Stat3α cells is performed to assess the ability of probes to inhibit GFP-Stat3 cytoplasmic-to-nuclear translocation, as described (Xu et al., 2009), using the robotic system available as part of the John S. Dunn Gulf Coast Consortium for Chemical Genomics at the University of Texas-Houston School of Medicine. Briefly, cells are seeded into 96-well CC3 plates at a density of 5,000 cells/well and cultured under standard conditions until 85-90% confluent. Cells are pre-treated with compound for 1 hour at 37 °C then stimulated with IL-6 (100ng/ml) and IL-6sR (150ng/ml) for 30 minutes. Cells are fixed with 4% formaldehyde in PEM Buffer (80 mM Potassium PIPES, pH 6.8, 5 mM EGTA pH 7.0, 2 mM MgCl₂) for 30 minutes at 4 °C, quenched in 1 mg/ml of NaBH₄ (Sigma) in PEM buffer and counterstained for 1 min in 4,6-diamidino-2-phenylindole (DAPI; Sigma; 1mg/ml) in PEM buffer. Plates are analyzed by automated HTFM using the Cell Lab IC Image Cytometer (IC100) platform and CytoshopVersion 2.1 analysis software (Beckman Coulter).

Nuclear translocation is quantified by using the fraction localized in the nucleus (FLIN) measurement. FLIN values are normalized by subtracting the FLIN for unstimulated cells then dividing this difference by the maximum difference (delta, Δ) in FLIN (FLIN in cells stimulated with IL-6/sIL-6R in the absence of compound minus FLIN of unstimulated cells). This ratio is multiplied by 100 to obtain the percentage of maximum difference in FLIN and is plotted as a function of the log compound concentration. The best-fitting curve and IC₅₀ value are determined using 4-Parameter LogisticModel/Dose Response/XLfit 4.2, IDBS software.

EXP IIC. Ligand-mediated pStat3 and pStat1 inhibition assays. Newly synthesized Stat3 probes with activity equivalent to or greater than parent compound 188 in the SPR and HTFM assays will be tested for the ability to selectively inhibit ligand-mediated phosphorylation of Stat3 as described (Xu et al., 2009). Briefly, human hepatocellular carcinoma cells (HepG2) are grown in 6-well plates and pretreated with compounds (0, 0.1, 0.3, 1, 3, 10, 30, 100 µM) for 1 hour then stimulated under optimal conditions with either interleukin-6 (IL-6; 30 ng/ml for 30 min) to activate Stat3 or interferon gamma (IFN-γ; 30 ng/ml for 30 min) to activate Stat1. Cells are harvested and proteins extracted using high-salt buffer, mixed with 2X sodium dodecyl sulfate (SDS) sample buffer (125mmol/L Tris-HCL pH 6.8; 4% SDS; 20% glycerol; 10%2-mercaptoethanol) at a 1:1 ratio then heated for 5 minutes at 100 °C. Proteins (20 µg) are separated by 7.5% SDS-PAGE and transferred to polyvinylidene fluoride (PVDF) membrane (Millipore, Waltham, MA) and immunoblotted. Membranes are probed serially with antibody against Stat1 pY701 or Stat3 pY705 followed by antibody against Stat1 or Stat3 (Transduction labs, Lexington, KY) then antibody against β-actin (Abcam, Cambridge, MA). Membranes are stripped between antibody probings using RestoreTM Western Blot Stripping Buffer (Thermo Fisher Scientific Inc., Waltham, MA) per the manufacturer's instructions. Horseradish peroxidase-conjugated goat-anti-mouse IgG is used as the secondary antibody (Invitrogen Carlsbad, CA) and the membranes are developed with enhanced chemiluminescence (ECL) detection system (Amersham Life Sciences Inc.; Arlington Heights, IL.). Band intensities are quantified by densitometry. The value of each pStat3 band is divided by its corresponding total Stat3 band intensity; the results are normalized to the DMSO-treated control value. This value was plotted as a function of the log compound concentration. The best-fitting curve is determined using 4-Parameter Logistic Model/Dose Response/XLfit 4.2, IDBS software and was used to calculate the IC₅₀ value.

EXP IID. Molecular modeling of probe-Stat3 interactions. The results of modeling of the binding of the first generation probe to the Stat3 *vs.* Stat1 SH2 domains suggested that the basis for experimental selectivity of probes for Stat3 *vs.* Stat1 rested on the ability of the probes to have greater interaction with the hydrophobic binding site within the pY-peptide binding pocket of Stat3 compared to Stat1. Thus, the hydrophobic binding site served as a selectivity filter. To test if this remains the case for newly synthesized 3rd generation probes, one can use 2 complementary docking programs GLIDE (Schrodinger) and ICM (MolSoft) to determine the lowest energy docking configuration of each probe within the pY-peptide binding domain of Stat3 and Stat1 SH2 domain. One can review the computational models of each probe in a complex with the Stat3 *vs*. Stat1 SH2 domain and, in particular, compare the van der Waals energies and determine if they are equivalent for their interaction with the Stat3 SH2 domain *vs*. the Stat1 SH2 domain. It was this calculation that determined the selectivity of 1st generation probes for Stat3 *vs*. Stat1. In particular, van der Waals energy calculations implicated residues that form the hydrophobic binding site (W623, Q635, V637, Y640 and Y657) as critical for this selectivity.

There is identification of probes with one log or greater activity than 2^{nd} generation probes in SPR, HTFM and pStat3 assays. Furthermore, in certain aspects some of the most active 3^{rd} generation probes that emerge from this analysis are selective for Stat3 *vs.* Stat1 based on their greater interaction with the hydrophobic binding site within the Stat3 *vs.* Stat1 SH2 pY-peptide binding pocket.

### EXAMPLE 10

### REFERENCE COMPOSITIONS

Reference composition(s) are provided in Table 7 below.

**TABLE 7**

| IDNUMBER | Structure | Formula structure | MW | LogP |
|---|---|---|---|---|
| F0808-0081 | 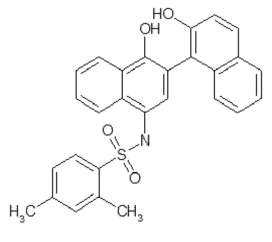 | C28H23NO4S | 469.5638 | 7.101 |
| F0808-0084 | 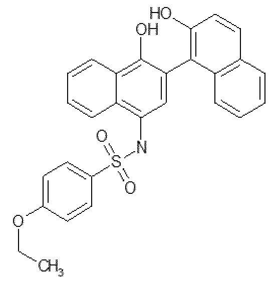 | C28H23NO5S | 485.5632 | 6.767 |
| F0808-0085 | 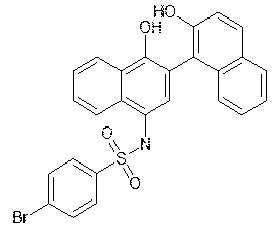 | C26H18BrNO4S | 520.4057 | 7.268 |
| F0808-0086 | 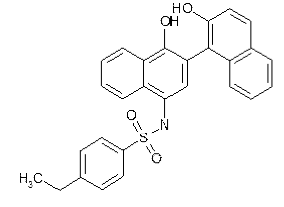 | C28H23NO4S | 469.5638 | 7.243 |
| F0808-0089 | 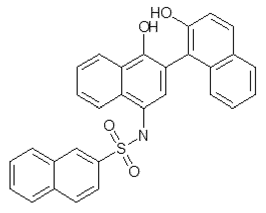 | C30H21NO4S | 491.5702 | 7.729 |
| F0808-0091 | 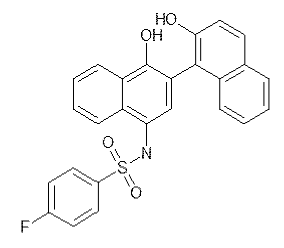 | C26H18FNO4S | 459.5001 | 6.623 |
| F0808-0092 | 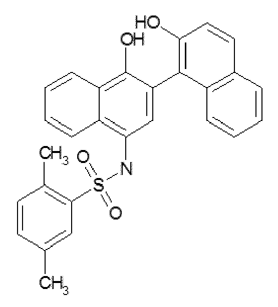 | C28H23NO4S | 469.5638 | 7.101 |
| F0808-0094 | 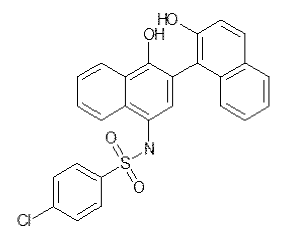 | C26H18ClNO4S | 475.9547 | 7.062 |
| F1269-0222 | 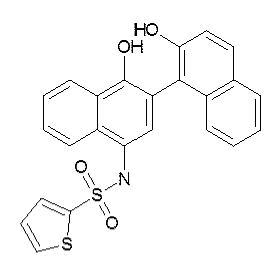 | C24H17NO4S2 | 447.5354 | 5.983 |
| F1269-2003 | 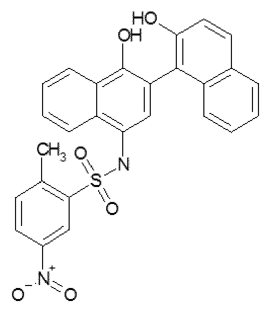 | C27H20N2O6S | 500.5343 | 6.738 |
| F1566-1138 | 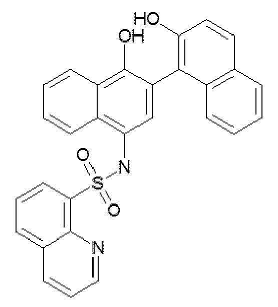 | C29H20N2O4S | 492.5578 | 6.67 |
| F5749-0001 | 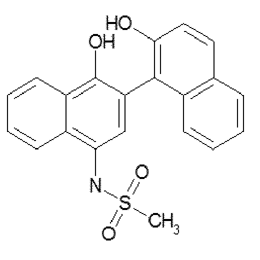 | C21H17NO4S | 379.4379 | 4.816 |
| F5749-0002 | 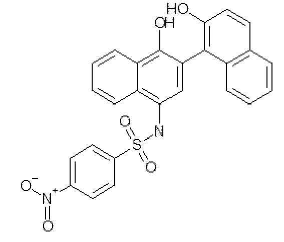 | C26H18N2O6S | 486.5072 | 6.405 |
| F5749-0003 | 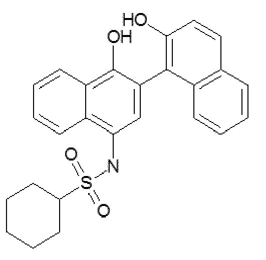 | C26H25NO4S | 447.5575 | 6.795 |
| F5749-0004 | 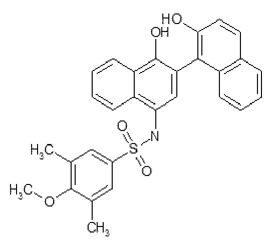 | C29H25NO5S | 499.5903 | 7.092 |
| F5749-0005 | 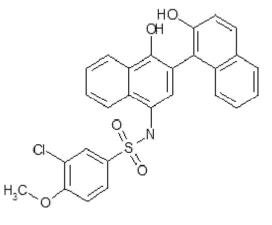 | C27H20ClNO5S | 505.9812 | 7.053 |
| F5749-0006 | 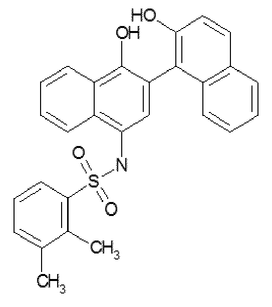 | C28H23NO4S | 469.5638 | 7.062 |
| F5749-0007 | 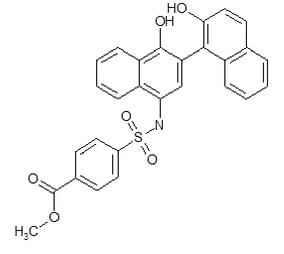 | C28H21NO6S | 499.5467 | 6.411 |
| F5749-0008 | 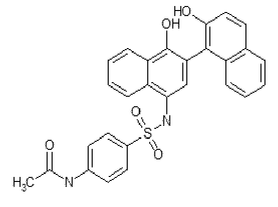 | C28H22N2O5S | 498.5619 | 5.761 |
| F5749-0009 | 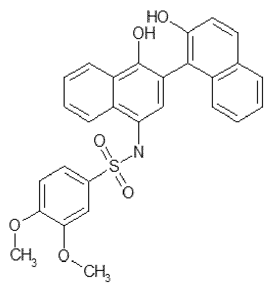 | C28H23NO6S | 501.5626 | 6.16874 |
| F5749-0010 | 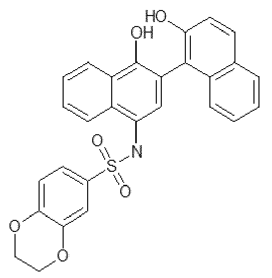 | C28H21NO6S | 499.5467 | 6.065 |
| F5749-0011 | 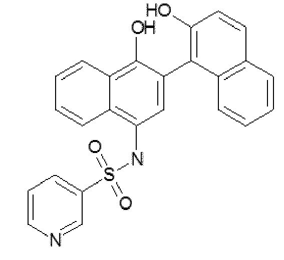 | C25H18N2O4S | 442.4972 | 5.237 |
| F5749-0012 | 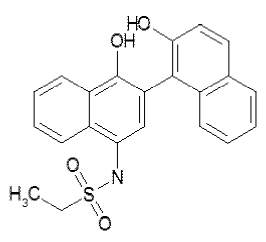 | C22H19NO4S | 393.465 | 5.329 |
| F5749-0013 | 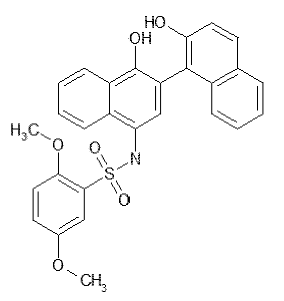 | C28H23NO6S | 501.5626 | 6.417 |
| F5749-0014 | 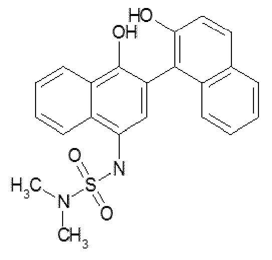 | C22H20N2O4S | 408.4797 | 4.364 |
| F5749-0015 | 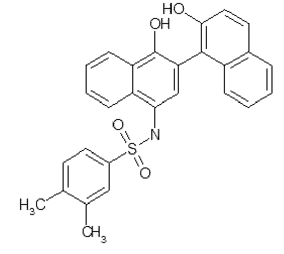 | C28H23NO4S | 469.5638 | 7.101 |
| F5749-0016 | 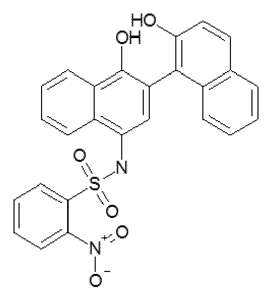 | C26H18N2O6S | 486.5072 | 6.403 |
| F5749-0017 | 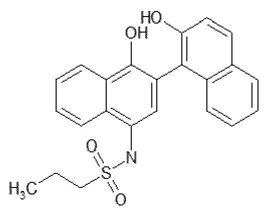 | C23H21NO4S | 407.4921 | 5.771 |
| F5749-0018 | 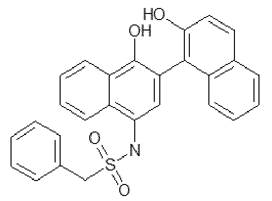 | C27H21NO4S | 455.5367 | 6.604 |
| F5749-0019 | 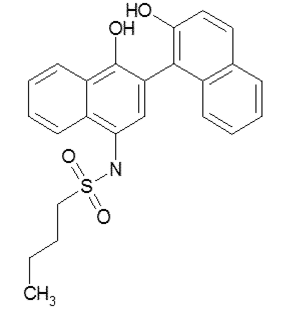 | C24H23NO4S | 421.5192 | 6.213 |
| F5749-0020 | 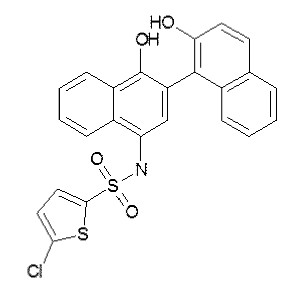 | C24H16ClNO4S2 | 481.9804 | 7.273 |
| F5749-0021 | 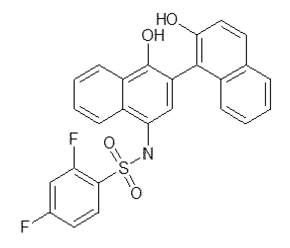 | C26H17F2NO4S | 477.4905 | 6.811 |
| F5749-0022 | 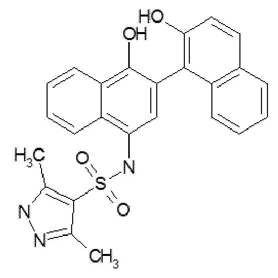 | C25H21N3O4S | 459.5278 | 5.58 |
| F5749-0023 | 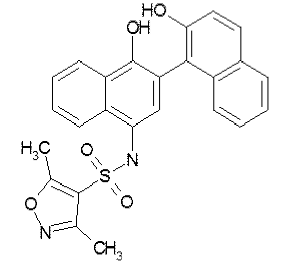 | C25H20N2O5S | 460.5126 | 5.614 |
| F5749-0024 | 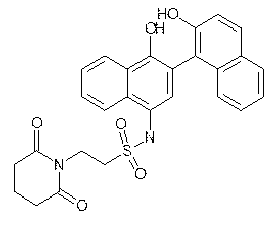 | C27H24N2O6S | 504.5661 | 4.257 |
| F5749-0025 | 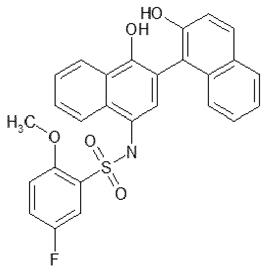 | C27H20FNO5S | 489.5266 | 6.614 |
| F5749-0026 | 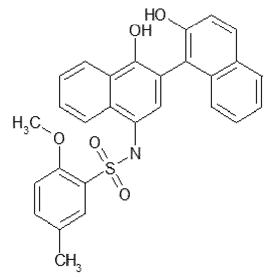 | C28H23NO5S | 485.5632 | 6.759 |
| F5749-0027 | 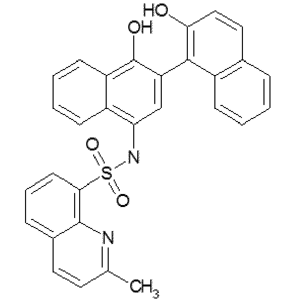 | C30H22N2O4S | 506.5848 | 6.929 |
| F5749-0028 | 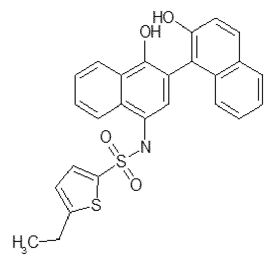 | C26H21NO4S2 | 475.5896 | 7.121 |
| F5749-0029 | 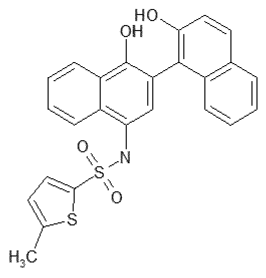 | C25H19NO4S2 | 461.5625 | 6.646 |
| F5749-0030 | 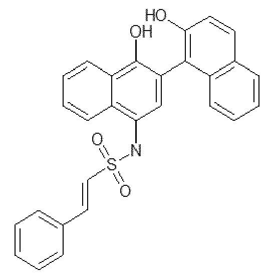 | C28H21 NO4S | 467.5479 | 6.828 |
| F5749-0031 | 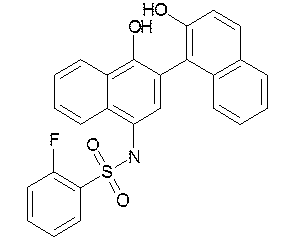 | C26H18FNO4S | 459.5001 | 6.621 |
| F5749-0032 | 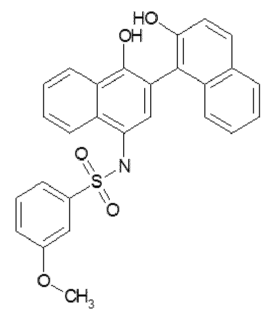 | C27H21NO5S | 471.5361 | 6.463 |
| F5749-0033 | 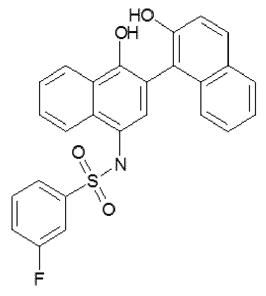 | C26H18FNO4S | 459.5001 | 6.66 |
| F5749-0034 | 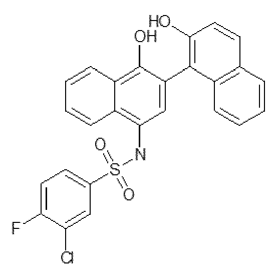 | C26H17ClFNO4S | 493.9451 | 7.25 |
| F5749-0035 | 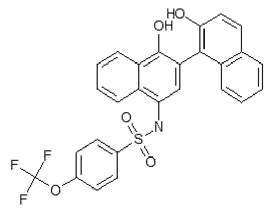 | C27H18F3NO5S | 525.5074 | 7.86876 |
| F5749-0036 | 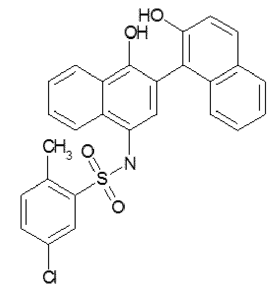 | C27H20ClNO4S | 489.9818 | 7.395 |
| F5749-0037 | 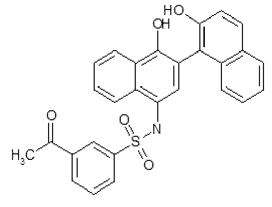 | C28H21NO5S | 483.5473 | 6.36 |
| F5749-0038 | 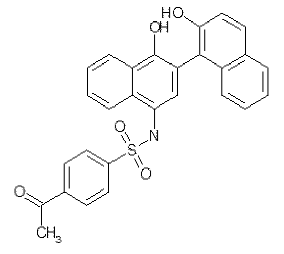 | C28H21NO5S | 483.5473 | 6.323 |
| F5749-0039 | 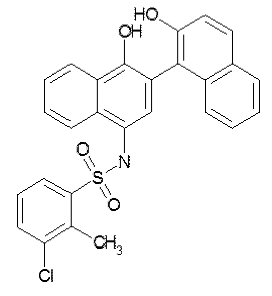 | C27H20ClNO4S | 489.9818 | 7.356 |
| F5749-0040 | 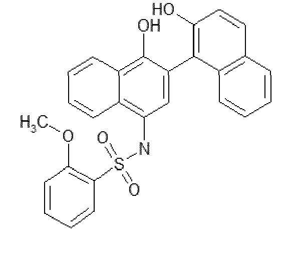 | C27H21NO5S | 471.5361 | 6.424 |
| F5749-0041 | 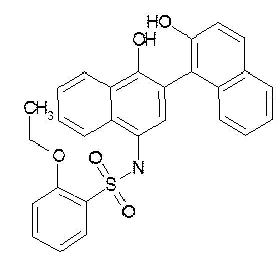 | C28H23NO5S | 485.5632 | 6.765 |
| F5749-0042 | 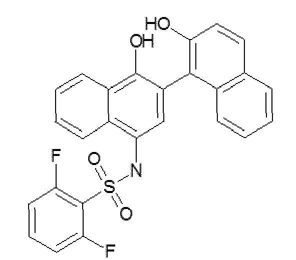 | C26H17F2NO4S | 477.4905 | 6.772 |
| F5749-0043 | 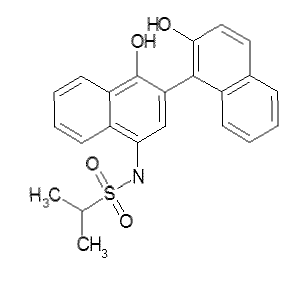 | C23H21 NO4S | 407.4921 | 5.963 |
| F5749-0044 | 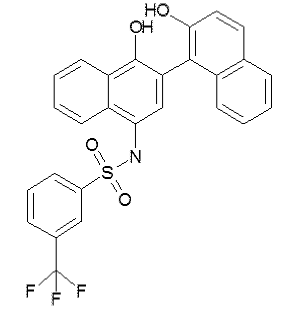 | C27H18F3NO4S | 509.508 | 7.44176 |
| F5749-0045 | 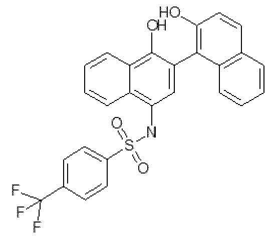 | C27H18F3NO4S | 509.508 | 7.40476 |
| F5749-0046 | 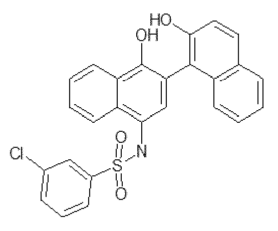 | C26H18ClNO4S | 475.9547 | 7.099 |
| F5749-0047 | 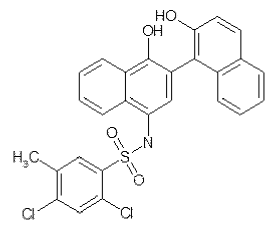 | C27H19Cl2NO4S | 524.4268 | 8.022 |
| F5749-0048 | 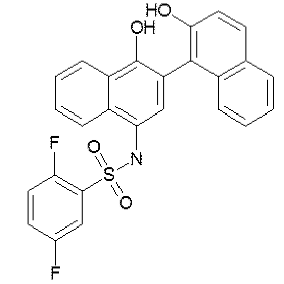 | C26H17F2NO4S | 477.4905 | 6.811 |
| F5749-0049 | 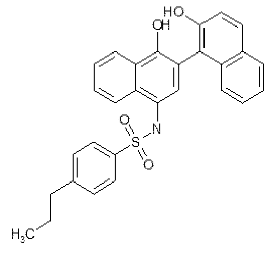 | C29H25NO4S | 483.5909 | 7.685 |
| F5749-0050 | 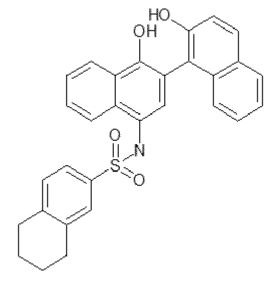 | C30H25NO4S | 495.6021 | 7.557 |
| F5749-0051 | 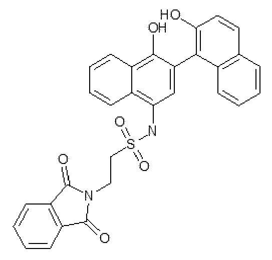 | C30H22N2O6S | 538.5836 | 5.597 |
| F5749-0052 | 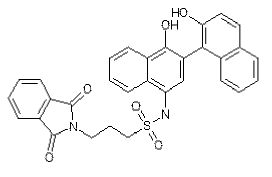 | C31H24N2O6S | 552.6107 | 6.039 |
| F5749-0053 | 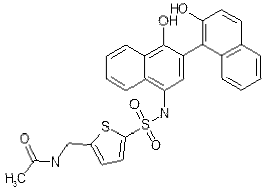 | C27H22N2O5S2 | 518.6148 | 5.773 |
| F5749-0054 | 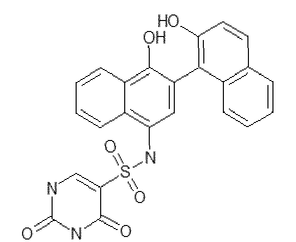 | C24H17N3O6S | 475.4836 | 3.515 |
| F5749-0055 | 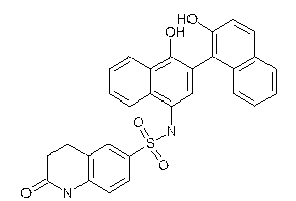 | C29H22N2O5S | 510.5731 | 5.666 |
| F5749-0056 | 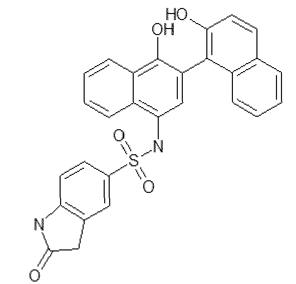 | C28H20N2O5S | 496.546 | 5.578 |
| F5749-0057 | 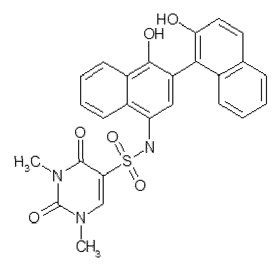 | C26H21N3O6S | 503.5378 | 3.579 |
| F5749-0058 | 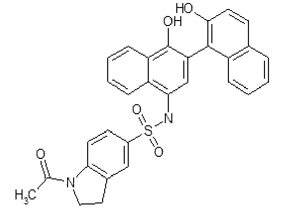 | C30H24N2O5S | 524.6002 | 5.901 |
| F5749-0059 | 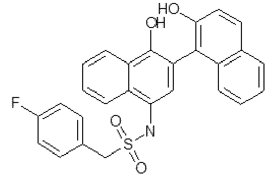 | C27H20FNO4S | 473.5272 | 6.757 |
| F5749-0060 | 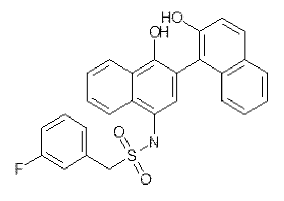 | C27H20FNO4S | 473.5272 | 6.794 |
| F5749-0061 | 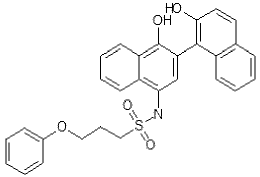 | C29H25NO5S | 499.5903 | 6.83 |
| F5749-0062 | 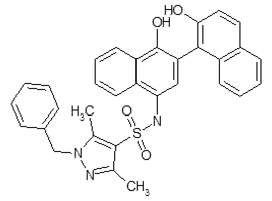 | C32H27N3O4S | 549.6537 | 6.749 |
| F5749-0063 | 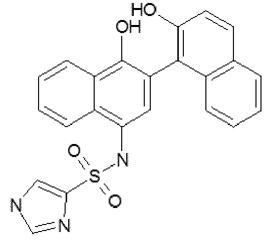 | C23H17N3O4S | 431.4736 | 4.61 |
| F5749-0064 | 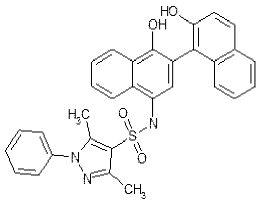 | C31H25N3O4S | 535.6266 | 6.615 |
| F5749-0065 | 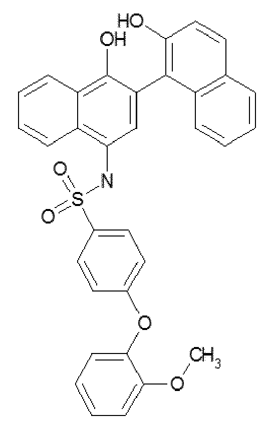 | C33H25NO6S | 563.6343 | 7.78574 |
| F5749-0066 | 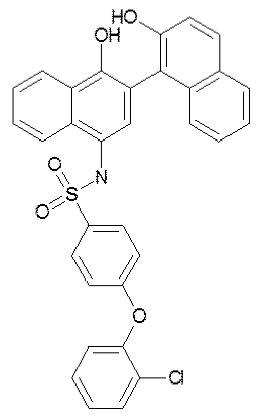 | C32H22ClNO5S | 568.0528 | 8.67 |
| F5749-0067 | 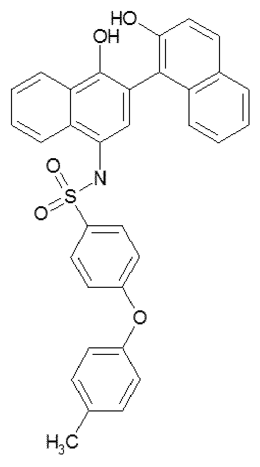 | C33H25NO5S | 547.6349 | 8.378 |
| F5749-0068 | 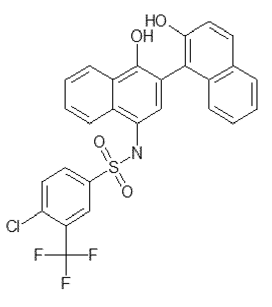 | C27H17ClF3NO4S | 543.953 | 8.03176 |
| F5749-0069 | 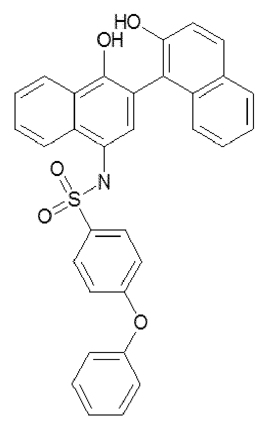 | C32H23NO5S | 533.6078 | 8.08 |
| F5749-0070 | 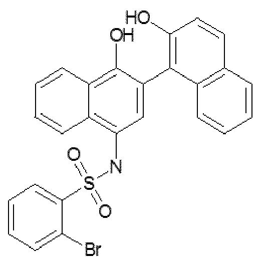 | C26H18BrNO4S | 520.4057 | 7.266 |
| F5749-0071 | 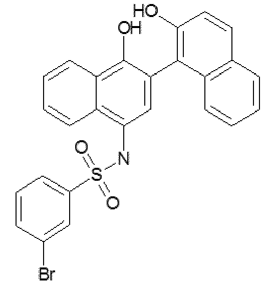 | C26H18BrNO4S | 520.4057 | 7.305 |
| F5749-0072 | 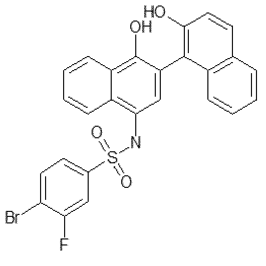 | C26H17BrFNO4S | 538.3961 | 7.456 |
| F5749-0073 | 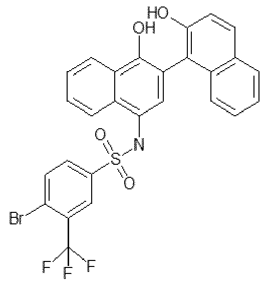 | C27H17BrF3NO4S | 588.404 | 8.23776 |
| F5749-0074 | 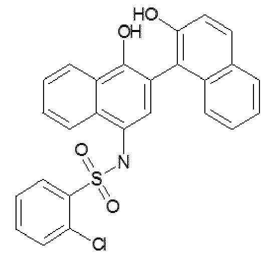 | C26H18ClNO4S | 475.9547 | 7.06 |
| F5749-0075 | 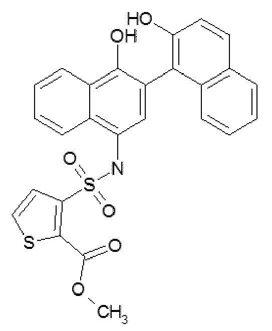 | C26H19NO6S2 | 505.5724 | 5.67 |
| F5749-0076 | 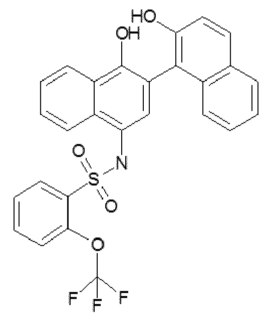 | C27H18F3NO5S | 525.5074 | 7.86676 |

### EXAMPLE 11

### A ROLE FOR STAT3 SIGNALING IN MAST CELL DEGRANULATION

Autosomal-dominant hyper-IgE syndrome (AD-HIES) patients carry dominant-negative STAT3 mutations, develop frequent skin and lung infections, and also have a variety of non-immunologic manifestations affecting bones and connective tissue. In addition, almost all have an eczematous rash present very early in life, as well as the markedly elevated serum IgE levels which give the disease its name. Of note, one-third of patients in the broader population with atopic dermatitis develop food allergies. Despite these observations, the susceptibility of AD-HIES patients to specific food allergies has not been carefully examined. The inventors have found that fewer AD-HIES patients develop food allergies and anaphylaxis than patients with marked IgE elevations and eczema without STAT3 mutations. In embodiments of the invention, this is due at least in part to the effects of defective STAT3 signaling on mast cell degranulation.

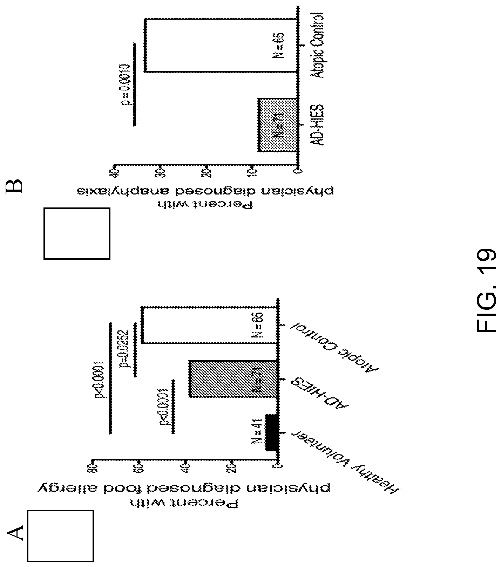

Thirty eight percent of STAT3-mutant patients had immediate hypersensitivity to food, significantly less than the 58.3% observed in atopic patients without a STAT3 mutation (FIG. 19A). Far fewer AD-HIES patients had anaphylaxis to a food allergen than atopic controls (8.5% vs. 33.3%) (FIG. 19B).

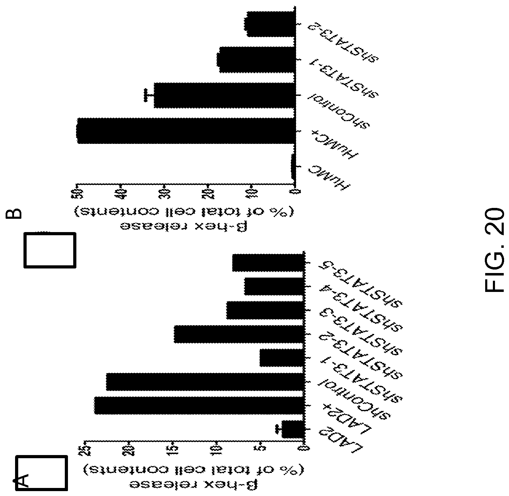

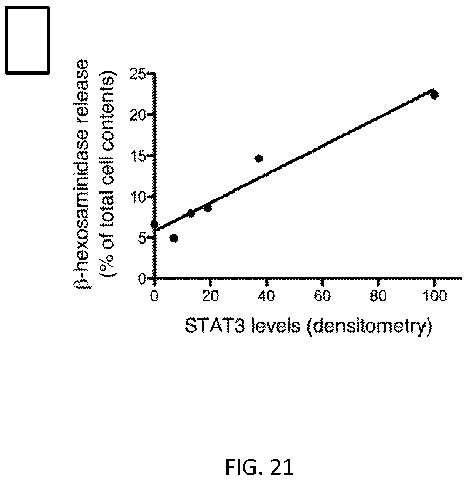

Furthermore, silencing of STAT3 expression inhibited mast cell degranulation following IgE crosslinking in direct proportion to the degree of silencing of STAT3 in LAD2 cells (FIG. 21). Similarly, silencing of STAT3 in primary human mast cells lead to decreased IgE-mediated mast cell degranulation (FIG. 20B).

### EXAMPLE 12

### EXAMPLES OF COMPOSITIONS FOR ANAPHYLAXIS TREATMENT

One or more compositions are characterized as anaphylaxis treatment and/or prevention using standard means in the art. In certain cases, a rodent model of anaphylaxis is employed to test one or more compositions of interest for effectiveness in anaphylaxis. In at least some aspects, a temperature drop in the rodent is used as a measure of anaphylaxis.

### Systemic Anaphylaxis Assay

Any suitable *in vivo* model of anaphylaxis may be employed. Mice may be sensitized (*i.v*., for example) with an effective amount of DNP-specific IgE (H1-DNP-e₋₂₆) in an appropriate buffer and challenged (*i.v*., for example) after an appropriate amount of time (24 h, for example) with an effective amount of rat anti-mouse IgE.

Alternatively, anaphylaxis may be induced by injection (*i.v*.) of compound 48/80 (Sigma Aldrich) at a sub-lethal concentration (for example, concentrations less than 100 µg in 200 µl of buffer were lethal). Implantable electronic transponders (Bio Medic Data Systems) may be inserted under the dorsal skin of anesthetized mice at least 24 hrs before the start of the anaphylaxis studies. Basal body temperatures before induction of anaphylaxis and temperature changes during anaphylaxis may be monitored using an electronic scanner (Bio Medic Data Systems).

### Therapeutic Assay

In certain embodiments, the composition being tested (for example, Cpd 188-9) is provided to the individual for a period of at least 1, 2, 3, 4, 5, 6, 7, or more days; the administration may be by any suitable route, including intravenous, subcutaneous, aerosolization, inhalation, orally, and so forth. Following this period of time, anaphylaxis may be induced in the model system.

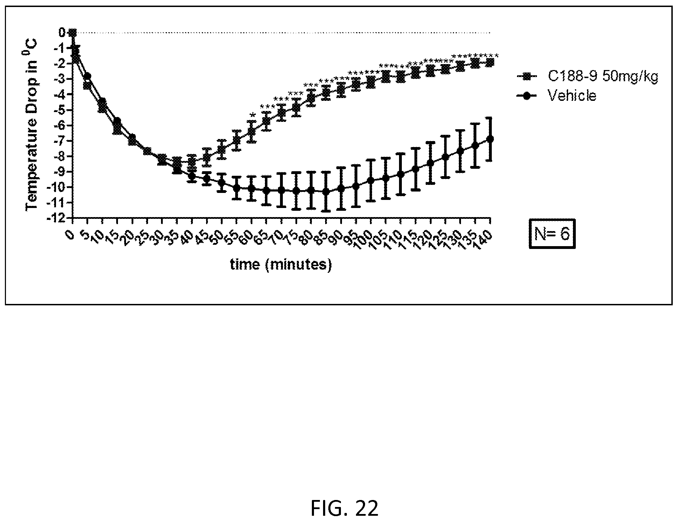

FIG. 22 demonstrates effective treatment in an anaphylaxis model using Cpd188-9. Mice as an anaphylaxis model were pre-treated with 50 mg/kg Cpd 188-9 or which vehicle for one week, after which anaphylaxis was induced *via* systemic IgE cross-linking at time 0. Detectably within at least 40 minutes the reverse in temperature drop occurred, demonstrating effective use in anaphylaxis conditions.

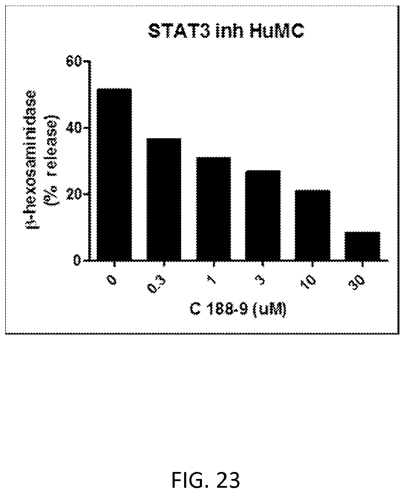

FIG. 23 illustrates dose response using different examples of dosages of Cpd188-9 on normal human mast cells *in vitro.* Beta-hexosaminidase (% release) is used as an example of a measure of mast cell degranulation, which reflects the intensity of anaphylaxis. With increasing amounts of Cpd 188-9 administered for at least three days prior, mast cell degranulation was reduced.

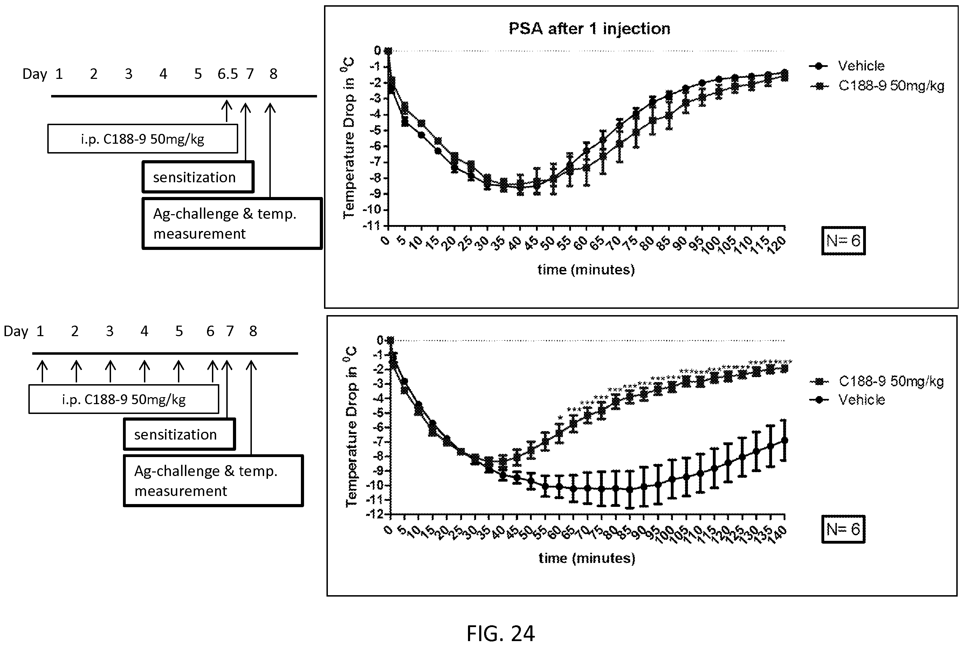

FIG. 24 shows that systemic anaphylaxis was prevented *in vivo* with an exemplary STAT3 inhibitor. Healthy wild-type mice were pretreated for either one day (top panel) or one week (bottom panel) with C188-9 at 50mg/kg. Mice were then injected with IgE specific for an antigen, and the following day the antigen was injected and drop in body temperature recorded as a measure of anaphylaxis. Inhibition of the drop is shown only in the bottom panel, when mice were pretreated (in red) for one week with C188-9.

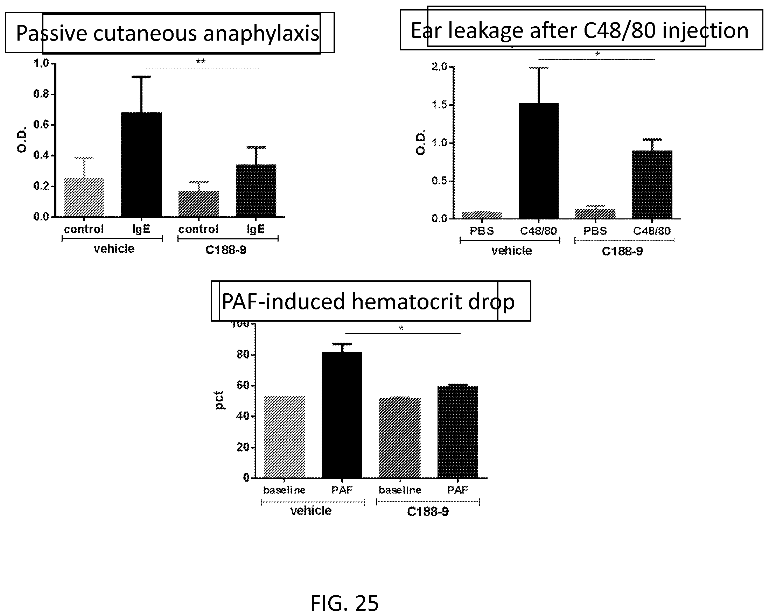

FIG 25 demonstrates that peripheral and central vascular leakage is decreased by Cpd 188-9. Mice were pretreated with C188-9 for one week as in FIG. 24, then injected with a dye to measure the inhibition by C188-9 of locally induced IgE-mediated vascular leakage (top left) or mast cell secretagogue C48/80-induced vascular leakage (top right) or platelet activating factor-induced drop in hematocrit--a measure of vascular leakage (bottom).

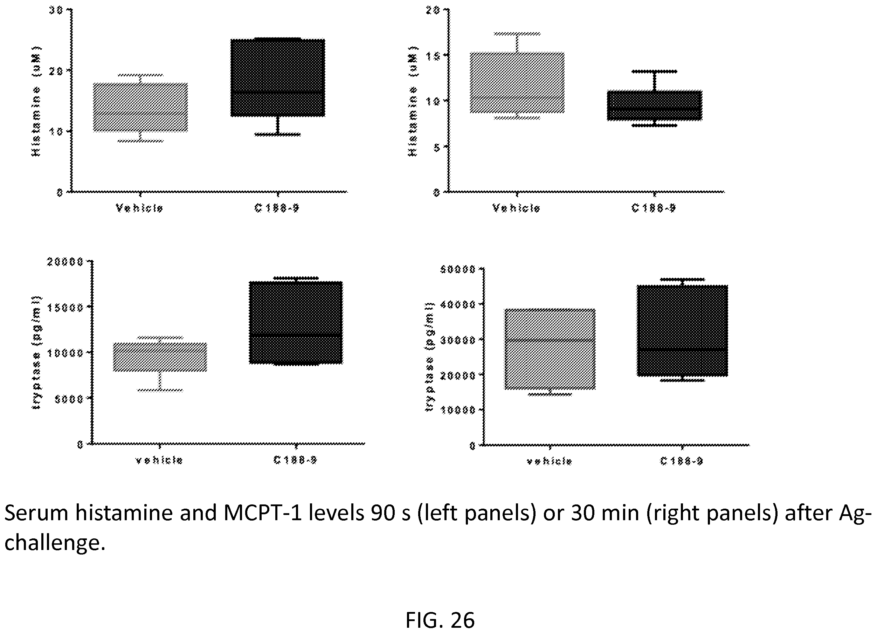

FIG. 26 illustrates that the effect of Cpd188-9 is not because of a decreased mast cell degranulation *in vivo.* Serum histamine and MCPT-1 levels are shown at 90 seconds (left panels) or 30 minutes (right panels) after Ag-challenge after 1 week C188-9 treatment as in FIG. 24. There was no statistical difference, suggesting that mast cell degranulation was not a factor in C188-9 mediated inhibition in mice.

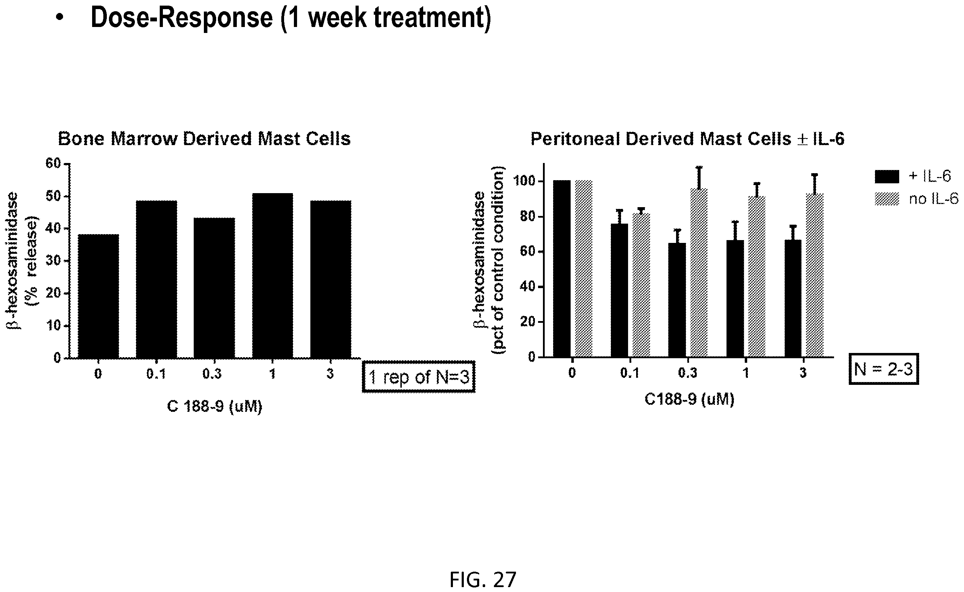

FIG. 27 demonstrates the effct of Cpd 188-9 on Ag-induced degranulation in murine mast cells. Pretreatment of murine bone marrow derived mast cells or peritoneal derived mast cells with C188-9 does not lead to inhibition of mast cell degranulation, in contrast to human mast cells as in FIG. 23. Only incubation of mouse peritoneal mast cells with IL-6 (left panel) enables mast cells to be mildly inhibited by C188-9.

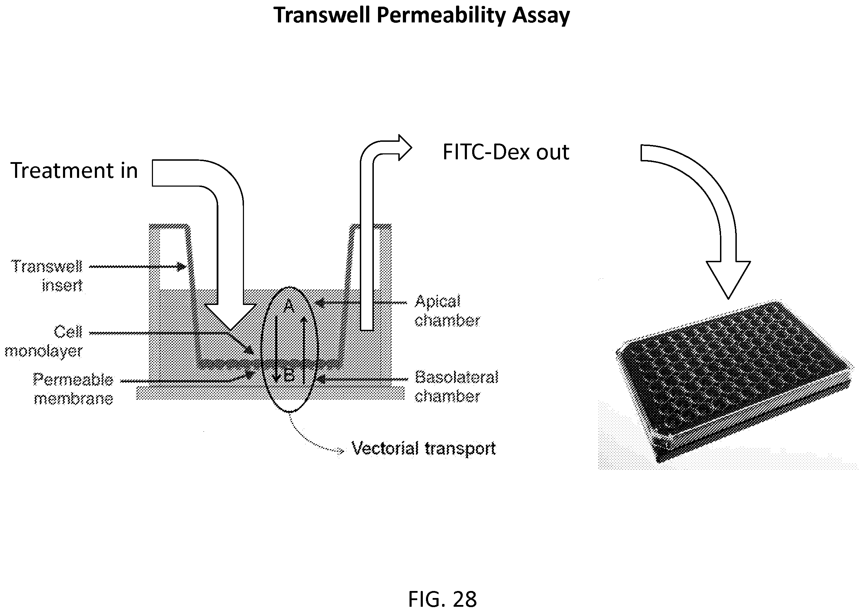

FIG. 28 shows a schematic representation of transwell permeability assay to measure vascular endothelial cell permeability in response to soluble factors and inhibitors.

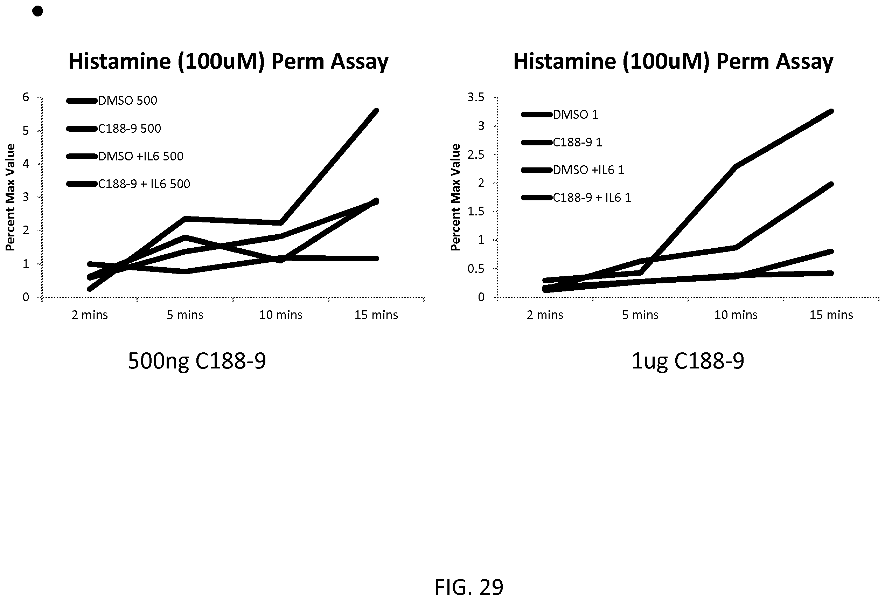

FIG. 29 demonstrates inhibition of vascular permeability of human umbilical vein endothelial cells (HUVECs) by C188-9 pretreated for one week +/-IL-6. DMSO was used as a control for C188-9. Transwell assay was performed in response to 100um of histamine. Maximal inhibition was seen with 1ug C188-9 (on right).

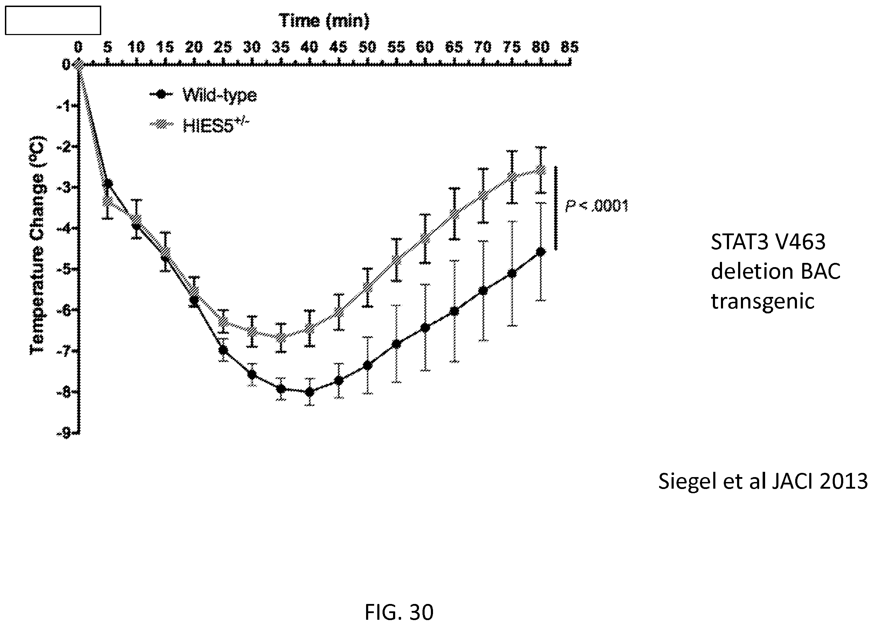

FIG. 30 shows that Hyper-IgE syndrome mouse is resistant to anaphylaxis (Siegel et al., JACI, 2013). Systemic anaphylaxis is induced as in FIG. 24 in a mouse model of the Hyper-IgE syndrome. Mice with dominant negative STAT3 mutations were less prone to severe temperature drop with IgE crosslinking than littermate controls.

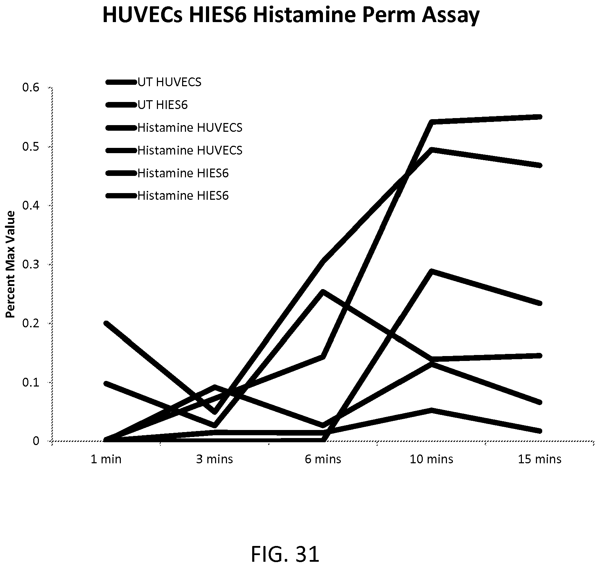

FIG. 31 demonstrates STAT3 mutant (HIES6) HUVECS resistant to histamine-induced permeability. Human umbilical vein endothelial cells derived from patients with dominant negative STAT3 mutations (HIES6) were less responsive to histamine induced vascular permeability than healthy controls (labeled HUVECS). This is direct evidence that impaired STAT3 signaling leads to impaired vascular permeability responses to histamine.

Thus, Cpd188-9 pretreatment of 7 days (but under at least some conditions not one) inhibits systemic anaphylaxis *in vivo.* In specific embodiments, the action is more in vascular endothelial responses to STAT3 than in mast cells, whereas human mast cell and endothelial responses are both highly affected.

### REFERENCES

Full citations for the references cited herein are provided in the following list.

### PUBLICATIONS

Akira, S., 2000. Roles of STAT3 defined by tissue-specific gene targeting. Oncogene 19:2607-2611.
Akira, S., 1997, IL-6-regulated transcription factors. Int J Biochem Cell Biol 29:1401-1418.
Akira, S., Isshiki,H., Sugita,T., Tanabe,O., Kinoshita,S., Nishio,Y., Nakajima,T., Hirano,T., and Kishimoto,T. (1990). A nuclear factor for IL-6 expression (NF-IL6) is a member of a C/EBP family. EMBO J. 9, 1897-1906.
Al-Hajj, M., Wicha, M.S., Benito-Hernandez, A., Morrison, S.J., and Clarke, M.F. 2003. Prospective identification of tumorigenic breast cancer cells. Proc Natl Acad Sci U S A 100:3983-3988.
Becker, S., Groner B, Muller CW (1998) Three-dimensional structure of the Stat3[beta] homodimer bound to DNA. Nature 394(6689): 145-151.
Bhasin, D., Cisek,K., Pandharkar,T., Regan,N., Li,C., Pandit,B., Lin,J., and Li,P.K. (2008). Design, synthesis, and studies of small molecule STAT3 inhibitors. Bioorg. Med. Chem. Lett. 18, 391-395.
Brinkley, BR, Beall PT, Wible LJ, Mace ML, Turner DS et al. (1980) Variations in Cell Form and Cytoskeleton in Human Breast Carcinoma Cells in Vitro. Cancer Res 40(9): 3118-3129.
Bromberg, J., 2002. Stat proteins and oncogenesis. J Clin Invest 109:1139-1142.
Bromberg, J., and Darnell, J.E., Jr. 2000. The role of STATs in transcriptional control and their impact on cellular function. Oncogene 19:2468-2473.
Bromberg, J.F., Horvath, C.M., Besser, D., Lathem, W.W., and Darnell, J.E., Jr. 1998. Stat3 activation is required for cellular transformation by v-src. Mol Cell Biol 18:2553-2558.
Bromberg, J.F., Wrzeszczynska, M.H., Devgan, G., Zhao, Y., Pestell, R.G., Albanese, C., and Darnell, J.E., Jr. 1999. Stat3 as an oncogene [published erratum appears in Cell 1999 Oct 15;99(2):239]. Cell 98:295-303.
Cailleau R OM, Crueiger QVJ. (1978) Long term human breast carcinoma cell lines of metastatic origin: preliminary characterization. In Vitro 14: 911-915.
Caldenhoven, E., van, D.T.B., Solari, R., Armstrong, J., Raaijmakers, J.A.M., Lammers, J.W.J., Koenderman, L., and de, G.R.P. 1996. STAT3beta, a splice variant of transcription factor STAT3, is a dominant negative regulator of transcription. Journal of Biological Chemistry 271:13221-13227.
Catlett-Falcone, R., Landowski, T.H., Oshiro, M.M., Turkson, J., Levitzki, A., Savino, R., Ciliberto, G., Moscinski, L., Fernandez-Luna, J.L., Nunez, G., et al. 1999. Constitutive activation of Stat3 signaling confers resistance to apoptosis in human U266 myeloma cells. Immunity 10:105-115.
Chakraborty, A., Dyer KF, Cascio M, Mietzner TA, Tweardy DJ (1999) Identification of a Novel Stat3 Recruitment and Activation Motif Within the Granulocyte Colony-Stimulating Factor Receptor. Blood 93(1): 15-24.
Chakraborty, A., White, S.M., Schaefer, T.S., Ball, E.D., Dyer, K.F., and Tweardy, D.J. 1996. Granulocyte colony-stimulating factor activation of Stat3 alpha and Stat3 beta in immature normal and leukemic human myeloid cells. Blood 88:2442-2449.
Chapman, R.S., Lourenco, P.C., Tonner, E., Flint, D.J., Selbert, S., Takeda, K., Akira, S., Clarke, A.R., and Watson, C.J. 1999. Suppression of epithelial apoptosis and delayed mammary gland involution in mice with a conditional knockout of Stat3. Genes Dev 13:2604-2616.
Chen, X., Vinkemeier U, Zhao Y, Jeruzalmi D, Darnell JE et al. (1998) Crystal Structure of a Tyrosine Phosphorylated STAT-1 Dimer Bound to DNA. Cell 93(5): 827-839.
Cohen, M.S., Zhang C, Shokat KM, Taunton J (2005) Structural Bioinformatics-Based Design of Selective, Irreversible Kinase Inhibitors. Science 308(5726): 1318-1321.
Coleman, DR, Ren Z, Mandal PK, Cameron AG, Dyer GA et al. (2005) Investigation of the Binding Determinants of Phosphopeptides Targeted to the Src Homology 2 Domain of the Signal Transducer and Activator of Transcription 3. Development of a High-Affinity Peptide Inhibitor. J Med Chem 48(21): 6661-6670.
Costa-Pereira, A.P., Tininini, S., Strobl, B., Alonzi, T., Schlaak, J.F., Is'harc, H., Gesualdo, I., Newman, S.J., Kerr, I.M., and Poli, V. 2002. Mutational switch of an IL-6 response to an interferon-gamma-like response. Proc Natl Acad Sci U S A 99:8043-8047.
Daling, J.R., and Malone, K.E. 2003. Incidence of invasive breast cancer by hormone receptor status from 1992 to 1998. J Clin Oncol 21:28-34.
Darnell JE (2005), Validating Stat3 in cancer therapy. Nat Med 11(6): 595-596.
Dave, B., , and Chang, J. 2009. Treatment resistance in stem cells and breast cancer. J Mammary Gland Biol Neoplasia 14:79-82.
Diaz, N., Minton, S., Cox, C., Bowman, T., Gritsko, T., Garcia, R., Eweis, I., Wloch, M., Livingston, S., Seijo, E., et al. 2006. Activation of stat3 in primary tumors from high-risk breast cancer patients is associated with elevated levels of activated SRC and survivin expression. Clin Cancer Res 12:20-28.
Dong, S., Chen S-J, Tweardy DJ (2003) Cross-talk between Retinoic Acid and Stat3 Signaling Pathways in Acute Promyelocytic Leukemia. Leuk Lymphoma 44: 2023-2029.
Dunn, GP, Bruce AT, Ikeda H, Old LJ, Schreiber RD (2002) Cancer immunoediting: from immunosurveillance to tumor escape. Nat Immunol 3(11): 991-998.
Durbin, J.E., Hackenmiller, R., Simon, M.C., and Levy, D.E. 1996. Targeted disruption of the mouse Stat1 gene results in compromised innate immunity to viral disease. Cell 84:443-450.
Eckert, H., Bajorath J (2007) Molecular similarity analysis in virtual screening: foundations, limitations and novel approaches. Drug discovery today 12(5-6): 225-233.
Epling-Burnette, P.K., Liu, J.H., Catlett-Falcone, R., Turkson, J., Oshiro, M., Kothapalli, R., Li, Y., Wang, J.M., Yang-Yen, H.F., Karras, J., et al. 2001. Inhibition of STAT3 signaling leads to apoptosis of leukemic large granular lymphocytes and decreased Mcl-1 expression. J Clin Invest 107:351-362.
Fiala, S., 1968. The cancer cell as a stem cell unable to differentiate. A theory of carcinogenesis. Neoplasma 15:607-622.
Fu, X.-Y., Schindler, C, Improta, T., Aebersold, R., and Darnell, J.E., Jr. 1992. The proteins of ISGF-3, the interferon alpha-induced transcriptional activator, define a gene family involved in signal transduction. Proceedings of the National Academy of Sciences of the United States of America 89:7840-7843.
Garcia, R., and, Jove, R. 1998. Activation of STAT transcription factors in oncogenic tyrosine kinase signaling. Journal of Biomedical Science In press.
Garcia R, Yu CL, Hudnall A, Catlett R, Nelson KL et al. (1997) Constitutive activation of Stat3 in fibroblasts transformed by diverse oncoproteins and in breast carcinoma cells. Cell Growth Differ 8(12): 1267-1276.
Garcia R, Bowman TL, Niu G, Yu H, Minton S et al. (2001) Constitutive activation of Stat3 by the Src and Jak tyrosine kinases participates in growth regulation of human breast carcinoma cells. Oncogene 20: 2499-2513.
Grandis, J.R., Drenning, S.D., Zeng, Q., Watkins, S.C., Melhem, M.F., Endo, S., Johnson, D.E., Huang, L., He, Y., and Kim, J.D. 2000. Constitutive activation of Stat3 signaling abrogates apoptosis in squamous cell carcinogenesis in vivo. Proc Natl Acad Sci U S A 97:4227-4232.
Gritsko, T., Williams, A., Turkson, J., Kaneko, S., Bowman, T., Huang, M., Nam, S., Eweis, I., Diaz, N., Sullivan, D., et al. 2006. Persistent activation of stat3 signaling induces survivin gene expression and confers resistance to apoptosis in human breast cancer cells. Clin Cancer Res 12:11-19.
Haan, S., Hemmann, U., Hassiepen, U., Schaper, F., Schneider-Mergener, J., Wollmer, A., Heinrich, P.C., and Grotzinger, J. 1999. Characterization and binding specificity of the monomeric STAT3-SH2 domain. J Biol Chem 274:1342-1348.
Huang, Y., Qiu J, Dong S, Redell MS, Poli V et al. (2007) Stat3 Isoforms, {alpha} and, Demonstrate Distinct Intracellular Dynamics with Prolonged Nuclear Retention of Stat3 Mapping to Its Unique C-terminal End. J Biol Chem 282(48): 34958-34967.
Jemal, A., Siegel, R., Ward, E., Murray, T., Xu, J., Smigal, C., and Thun, M.J. 2006. Cancer statistics, 2006. CA Cancer J Clin 56:106-130.
Jing, N., Tweardy DJ (2005) Targeting Stat3 in cancer therapy. anticancer Drugs 16(6): 601-607.
Jing, N., Zhu Q, Yuan P, Li Y, Mao L et al. (2006) Targeting signal transducer and activator of transcription 3 with G-quartet oligonucleotides: a potential novel therapy for head and neck cancer. Mol Cancer Ther 5(2): 279-286.
Jing, N., Li Y, Xu X, Sha W, Li P et al. (2003) Targeting Stat3 with G-quartet oligodeoxynucleotides in human cancer cells. DNA Cell Biol 22(11): 685-696.
Jing, N., Li, Y., Xiong, W., Sha, W., Jing, L., and Tweardy, D.J. 2004. G-quartet oligonucleotides: a new class of signal transducer and activator of transcription 3 inhibitors that suppresses growth of prostate and breast tumors through induction of apoptosis. Cancer Res 64:6603-6609.
Kaplan, D.H., Shankaran, V., Dighe, A.S., Stockert, E., Aguet, M., Old, L.J., and Schreiber, R.D. 1998. Demonstration of an interferon gamma-dependent tumor surveillance system in immunocompetent mice. Proc Natl Acad Sci U S A 95:7556-7561.
Kato, T., Sakamoto E, Kutsuna H, Kimura-Eto A, Hato F et al. (2004) Proteolytic Conversion of STAT3 {alpha} to STAT3{gamma} in Human Neutrophils: ROLE OF GRANULE-DERIVED SERINE PROTEASES. J Biol Chem 279(30): 31076- 31080.
Kim, J.K., Xu Y, Xu X, Keene DR, Gurusiddappa S et al. (2005) A Novel Binding Site in Collagen Type III for Integrins {alpha}1{beta}1 and {alpha}2{beta}1. J Biol Chem 280(37): 32512-32520.
Kortylewski, M., Kujawski M, Wang T, Wei S, Zhang S et al. (2005) Inhibiting Stat3 signaling in the hematopoietic system elicits multicomponent antitumor immunity. Nat Med 11(12): 1314-1321.
Leong, P.L., Andrews, G.A., Johnson, D.E., Dyer, K.F., Xi, S., Mai, J.C., Robbins, P.D., Gadiparthi, S., Burke, N.A., Watkins, S.F., et al. 2003. Targeted inhibition of Stat3 with a decoy oligonucleotide abrogates head and neck cancer cell growth. Proc Natl Acad Sci U S A 100:4138-4143.
Li, C.I., Daling, J.R., and Malone, K.E. 2003. Incidence of invasive breast cancer by hormone receptor status from 1992 to 1998. J Clin Oncol 21:28-34.
Li, L., and Shaw, P.E. 2002. Autocrine-mediated activation of STAT3 correlates with cell proliferation in breast carcinoma lines. J Biol Chem 277:17397-17405.
Li, X., Lewis, M.T., Huang, J., Gutierrez, C., Osborne, C.K., Wu, M.F., Hilsenbeck, S.G., Pavlick, A., Zhang, X., Chamness, G.C., et al. 2008. Intrinsic resistance of tumorigenic breast cancer cells to chemotherapy. J Natl Cancer Inst 100:672-679.
Lin, Q., Lai R, Chirieac LR, Li C, Thomazy VA et al. (2005) Constitutive Activation of JAK3/STAT3 in Colon Carcinoma Tumors and Cell Lines: Inhibition of JAK3/STAT3 Signaling Induces Apoptosis and Cell Cycle Arrest of Colon Carcinoma Cells. Am J Pathol 167(4): 969-980.
Maritano, D., Sugrue, M.L., Tininini, S., Dewilde, S., Strobl, B., Fu, X., Murray-Tait, V., Chiarle, R., and Poli, V. 2004. The STAT3 isoforms alpha and beta have unique and specific functions. Nat Immunol 5:401-409.
McMurray JS (2006), A New Small-Molecule Stat3 Inhibitor. Chemistry & Biology 13(11): 1123-1124.
Meraz, M.A., White, J.M., Sheehan, K.C., Bach, E.A., Rodig, S.J., Dighe, A.S., Kaplan, D.H., Riley, J.K., Greenlund, A.C., Campbell, D., et al. 1996. Targeted disruption of the Stat1 gene in mice reveals unexpected physiologic specificity in the JAK-STAT signaling pathway. Cell 84:431-442.
Minino, A.M., Heron, M.P., Murphy, S.L., and Kochanek, K.D. 2007. Deaths: final data for 2004. Natl Vital Stat Rep 55:1-119.
Mora, L.B., Buettner, R., Seigne, J., Diaz, J., Ahmad, N., Garcia, R., Bowman, T., Falcone, R., Fairclough, R., Cantor, A., et al. 2002. Constitutive activation of Stat3 in human prostate tumors and cell lines: direct inhibition of Stat3 signaling induces apoptosis of prostate cancer cells. Cancer Res 62:6659-6666.
Neculai, D., Neculai AM, Verrier S, Straub K, Klumpp K et al. (2005) Structure of the Unphosphorylated STAT5a Dimer. J Biol Chem 280(49): 40782-40787.
Nemethy, G., Gibson KD, Palmer KA, Yoon CN, Paterlini G et al. (1992) Energy Parameters in Polypeptides. 10. Improved Geometrical Parameters and Nonbonded Interactions for Use in the ECEPP/3 Algorithm, with Application to Proline-Containing Peptides. JPhys Chem 96: 6472-6484.
Park, O.K., Schaefer, T.S., and Nathans, D. 1996. In vitro activation of Stat3 by epidermal growth factor receptor kinase. Proceedings of the National Academy of Sciences of the United States of America 93:13704-13708.
Park, O.K., Schaefer, L.K., Wang, W., and Schaefer, T.S. 2000. Dimer stability as a determinant of differential DNA binding activity of Stat3 isoforms. J Biol Chem 275:32244-32249.
Qing, Y., and Stark, G.R. 2004. Alternative activation of STAT1 and STAT3 in response to interferon-gamma. J Biol Chem 279:41679-41685.
Ramana, C., Chatterjee-Kishore M, Nguyen H, Stark G (2000) Complex roles of Stat1 in regulating gene expression. Oncogene 19(21): 2619-2627.
Real, P.J., Sierra, A., De Juan, A., Segovia, J.C., Lopez-Vega, J.M., and Fernandez-Luna, J.L. 2002. Resistance to chemotherapy via Stat3-dependent overexpression of Bcl-2 in metastatic breast cancer cells. Oncogene 21:7611-7618.
Redell, MS, Tweardy DJ (2006) Targeting transcription factors in cancer: Challenges and evolving strategies. Drug Discovery Today: Technologies 3(3): 261-267.
Redell, M.S., and Tweardy, D.J. 2005. Targeting transcription factors for cancer therapy. Curr Pharm Des 11:2873-2887.
Ren, Z., Cabell, L.A., Schaefer, T.S., and McMurray, J.S. 2003. Identification of a high-affinity phosphopeptide inhibitor of stat3. Bioorg Med Chem Lett 13:633-636.
Ryan, J.J., McReynolds, L.J., Huang, H., Nelms, K., and Paul, W.E. 1998. Characterization of a mobile Stat6 activation motif in the human IL-4 receptor. J Immunol 161:1811-1821.
Satya-Prakash KL PS, Hsu TC, Olive M, Cailleau R (1981) Cytogenetic analysis on eight human breast tumor cell lines: high frequencies of 1q, 11q, and HeLa-like marker chromosomes. Cancer GenetCytogenet 3: 61-73.
Schaefer, T.S., Sanders, L.K., and Nathans, D. 1995. Cooperative transcriptional activity of Jun and Stat3 beta, a short form of Stat3. Proceedings of the National Academy of Sciences of the United States of America 92:9097-9101.
Schindler, C., and Darnell, J.E., Jr. 1995. Transcriptional responses to polypeptide ligands: the JAK-STAT pathway. [Review]. Annual Review of Biochemistry 64:621-651.
Schindler, C., Fu, X.Y., Improta, T., Aebersold, R., and Darnell, J.E., Jr. 1992. Proteins of transcription factor ISGF-3: one gene encodes the 91-and 84-kDa ISGF-3 proteins that are activated by interferon alpha. Proceedings of the National Academy of Sciences of the United States of America 89:7836-7839.
Schust, J., Sperl, B., Hollis, A., Mayer, T.U., and Berg, T. (2006). Stattic: a small-molecule inhibitor of STAT3 activation and dimerization. Chem. Biol. 13, 1235-1242.
Shao, H., Cheng HY, Cook RG, Tweardy DJ (2003) Identification and Characterization of Signal Transducer and Activator of Transcription 3 Recruitment Sites within the Epidermal Growth Factor Receptor. Cancer Res 63(14): 3923-3930.
Shao, H., Xu X, Jing N, Tweardy DJ (2006) Unique Structural Determinants for Stat3 Recruitment and Activation by the Granulocyte Colony-Stimulating Factor Receptor at Phosphotyrosine Ligands 704 and 744. J Immunol 176(5): 2933-2941.
Shao, H., Xu X, Mastrangelo M-AA, Jing N, Cook RG et al. (2004) Structural Requirements for Signal Transducer and Activator of Transcription 3 Binding to Phosphotyrosine Ligands Containing the YXXQ Motif. J Biol Chem 279(18): 18967-18973.
Sharp, Z.D., Mancini MG, Hinojos CA, Dai F, Berno V et al. (2006) Estrogen-receptor- {alpha} exchange and chromatin dynamics are ligand- and domain-dependent. J Cell Sci 119(19): 4101-4116.
Siddiquee, K., Zhang S, Guida WC, Blaskovich MA, Greedy B et al. (2007) Selective chemical probe inhibitor of Stat3, identified through structure-based virtual screening, induces antitumor activity. Proceedings of the National Academy of Sciences 104(18): 7391-7396.
Siegel, et al. (2013) Diminished allergic disease in patients with STAT3 mutations reveals a role for STAT3 signaling in mast cell degranulation. J Allergy Clin Immunol. 2013 Dec;132(6):1388-96.
Song, H., Wang R, Wang S, Lin J (2005) A low-molecular-weight compound discovered through virtual database screening inhibits Stat3 function in breast cancer cells. Proceedings of the National Academy of Sciences 102(13): 4700-4705.
Strecker, T.E., Shen, Q., Zhang, Y., Hill, J.L., Li, Y., Wang, C., Kim, H.T., Gilmer, T.M., Sexton, K.R., Hilsenbeck, S.G., et al. 2009. Effect of lapatinib on the development of estrogen receptor-negative mammary tumors in mice. J Natl Cancer Inst 101:107-113.
Takeda, K., Noguchi, K., Shi, W., Tanaka, T., Matsumoto, M., Yoshida, N., Kishimoto, T., and Akira, S. 1997. Targeted disruption of the mouse Stat3 gene leads to early embryonic lethality. Proc Natl Acad Sci U S A 94:3801-3804.
Totrov, M., Abagyan R (1997) Proteins 1: 215-220.
Turkson, J., 2004. STAT proteins as novel targets for cancer drug discovery. Expert Opin Ther Targets 8:409-422.
Turkson, J., Bowman, T., Garcia, R., Caldenhoven, E., De Groot, R.P., and Jove, R. 1998. Stat3 activation by Src induces specific gene regulation and is required for cell transformation. Mol Cell Biol 18:2545-2552.
Turkson, J., Jove R (2000) STAT proteins: novel molecular targets for cancer drug discovery. Oncogene 19: 6613-6626.
Turkson, J., Ryan, D., Kim, J.S., Zhang, Y., Chen, Z., Haura, E., Laudano, A., Sebti, S., Hamilton, A.D., and Jove, R. 2001. Phosphotyrosyl peptides block Stat3-mediated DNA binding activity, gene regulation, and cell transformation. J Biol Chem 276:45443-45455.
Turkson, J., Zhang, S., Palmer, J., Kay, H., Stanko, J., Mora, L.B., Sebti, S., Yu, H., and Jove, R. 2004. Inhibition of constitutive signal transducer and activator of transcription 3 activation by novel platinum complexes with potent antitumor activity. Mol Cancer Ther 3:1533-1542.
Tweardy, DJ, Redell MS (2005) Targeting Transcription Factors for Cancer Therapy. Curr Pharm Des 11: 2873-2887.
Tweardy, DJ, Wright TM, Ziegler SF, Baumann H, Chakraborty A et al. (1995) Granulocyte colony-stimulating factor rapidly activates a distinct STAT- like protein in normal myeloid cells. Blood 86(12): 4409-4416.
Uddin, S., Hussain, A.R., Manogaran, P.S., Al-Hussein, K., Platanias, L.C., Gutierrez, M.I., and Bhatia, K.G. 2005. Curcumin suppresses growth and induces apoptosis in primary effusion lymphoma. Oncogene 24:7022-7030.
Wiederkehr-Adam, M., Ernst, P., Muller, K., Bieck, E., Gombert, F.O., Ottl, J., Graff, P., Grossmuller, F., and Heim, M.H. 2003. Characterization of phosphopeptide motifs specific for the Src homology 2 domains of signal transducer and activator of transcription 1 (STAT1) and STAT3. J Biol Chem 278:16117-16128.
Xu, X., Kasembeli, M.M., Jiang, X., Tweardy, B.J., and Tweardy, D.J. 2009. Chemical probes that competitively and selectively inhibit Stat3 activation. PLoS ONE 4:e4783.
Yang, H., Mammen, J., Wei, W., Menconi,M., Evenson,A., Fareed,M., Petkova,V., and Hasselgren,P.O. (2005). Expression and activity of C/EBPbeta and delta are upregulated by dexamethasone in skeletal muscle. J. Cell Physiol 204, 219-226.
Yoo, J.Y., Huso, D.L., Nathans, D., and Desiderio, S. 2002. Specific ablation of Stat3beta distorts the pattern of Stat3-responsive gene expression and impairs recovery from endotoxic shock. Cell 108:331-344.
Yoshikawa, H., Matsubara, K., Qian, G.S., Jackson, P., Groopman, J.D., Manning, J.E., Harris, C.C., and Herman, J.G. 2001. SOCS-1, a negative regulator of the JAK/STAT pathway, is silenced by methylation in human hepatocellular carcinoma and shows growth-suppression activity. Nat Genet 28:29-35.
Yu, C.L., Meyer, D.J., Campbell, G.S., Larner, A.C., Carter-Su, C., Schwartz, J., and Jove, R. 1995. Enhanced DNA-binding activity of a Stat3-related protein in cells transformed by the Src oncoprotein. Science 269:81-83.
Yu, H., Jove R (2004) The STATs of cancer- new molecular targets come of age. Nature Reviews Cancer 4(2): 97-105.
Zhang RD FI, Price JE (1991) Relative malignant potential of human breast carcinoma cell lines established from pleural effusions and brain metastasis. Invasion Metastasis 11: 204-215.
Zhu, Q., Jing N (2007) Computational study on mechanism of G-quartet oligonucleotide T40214 selectively targeting Stat3. Journal of Computer-Aided Molecular Design 21(10): 641-648.

## Claims

1. A composition for use in a method of preventing or reducing the risk or severity of anaphylaxis in an individual,
wherein said composition is selected from the group consisting of N-(1',2-dihydroxy-1,2'-binaphthalen-4'-yl)-4-methoxybenzenesulfonamide, N-(3,1'-Dihydroxy-[1,2']binaphthalenyl-4'-yl)-4-methoxy-benzenesulfonamide, N-(4,1'-Dihydroxy-[1,2']binaphthalenyl-4'-yl)-4-methoxy-benzenesulfonamide, N-(5,1'-Dihydroxy-[1,2']binaphthalenyl-4'-yl)-4-methoxy-benzenesulfonamide, N-(6,1'-Dihydroxy-[1,2']binaphthalenyl-4'-yl)-4-methoxy-benzenesulfonamide, N-(7,1'-Dihydroxy-[1,2']binaphthalenyl-4'-yl)-4-methoxy-benzenesulfonamide, N-(8,1'-Dihydroxy-[1,2']binaphthalenyl-4'-yl)-4-methoxy-benzenesulfonamide, 4-Bromo-N-(1,6'-dihydroxy-[2,2']binaphthalenyl-4-yl)-benzenesulfonamide, and combinations thereof; and
wherein said method comprises the step of providing to the individual an effective amount of one or more of said compositions.

2. A composition for use according to claim 1, wherein said method comprises providing the composition to said individual in multiple doses, wherein preferably the multiple doses are separated by minutes, hours, days, or weeks.

3. A composition for use according to claim 1, wherein said individual is provided with an additional therapy for the anaphylaxis.

4. A composition for use according to claim 1, wherein the composition comprises N-(1',2-dihydroxy-1,2'-binaphthalen-4'-yl)-4-methoxybenzenesulfonamide.

5. A composition for use according to claim 1, wherein the composition inhibits Stat3, Stat1, or both.

6. A composition for use according to claim 1, wherein in said method the composition is provided to said individual prior to exposure or following exposure to at least one allergen that induces the anaphylaxis.

7. A composition for use according to claim 6, wherein in said method the composition is provided to said individual at least 0.25, 0.5, 0.75, 1, 2, 3, 4, 5, 6, 7, 8, 9, 10, 11, 12, 13, 14, 15, 16, 17, 18, 19, 20, 21, 22, 23, or 24 hours; or at least 1, 2, 3, 4, 5, 6, or 7 days; or at least 1, 2, 3, or 4 weeks prior to or following exposure to at least one allergen that induces the anaphylaxis.

8. A composition for use according to claim 6, wherein said allergen is a food allergen, wherein preferably the food allergen is one or more food allergens selected from the group consisting of milk, legumes, shellfish, tree nuts, eggs, fish, soy, and wheat.

9. A composition for use according to claim 6, wherein said allergen is an environmental allergen or seasonal allergen, wherein preferably the environmental allergen or seasonal allergen is pollen or mold.

10. A composition for use according to claim 6, wherein said allergen is a venom allergen, wherein preferably the venom allergen is from an organism selected from the group consisting of wasp, bee, ant, hornet, yellow jacket, and asp.

11. A composition for use according to claim 6, wherein said allergen is a medication allergen, wherein preferably the medication allergen is selected from the group consisting of anesthetics, β-lactam antibiotics, aspirin, non-steroidal anti-inflammatory drug, chemotherapy, vaccine, protamine and herbal preparations.

12. A composition for use according to claim 6, wherein the allergen is latex.

13. A composition for use according to claim 1, wherein said method further comprises the step of diagnosing the anaphylaxis.

14. A composition for use according to claim 1, wherein in said method the composition is delivered intravenously, intradermally, intraarterially, intraperitoneally, intralesionally, intracranially, intraarticularly, intraprostaticaly, intrapleurally, intratracheally, intranasally, intravitreally, intravaginally, intrarectally, topically, intratumorally, intramuscularly, intraperitoneally, subcutaneously, subconjunctival, intravesicularlly, mucosally, intrapericardially, sublingually, intraumbilically, intraocularally, orally, topically, locally, injection, infusion, continuous infusion, localized perfusion, *via* a catheter, *via* a lavage, in lipid compositions, in liposome compositions, or as an aerosol.

## Patentansprüche

1. Zusammensetzung zur Verwendung in einem Verfahren zur Verhinderung oder Verringerung des Risikos oder der Schwere einer Anaphylaxie bei einem Individuum,
wobei die Zusammensetzung ausgewählt ist aus der Gruppe bestehend aus N-(1',2-Dihydroxy-1,2'-binaphthalin-4'-yl)-4-methoxybenzolsulfonamid, N-(3,1'-Dihydroxy-[1,2']-binaphthalinyl-4'-yl)-4-methoxybenzolsulfonamid, N-(4,1'-Dihydroxy-[1,2']-binaphthalinyl-4'-yl) -4-methoxybenzolsulfonamid, N-(5,1'-Dihydroxy-[1,2']-Binaphthalinyl-4'-yl)-4-methoxybenzolsulfonamid, N-(6,1'-Dihydroxy-[1,2']-Binaphthalinyl-4'-yl)-4-methoxybenzolsulfonamid, N-(7,1'-Dihydroxy-[1,2']binaphthalinyl-4'-yl)-4-methoxybenzolsulfonamid, N-(8,1'-Dihydroxy-[1,2']binaphthalinyl-4'-yl)-4-methoxybenzolsulfonamid, 4-Brom-N-(1,6'-dihydroxy-[2, 2']binaphthalinyl-4-yl)benzolsulfonamid und Kombinationen davon; und
wobei das Verfahren den Schritt umfasst, dem Individuum eine wirksame Menge einer oder mehrerer der Zusammensetzungen bereitzustellen.

2. Zusammensetzung zur Verwendung nach Anspruch 1, wobei das Verfahren Bereitstellen der Zusammensetzung dem Individuum in mehreren Dosen umfasst, wobei die mehreren Dosen vorzugsweise durch Minuten, Stunden, Tage oder Wochen getrennt sind.

3. Zusammensetzung zur Verwendung nach Anspruch 1, wobei dem Individuum eine zusätzliche Therapie für die Anaphylaxie bereitgestellt wird.

4. Zusammensetzung zur Verwendung nach Anspruch 1, wobei die Zusammensetzung N-(1',2-Dihydroxy-1,2'-binaphthalin-4'-yl)-4-methoxybenzolsulfonamid umfasst.

5. Zusammensetzung zur Verwendung nach Anspruch 1, wobei die Zusammensetzung Stat3, Stat1 oder beides hemmt.

6. Zusammensetzung zur Verwendung nach Anspruch 1, wobei in dem Verfahren die Zusammensetzung dem Individuum vor der Exposition oder nach der Exposition gegenüber mindestens einem Allergen, das die Anaphylaxie induziert, bereitgestellt wird.

7. Zusammensetzung zur Verwendung nach Anspruch 6, wobei in dem Verfahren die Zusammensetzung dem Individuum mindestens 0,25, 0,5, 0,75, 1, 2, 3, 4, 5, 6, 7, 8, 9, 10, 11, 12, 13, 14, 15, 16, 17, 18, 19, 20, 21, 22, 23 oder 24 Stunden oder mindestens 1, 2, 3, 4, 5, 6 oder 7 Tage; oder mindestens 1, 2, 3 oder 4 Wochen vor oder nach der Exposition gegenüber mindestens einem Allergen, das die Anaphylaxie induziert, bereitgestellt wird.

8. Zusammensetzung zur Verwendung nach Anspruch 6, wobei das Allergen ein Nahrungsmittelallergen ist, wobei das Nahrungsmittelallergen vorzugsweise ein oder mehrere Nahrungsmittelallergene ist, ausgewählt aus der Gruppe bestehend aus Milch, Hülsenfrüchten, Schalentieren, Baumnüssen, Eiern, Fisch, Soja und Weizen.

9. Zusammensetzung zur Verwendung nach Anspruch 6, wobei das Allergen ein Umweltallergen oder ein saisonales Allergen ist, wobei das Umweltallergen oder das saisonale Allergen vorzugsweise Pollen oder Schimmel ist.

10. Zusammensetzung zur Verwendung nach Anspruch 6, wobei das Allergen ein Giftallergen ist, wobei das Giftallergen vorzugsweise aus einem Organismus ausgewählt ist aus der Gruppe bestehend aus Wespe, Biene, Ameise, Hornisse, Yellowjacket und Aspisviper.

11. Zusammensetzung zur Verwendung nach Anspruch 6, wobei das Allergen ein Arzneimittelallergen ist, wobei das Arzneimittelallergen vorzugsweise ausgewählt ist aus der Gruppe bestehend aus Anästhetika, β-Lactam-Antibiotika, Aspirin, nichtsteroidalem entzündungshemmenden Arzneimittel, Chemotherapie, Impfstoffen, Protamin- und Kräuterpräparaten.

12. Zusammensetzung zur Verwendung nach Anspruch 6, wobei das Allergen Latex ist.

13. Zusammensetzung zur Verwendung nach Anspruch 1, wobei das Verfahren weiterhin den Schritt des Diagnostizierens der Anaphylaxie umfasst.

14. Zusammensetzung zur Verwendung nach Anspruch 1, wobei in dem Verfahren die Zusammensetzung intravenös, intradermal, intraarteriell, intraperitoneal, intraläsional, intracranial, intraartikulär, intraprostatisch, intrapleural, intratracheal, intranasal, intravitreal, intravaginal, intrarektal, topisch, intratumoral, intramuskulär, intraperitoneal, subkutan, subkonjunktival, intravesikular, mukosal, intraperikardial, sublingual, intraumbilikal, intraokular, oral, topisch, lokal, durch Injektion, durch Infusion, kontinuierliche Infusion, durch lokalisierte Perfusion, über einen Katheter, über eine Spülung, in Lipidzusammensetzungen, in Liposomenzusammensetzungen oder als Aerosol verabreicht wird.

## Revendications

1. Composition destinée à être utilisée dans un procédé de prévention ou de réduction du risque ou de la gravité de l'anaphylaxie chez un individu,
dans lequel ladite composition est choisie dans le groupe constitué de N-(1',2-dihydroxy-1,2'-binaphtalène-4'-yl) -4-méthoxybenzènesulfonamide, N-(3,1'-dihydroxy-[1,2']binaphtalényl-4'-yl)-4-méthoxy-benzènesulfonamide, N-(4,1'-dihydroxy-[1,2']binaphtalényl-4'-yl)-4-méthoxy-benzènesulfonamide, N-(5,1'-dihydroxy-[1,2']binaphtalényl-4'-yl)-4-méthoxy-benzènesulfonamide, N-(6,1'-dihydroxy-[1,2']binaphtalényl-4'-yl)-4-méthoxy-benzènesulfonamide, N-(7,1'-dihydroxy-[1,2']binaphthalenyl-4'-yl)-4-méthoxy-benzènesulfonamide, N-(8,1'-dihydroxy-[1,2']binaphthalenyl-4'-yl)-4-méthoxy-benzènesulfonamide, 4-Bromo-N-(1,6'-dihydroxy-[2,2']binaphtalényl-4-yl)-benzènesulfonamide, et leurs combinaisons; et
dans lequel ledit procédé comprend l'étape consistant à fournir à l'individu une quantité efficace d'une ou plusieurs desdites compositions.

2. Composition à utiliser selon la revendication 1, dans laquelle ledit procédé comprend la fourniture de ladite composition audit individu en doses multiples, dans lequel de préférence les doses multiples sont séparées par des minutes, des heures, des jours ou des semaines.

3. Composition à utiliser selon la revendication 1, dans laquelle ledit individu reçoit une thérapie supplémentaire pour l'anaphylaxie.

4. Composition à utiliser selon la revendication 1, dans laquelle la composition comprend du N-(1',2-dihydroxy-1,2'-binaphtalène-4'-yl)-4-méthoxybenzènesulfonamide.

5. Composition à utiliser selon la revendication 1, dans laquelle la composition inhibe Stat3, Stat1, ou les deux.

6. Composition à utiliser selon la revendication 1, dans laquelle dans ledit procédé, la composition est fournie audit individu avant l'exposition ou après l'exposition à au moins un allergène qui induit l'anaphylaxie.

7. Composition à utiliser selon la revendication 6, dans laquelle dans ladite méthode la composition est fournie audit individu au moins 0,25, 0,5, 0,75, 1, 2, 3, 4, 5, 6, 7, 8, 9, 10, 11, 12, 13, 14, 15, 16, 17, 18, 19, 20, 21, 22, 23 ou 24 heures; ou au moins 1, 2, 3, 4, 5, 6 ou 7 jours; ou au moins 1, 2, 3 ou 4 semaines avant ou après l'exposition à au moins un allergène qui induit l'anaphylaxie.

8. Composition à utiliser selon la revendication 6, dans laquelle ledit allergène est un allergène alimentaire, dans lequel de préférence l'allergène alimentaire est un ou plusieurs allergènes alimentaires sélectionnés dans le groupe comprenant le lait, les légumineuses, les crustacés, les noix, les œufs, le poisson, le soja et le blé.

9. Composition à utiliser selon la revendication 6, dans laquelle ledit allergène est un allergène environnemental ou allergène saisonnier, dans lequel de préférence l'allergène environnemental ou l'allergène saisonnier est le pollen ou la moisissure.

10. Composition à utiliser selon la revendication 6, dans laquelle ledit allergène est un allergène de venin, dans lequel de préférence l'allergène de venin provient d'un organisme choisi dans le groupe constitué par la guêpe, l'abeille, la fourmi, le frelon, la jaquette jaune et le mégalopyge opercularis.

11. Composition à utiliser selon la revendication 6, dans laquelle ledit allergène est un allergène médicamenteux, dans lequel de préférence l'allergène médicamenteux est choisi dans le groupe comprenant les anesthésiques, les antibiotiques β-lactamines, l'aspirine, le médicament anti-inflammatoire non stéroïdien, la chimiothérapie, vaccin, protamine et préparations à base de plantes.

12. Composition à utiliser selon la revendication 6, dans laquelle l'allergène est le latex.

13. Composition à utiliser selon la revendication 1, dans laquelle ledit procédé comprend en outre l'étape de diagnostic de l'anaphylaxie.

14. Composition à utiliser selon la revendication 1, dans laquelle dans ledit procédé, la composition est délivrée par voie intraveineuse, intradermique, intraartérielle, intraperitoneale, intralesionale, intracrânienne, intraarticulaire, intraprostatique, intrapleurale, intratrachéale, intranasale, intravitréale, intravaginale, intrarectale, topique, intratumale , intramusculaire, intrapéritonéale, sous-cutanée, sous-conjonctivale, intra-vésiculaire, muqueuse, intra-péricardique, sublinguale, intra-utérine, intraoculaire, orale, topique, locale, injection, perfusion, perfusion continue, perfusion localisée, *via* un cathéter, *via* un lavage, dans des compositions lipidiques, dans des compositions de liposomes, ou sous forme d'aérosol.
